# EUROPEAN PATENT APPLICATION

(11) **EP 4 491 230 A1**
(43) Date of publication of application: **15.01.2025**
(21) Application number: 23185667.5
(22) Date of filing: 14.07.2023
(51) Int. Cl.: A61P 31/00, A61P 35/00, C07K 16/28

(54) **CROSS-SPECIFIC ANTIGEN BINDING PROTEINS (ABP) TARGETING LEUKOCYTE IMMUNOGLOBULIN-LIKE RECEPTOR SUBFAMILY B1 (LILRB1) AND LILRB2, COMBINATIONS AND USES THEREOF**

(71) Applicant: iOmx Therapeutics AG, 82152 Martinsried (DE)
(72) Inventor: ZANTOW, Jonas, 82152, Martinsried (DE); SCHILZ, Jonas, 82152, Martinsried (DE); FRIEDRICH, Simone, 82152, Martinsried (DE); HARTL, Christina, 82152, Martinsried (DE); MILDE, Ronny, 82152, Martinsried (DE); AIGNER, Maximilian, 82152, Martinsried (DE); SUDAN, Kritika, 82152, Martinsried (DE)
(74) Representative: Kuttenkeuler, David

(57) **Abstract**

The invention relates to antigen binding proteins, such as antibodies, that bind to both leukocyte immunoglobulin-like receptor subfamily B1 (LILRBI) and LILRB2 while not binding to, or binding with significantly less affinity to, leukocyte immunoglobulin-like receptor subfamily A (LILRA), which can also inhibit the interaction between LILRB1 and/or LILRB2 and a natural ligand of LILRB receptors (interacting proteins, such as HLA-G) on immune cells and the inhibition of such interaction can reduce immune cell suppression and thereby support anti-infection and anti-tumour immune responses in a subject suffering from such diseases. In particular, the invention provides products, compositions and methods for treating diseases using antigen binding proteins of LILRB1 and/or LILRB2, especially antigen binding proteins which while, preferably specifically, binding to LILRB1 and LILRB2, such as antigen binding proteins that further do not bind to, or bind with less affinity to, one or more LILRA receptor proteins (such as LILRA1 and/or LILRA3). Also provided are methods of reducing the immune suppression of cells involved with a cell-mediated immune response, and/or methods for treating infective- and/or proliferative diseases, using an LILRB1 and/or LILRB2 antigen binding protein such as an antibody binding to both LILRB1 and/or LILRB2, as well as certain related aspects including detection, diagnostic and screening methods.

## Description

The invention relates to antigen binding proteins, such as antibodies, that bind to both leukocyte immunoglobulin-like receptor subfamily B1 (LILRB1) and LILRB2 while not binding to, or binding with significantly less affinity to, leukocyte immunoglobulin-like receptor subfamily A (LILRA), which can also inhibit the interaction between LILRB1 and/or LILRB2 and a natural ligand of LILRB receptors (interacting proteins, such as HLA-G) on immune cells and the inhibition of such interaction can reduce immune cell suppression and thereby support anti-infection and anti-tumour immune responses in a subject suffering from such diseases. In particular, the invention provides products, compositions and methods for treating diseases using antigen binding proteins of LILRB1 and/or LILRB2, especially antigen binding proteins which while, preferably specifically, binding to LILRB1 and LILRB2, such as antigen binding proteins that further do not bind to, or bind with less affinity to, one or more LILRA receptor proteins (such as LILRA1 and/or LILRA3). Also provided are methods of reducing the immune suppression of cells involved with a cell-mediated immune response, and/or methods for treating infective- and/or proliferative diseases, using an LILRB1 and/or LILRB2 antigen binding protein such as an antibody binding to both LILRB1 and/or LILRB2, as well as certain related aspects including detection, diagnostic and screening methods.

In the treatment of cancer there are a number of approaches by which therapies may lead to the elimination of tumour cells, including those that involve or exploit one or more components of the immune system, either directly or indirectly. One of the limitations associated with such therapies is that cancerous cells often exploit immune-checkpoints to evade a patient's immune system, such as by preventing immune-recognition or down-regulating a tumour-specific cytotoxic T cell (CTL) response, thereby generating resistance against an immune response (Rabinovich et al 2007, Annu Rev Immunol 25:267; Zitvogel et al 2006, Nat Rev Immunol 6:715). Under normal conditions, such immune-regulatory checkpoints are crucial for the maintenance of self-tolerance under physiological conditions, but there is an increasing recognition of the important role that they can also play in cancer (Hanahan and Weinberg 2011, Cell; 144:646); cancerous cells can take over these mechanisms to evade and suppress the immune system in order to develop into a tumour (Drake et al 2006, Adv Immunol 90:51).

Current state of the art cancer therapies include blockade of those few immune-regulatory checkpoints presently known and for which their mechanism of action is understood. For example, blocking antibodies against surface-expressed immune-regulatory proteins, such as CTLA4 and PD-L1 (Chambers et al 2001, Annu Rev Immunol 19:565; Blank et al 2004, Cancer Res 64:1140), can boost anti-tumour immunity and have shown clinical success against many cancer types (Page et al 2014, Annu Rev Med 65:185). However, a large proportion of cancer patients does not respond to such checkpoint blockage therapy (Bu et al 2016, Trends Mol Med 22:448; Hugo et al 2016, Cell 165:35; Topalian et al 2012, New Engl J Med 366:2443), indicating that other immune-checkpoint pathways may be active. Indeed, synergistic cooperation between several immune-regulatory pathways maintains immune tolerance against tumours, which might explain why blocking only one immune-regulatory checkpoint node can still result in tumour escape (Woo et al 2012, Cancer Res 72:917; Berrien-Elliott et al 2013, Cancer Res 73:605). However, little is known about the molecular factors that are central to the mechanism of action of such immune-regulatory pathways. Indeed, successful cancer immunotherapy requires a systematic delineation of the entire immune-regulatory circuit - the 'immune modulatome'- expressed by tumours. Therefore, today, there is still an unmet need for identifying further molecular targets that may serve as immune-regulatory checkpoints and in particular an unmet need for means and methods to modulate, detect and otherwise utilise such possible checkpoint targets, such as in medicine, diagnosis and research.

The human leukocyte immunoglobulin-like receptor (LILR), also known as immunoglobulin-like transcript (ILT) family belongs to the superfamily of paired receptors that have the potential to transmit stimulatory or inhibitory signals according to the presence or absence of tyrosine-based signaling motifs in their cytoplasmic tail. Human LILRs consist of six stimulatory receptors (LILRA1-6) and five inhibitory receptors (LILRBI -5). LILRs are predominately expressed on myeloid and lymphoid cells and some non-immune cells, and the expression patterns are different from receptor to receptor. Polymorphism and copy-number variation contribute to diversity within humans. Receptor engagement results in intracellular phosphorylation of the tyrosine-based motifs within the receptors (LILRB) or on associated adaptor molecules (LILRA). Downstream signaling events can be mediated by phosphatases, such as SHP1, SHP2 and SHIP. In general, LILR activity can result in the upregulation or downregulation of both innate and adaptive immune functions with a range of effects on different cell types. Certain LILRs also play regulatory roles in neuronal activity and osteoclast development.

LILRB1 is broadly expressed on myeloid cells, as well as B cells and subsets of T cells and natural killer (NK) cells. LILRB2-5 are more restricted to myeloid cells and dendritic cells (DCs). Some of the ligands and signaling pathways for LILRBs have been identified. LILRB1 and LILRB2 are the best characterized receptors and both bind to classical (HLA-A, HLA-B and HLA-C) and non-classical (HLA-E, HLA-F, HLA-G and HLA-H) MHC class I or HLA class I molecules, as well as to members of the angiopoietin-like protein family. Because the immune-suppressive function of LILRBs is similar to that of the classical immune checkpoint proteins, CTLA-4 and PD-1, the interaction between LILRBs and ligands is proposed to serve as immune checkpoints. For example, engagement of LILRB1 and LILRB2 by HLA-G on cancer cells inhibits immune cell activation and generates regulatory T cells (Tregs) and suppressive antigen presenting cells (APCs), which can indirectly support tumor development. Further, the interaction of P2-microglobulin (P2M)-associated MHC class I on cancer cells with LILRB2 on macrophages leads to loss of immune surveillance. Whether the interaction between LILRB2 and ligand also functions as a phagocytosis checkpoint is unknown. LILRBs may also represent targets for induction of transplantation tolerance to prevent allograft rejection. LILRBs (especially LILRB2 and LILRB4) are critical for induction of the tolerogenic phenotype of APCs and initiation of the T cell suppression cascade that results in immune tolerance. LILRB1 and LILRB2 can also mediate graft tolerance by binding to HLA-G. In addition to immune cells, LILRBs are expressed by cancer cells and may support malignant transformation and relapse, as well as the activity of cancer stem cells. Collectively, these findings reveal dual roles for LILRBs as immune checkpoint molecules and as tumor-sustaining factors. Development of agents useful in modulating signaling from LILRBs may be of great benefit in diseases involving dysregulation of the immune system, including cancer, inflammatory diseases and autoimmune diseases, as well as transplantation rejection.

Therefore, there is a need, from one or more of the above perspectives, for novel approaches to reduce the immune-suppressive functions of the LILRB receptors while avoiding any inhibition of the immune-stimulatory LILRA receptors on immune cells involved in the defense against certain disorders (such as an infection or tumour). The present invention seeks to provide, in particular, novel therapeutic approaches and methods involving novel compounds; for example, compounds and ABPs that reduce the immune-suppressive function of LILRB receptors while not inhibiting the immune-stimulatory function of any of the LILRA receptors. Furthermore, the invention seeks to provide novel strategies to diagnose, prognose and/or monitor immune cells on the basis of LILRB expression. Accordingly, it is an object of the present invention to provide alternative, improved, simpler, cheaper and/or integrated means or methods that address one or more of these or other problems. Such an object underlying the present invention is solved by the subject matter as disclosed or defined anywhere herein, for example by the subject matter of the itemized embodiments and/or attached claims.

The invention is grounded by the surprising finding that certain antigen binding proteins, such as antibodies, of the invention bind selectively and specifically to both LILRB1 and LILRB2, while not binding, or binding relatively less selective and less specific, to any of the LILRA receptors. In certain preferred aspects of the invention the antibodies of the invention are maintain cross-specificity with respect to their on targets LILRB1 and LILRB2 while binding significantly less to the off-target proteins LILRA1 and LILRA3. Maintinaing cross specific binding only with respect to the LILRB on-target proteins without off-target binding to LILRAs is one of multiple surprising features of the antibodies of the present invention.

Generally, therefore, and by way of brief description, the main aspects of the present invention can be described as follows:
In **a first aspect,** the invention relates to an antigen binding protein (ABP) which specifically binds to LILRB1 and/or LILRB2 protein (eg to the extra cellular domain (ECD) of LILRB1 and/or LILRB2 protein) and, optionally, wherein the ABP is able to inhibit the binding of a ligand of LILRB1 and/or LILRB2 protein or a variant thereof to LILRB1 and/or LILRB2 protein or a variant thereof. Preferably, the ABP does not specifically binding to, or binds significantly less to, leukocyte immunoglobulin-like receptor subfamily A (LILRA) type proteins.
In **a second aspect,** the invention relates to an ABP which competes with an ABP of a first aspect for binding to LILRB1 and/or LILRB2 protein (eg to the ECD of LILRB1 and/or LILRB2 protein). In a **related aspect,** the invention relates to an ABP which binds to the same epitope as an ABP of a first aspect.
   In **another aspect,** the invention relates to an antigen binding domain (ABD) of an ABP of the invention.
In **a third aspect,** the invention relates to a nucleic acid encoding for an ABP or ABD of the invention or of components thereof, and in **related aspects,** the invention relates to a nucleic acid construct (NAC) comprising such a nucleic acid, and relates to a host cell comprising a nucleic acid or NAC of the invention.
In **a fourth aspect,** the invention relates to a pharmaceutical composition comprising an ABP, ABD, nucleic acid, NAC or host cell of the invention, or comprising a compound that is a modulator of the expression, function, activity and/or stability of leukocyte immunoglobulin-like receptor subfamily B1 (LILRB1) and/or LILRB2 type proteins, and a pharmaceutically acceptable carrier, stabiliser and/or excipient.
In **a fifth aspect,** the invention relates to **a** method for the treatment of certain diseases, disorders or conditions in a subject by administering a product to the subject, wherein the product is selected from the list consisting of an ABP, ABD, nucleic acid, NAC and host cell of the invention, or is a compound that is a modulator of the expression, function, activity and/or stability of LILRB1 and/or LILRB2. In **related aspects,** the invention relates to a product for use in medicine, and relates to the use of a product for the manufacture of a medicament, wherein the product is selected from the list consisting of an ABP, ABD, nucleic acid, NAC or host cell of the invention, or is a compound that is modulator of the expression, function, activity and/or stability of LILRB1 and/or LILRB2.

The invention also relates in **other aspects** to various methods to produce a recombinant cell line or ABP of the invention, a hybridoma or host cell capable of producing an ABP of the present invention, as well as relating to various determination and/or diagnostic methods or uses, and to kits useful for such determination and/or diagnostic methods, as well as to various methods for identifying and/or charactering compounds, such as those suitable for use in medicine.

The figures show:

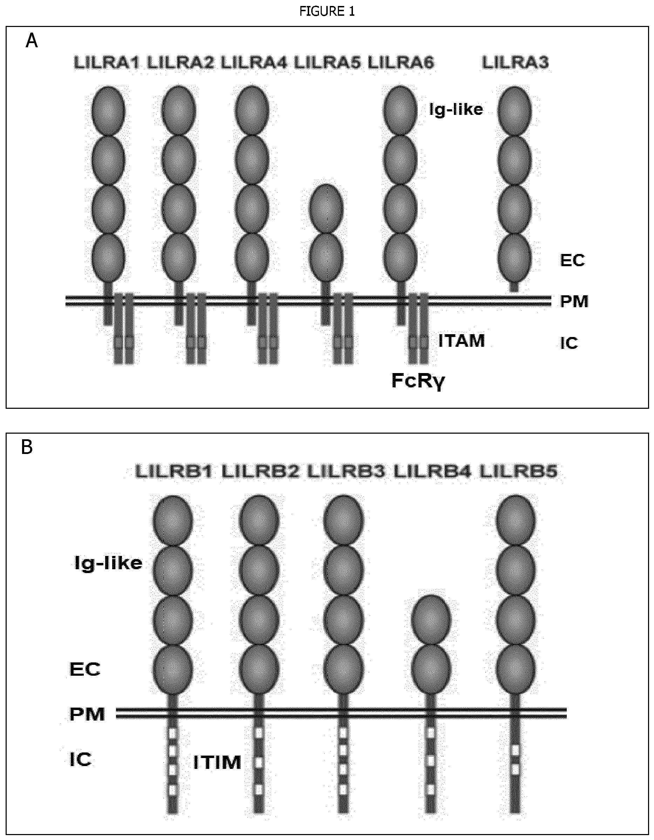

**Figure 1****:** shows pictorial representation of the domain structure of the Leukocyte Immunoglobulin-Like Receptor family members of LILRA **(A)** and LILRB **(B).** Ig-like = Ig-like domains, EC = Extracellular, PM = Plasma membrane, IC = Intracellular, white boxes = ΓΓIM. Adapted from Hirayasu K. & Arase H. (2016, Nat Microbiol.)

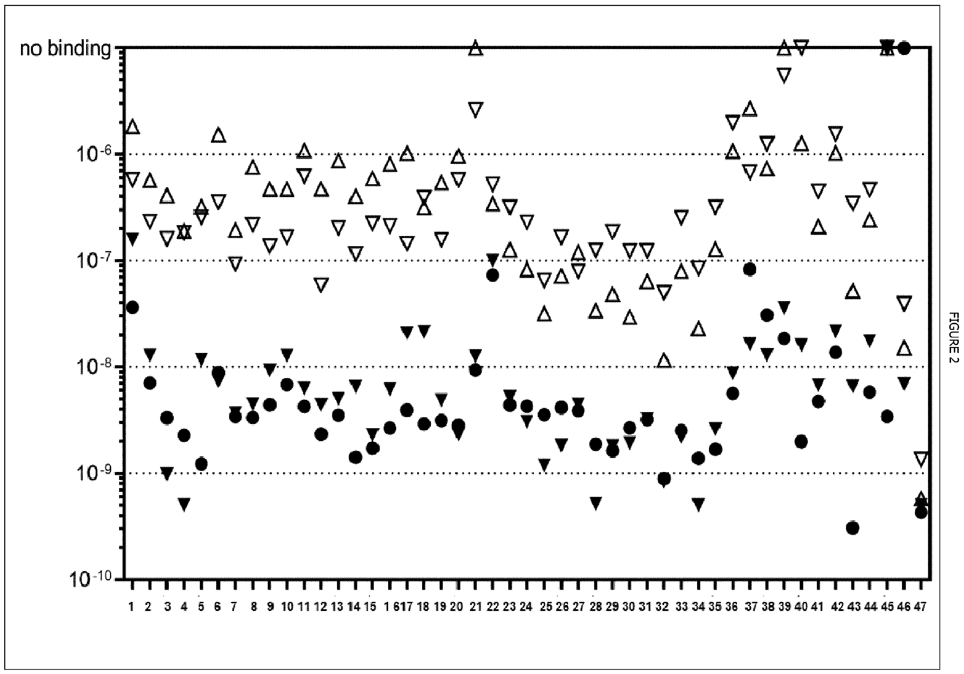

**Figure 2****:** shows binding affinity of antibodies of the invention to LILRB1 and LILRB2 and the counter targets LILRA1 and LILRA3. Y axis = KD (M), open triangle LILRA1, open inverted triangle = LILRA3, solid inverted triangle = LILRB1, solid circle = LILRB2; X axis = antibodies, 1 = A-001, 2 = A-004 , 3 = A-005 , 4 = A-006 , 5 = A-007 , 6 = A-008, 7 = A-009 , 8 = A-010 , 9 = A-011 , 10 = A-012 , 11 = A-0 13, 12 = A-014 , 13 = A-015 , 14 = A-016 , 15 = A-017 , 16 = A-018 , 17 = A-019 , 18 = A-020 , 19 = A-021 , 20 = A-045 , 21 = A-046, 22 = A-003 , 23 = A-022 , 24 = A-023 , 25 = A-024 , 26 = A-025 , 27 = A-026 , 28 = A-027, 29 = A-028 , 30 = A-029 , 31 = A-030 , 32 = A-031 , 33 = A-032 , 34 = A-033 , 35 = A-047 , 36 = A-048 , 37 = A-034 , 38 = A-035 , 39 = A-036 , 40 = A-037 , 41 = A-038 , 42 = A-039 , 43 = A-040 , 44 = A-041 , 45 = [Ref047, MK-4830, LILRB2 specific], 46 = [Ref051, BND-22, LILRB1 specific], 47 = [Ref062, NGM707, cross-specific].

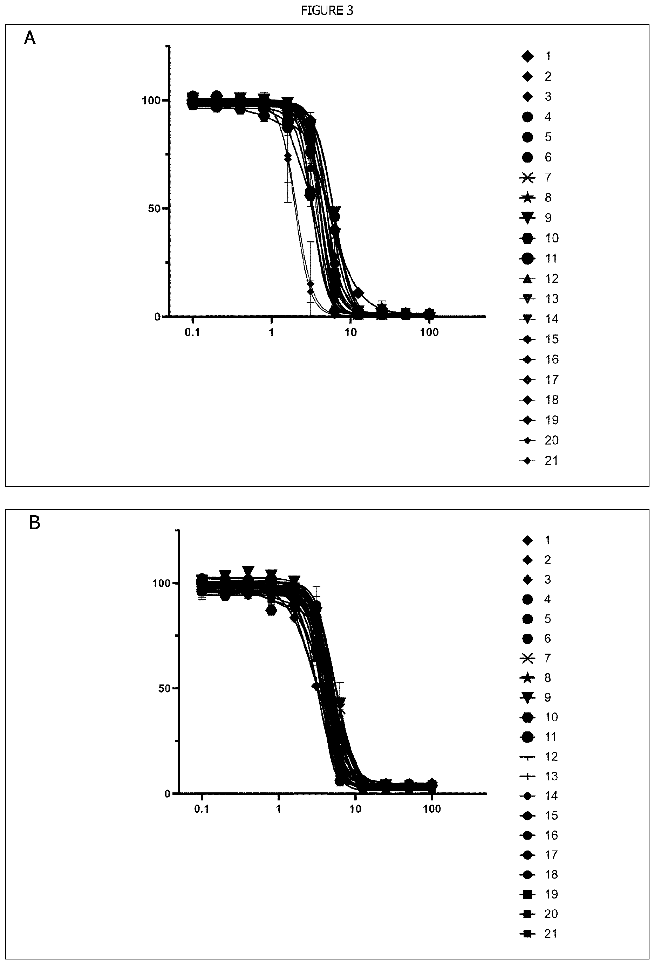

**Figure 3****:** shows inhibition of the interaction of (A) LILRB1 or (B) LILRB2 and their ligand HLA-G using antibody of the invention clone A-001, and maturated variants thereof. Y-axes = Residual Binding (%), x-axes = Antibody concentration (nM); 1 = A-001 , 2 = A-004 , 3 = A-005 , 4 = A-006 , 5 = A-007 , 6 = A-008 , 7 = A-009 , 8 = A-010 , 9 = A-011 , 10 = A-012 , 11 = A-013 , 12 = A-014 , 13 = A-015 , 14 = A-016 , 15 = A-017 , 16 = A-018 , 17 = A-019 , 18 = A-020 , 19 = A-021, 20 = A-045 , 21 = A-046.

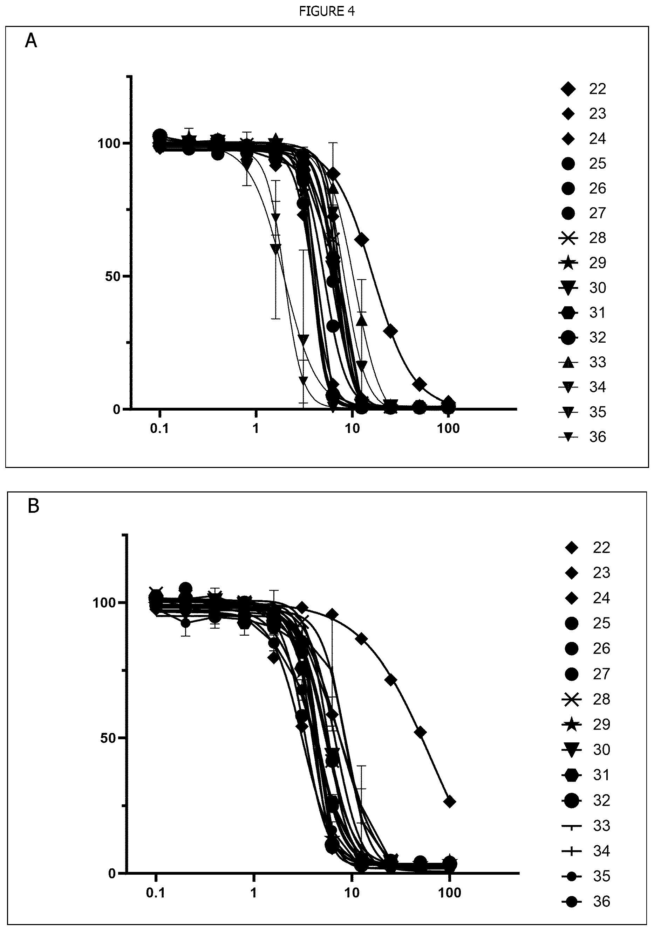

**Figure 4****:** shows inhibition of the interaction of **(A)** LILRB1 or **(B)** LILRB2 and their ligand HLA-G using antibody of the invention clone A-003 and maturated variants of thereof. Y-axes = Residual Binding (%), x-axes = Antibody concentration (nM); 22 = A-003 , 23 = A-022 , 24 = A-023 , 25 = A-024 , 26 = A-025 , 27 = A-026 , 28 = A-027 , 29 = A-028 , 30 = A-029 , 31 = A-030 , 32 = A-031, 33 = A-032 , 34 = A-033, 35 = A-047 , 36 = A-048.

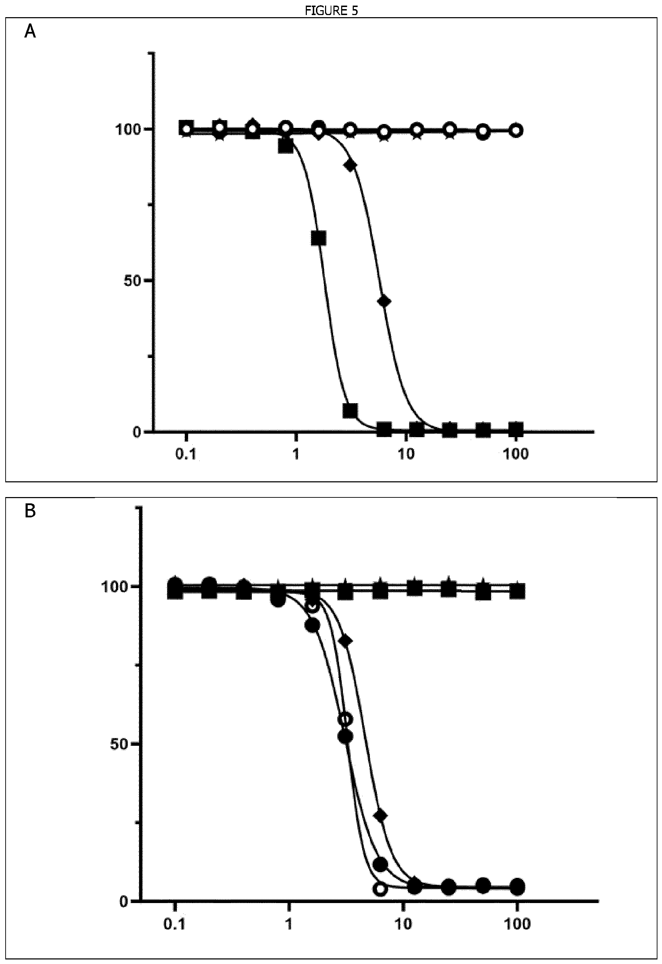

**Figure 5****:** shows inhibition of the interaction of LILRB1 or LILRB2 and their ligand HLA-G using reference antibodies. **(A)** shows inhibition of LILRB1 and HLA-G; **(B)** shows inhibition of LILRB2 and HLA-G. Y-axes = Residual Binding (%), x-axes = Antibody concentration (nM); open circle = Ref065 [IO-108, LILRB2 specific], diamond = Ref062 [NGM707, cross-specific], solid square = Ref051 [BND-22, LILRB1 specific], solid circle = Ref047 [MK-4830, LILRB2 specific], solid star = Ref001 (isotype control).

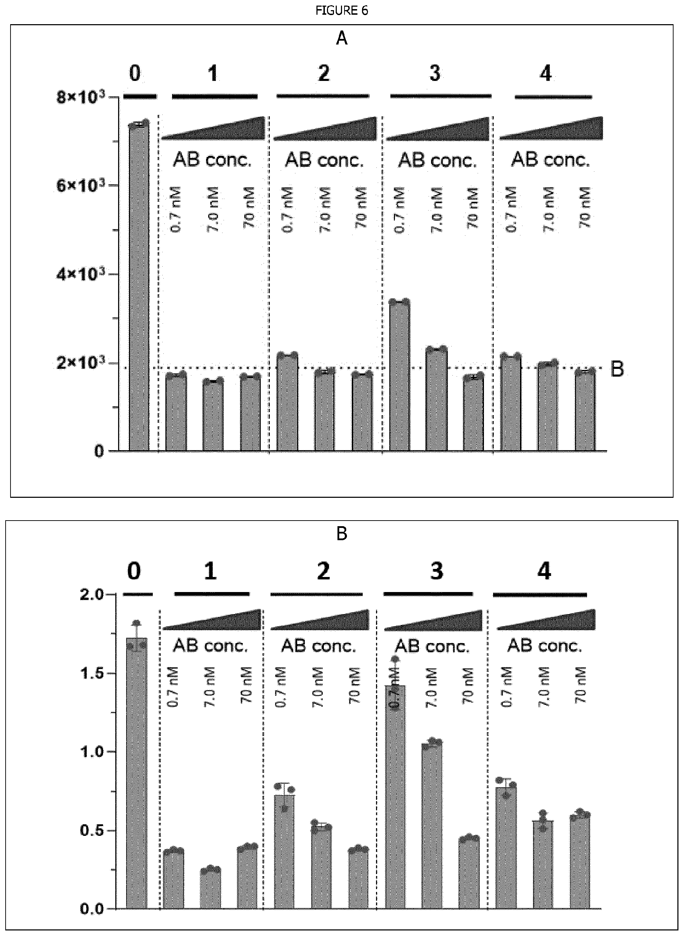

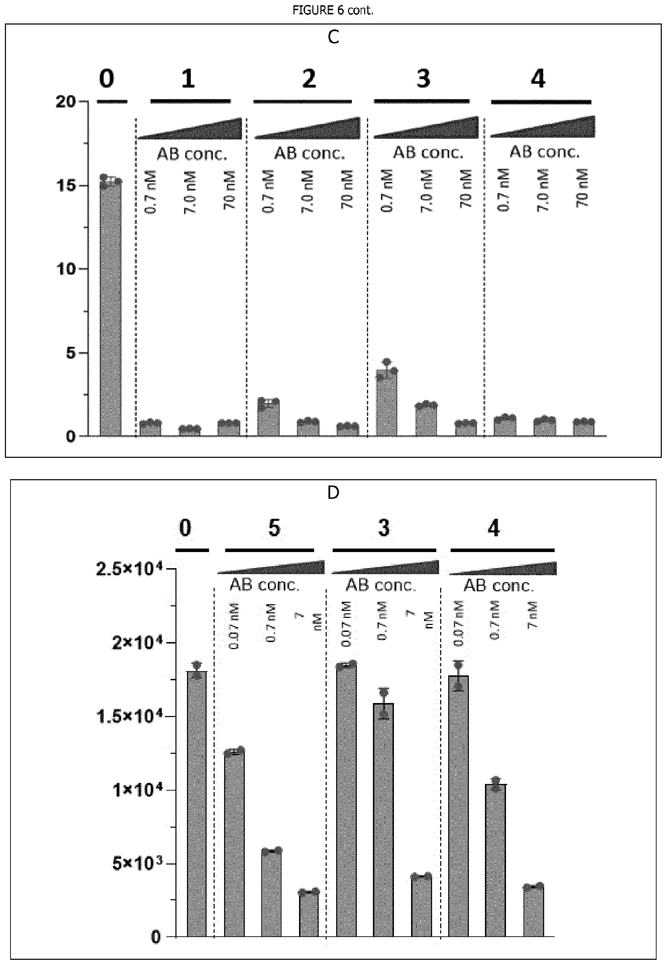

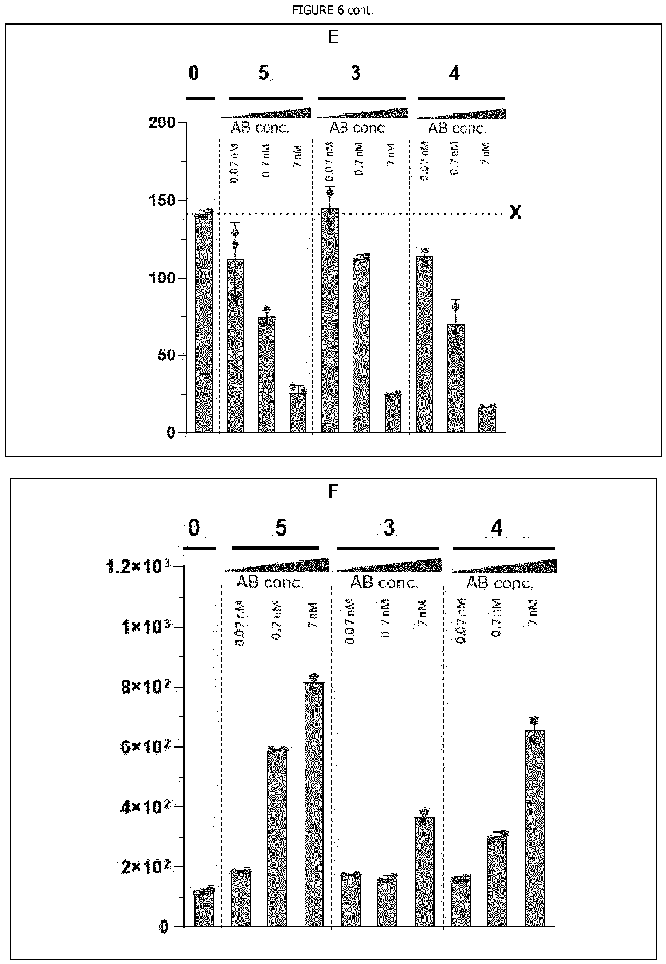

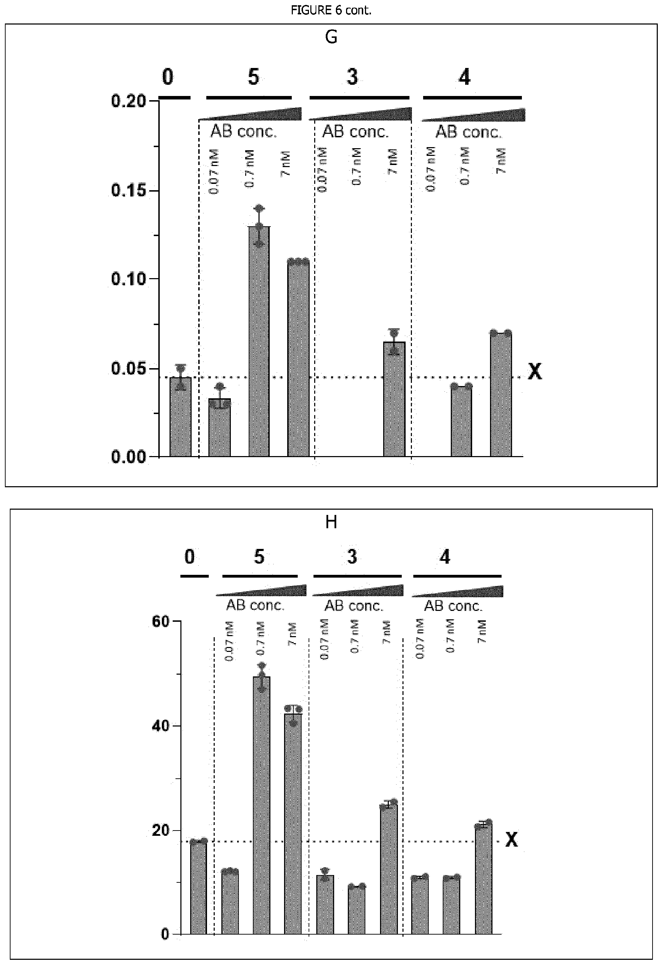

**Figure 6****:** shows effects of anti-LILRB2/1 blockade on **(A** - **C)** polarization of human M2 macrophages in a functional myeloid assay and **(D - H)** on polarization of human M2 macrophages and cytotoxic T cells in a myeloid suppression assay. Such effects were assessed by **(A and D)** surface M2 marker CD163 and (**F**) early activation marker CD69 (Y axis = median FI of live cells; B = background), and cytokine secretion of (**B**) CCL13, (**C**) CCL23, (**E**) CCL18, **(G)** GM-CSF and **(H)** IFNγ (Y axis = cytokine concentration (pg/ml); X axes = antibodies at indicated concentrations; 0 = Ref001 (isotype control); 1 = A-010 ; 2 = A-026 ; 3 = Ref047 [LILRB2 specific MK-4830]; 4 = Ref062 [NGM707, cross-specific]; 5 = A-045; X = isotype control).

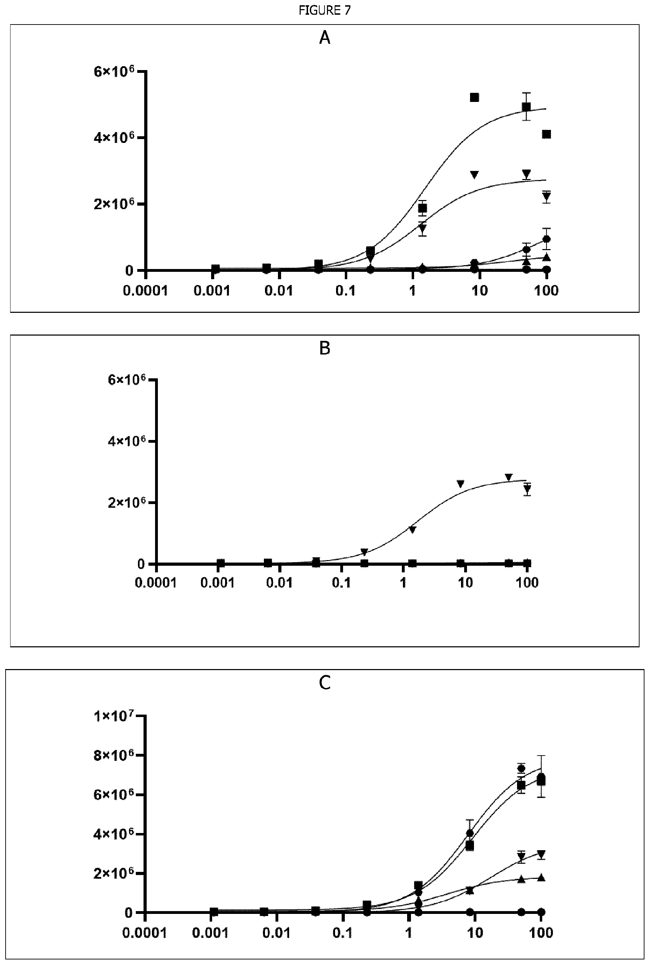

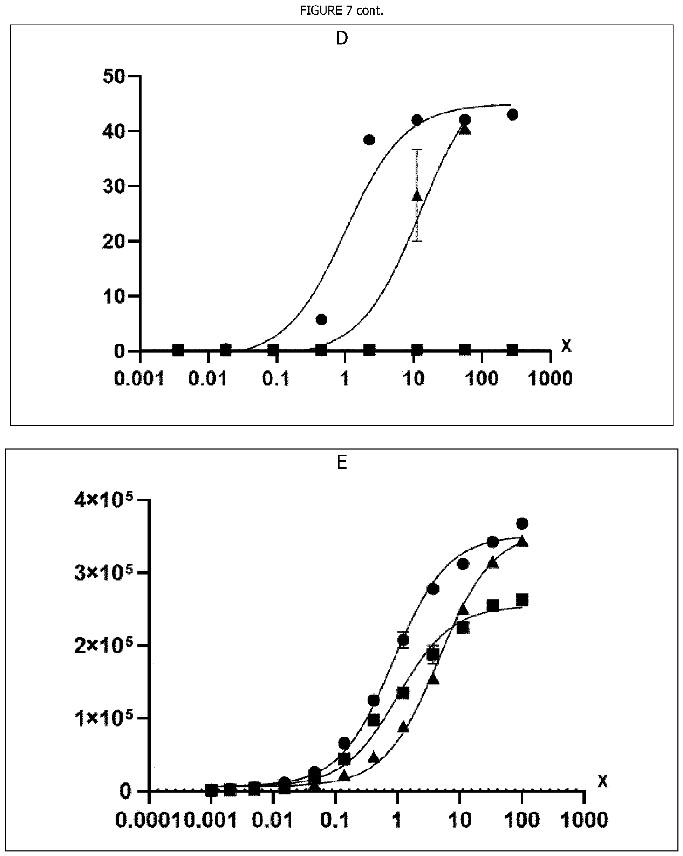

**Figure 7****:** shows target binding of LILRB2/1 antibodies **(A** = A-045, **B** = Ref047 [LILRB2 specific MK-4830], and **C** = Ref062 [NGM707, cross-specific]) to transfected Expi293 cells, **(D)** to primary **T** cells and **(E)** to monocytes; Y axis = % positive (of CD8+ cells) [for D only], otherwise Y axes = Median FI (RL1-H) of GFP+cells (pg/ml); X axes = antibodies concentrations (nM); X = isotype control. For A - C: circle = non-transfected, square = LILRB1, triangle = LILRA1, inverted triangle LILRB2, diamond = LILRA2, and hexagon = LILRA3. For D and E: circle = A-045, square = Ref047 [LILRB2 specific MK-4830], and triangle **C** = Ref062 [NGM707, cross-specific].

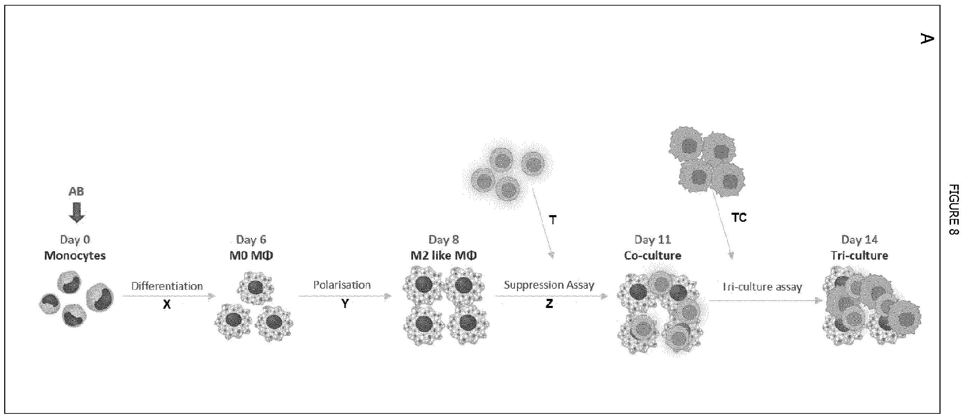

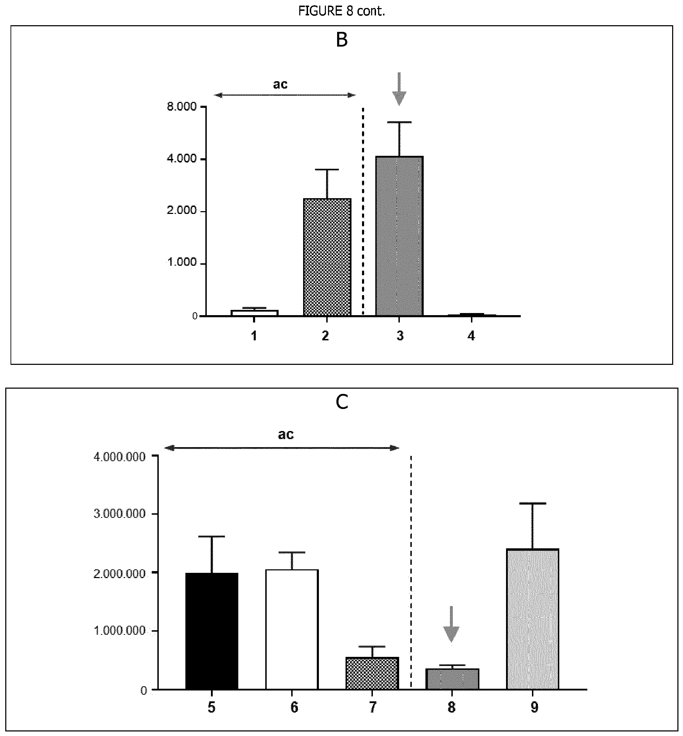

**Figure 8****:** depicts the **(A)** the Tri-culture Assay principle; AB = addition of antibodies (antibodies will be present throughout the assay), T = addition of T cells, TC = addition of tumor cells, X = differentiation of monocytes with M-CSF, Y = polarization of macrophages by addition of IL-4, IL-10 and TGF-β; Z = activation of autologous T cells by addition of anti-CD3 and anti-CD28; and shows **(B)** reversal of T cell suppression (Y-axis = IL-2 [pg/ml]) and **(C)** tumor cell killing (Y-axis = tumor cell signal [RLU]) by optimized A-010; X-axes: 1 = unstimulated T cells only, 2 = stimulated T cells only, 3 = stimulated T cells + M2-polarized macrophages + A-010; 4 = stimulated T cells + M2-polarized macrophages + Ref001 (isotype control); 5 = tumor cells only, 6 = tumor cells + unstimulated T cells only, 7 = tumor cells + stimulated T cells only, 8 = tumor cells + stimulated T cells + M2-polarized macrophages + A-010, 9 = tumor cells + stimulated T cells + M2-polarized macrophages + Ref 001 (isotype control); ac = assay controls.

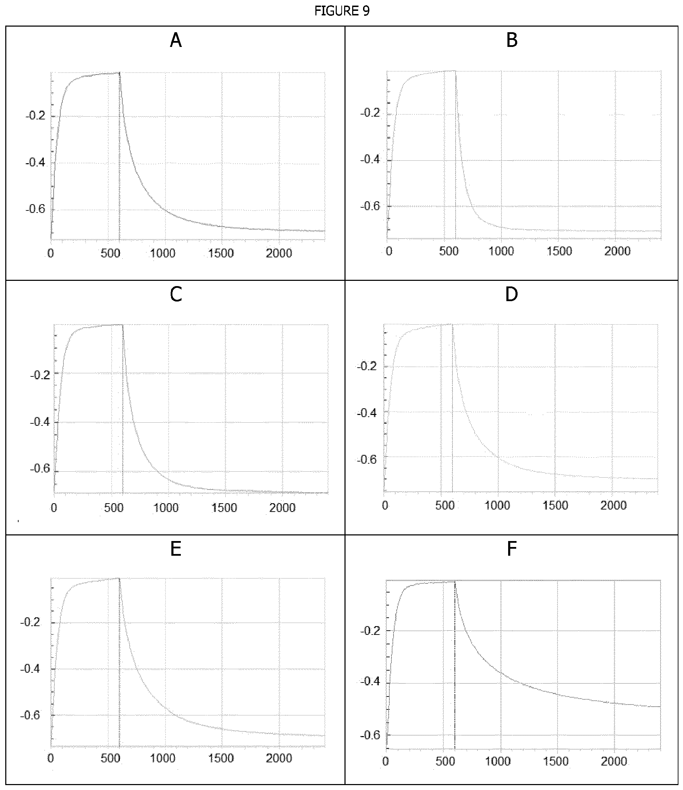

Figure **9****:** depicts BLI measurements showing displacement of LILRB2 from HLA-G interaction site and inhibition of new LILRB2 and HLA-G complex formation using antigen binding fragments obtained from antibodies of the invention (A) A-010, (B) A-045, (C) A-047 , (D) A-048, antigen binding fragment obtained from (E) reference molecule Ref062 [NGM707, cross-specific] or (F) assay buffer. Y-axes = wavelength shift (nm), x-axes = time (sec).

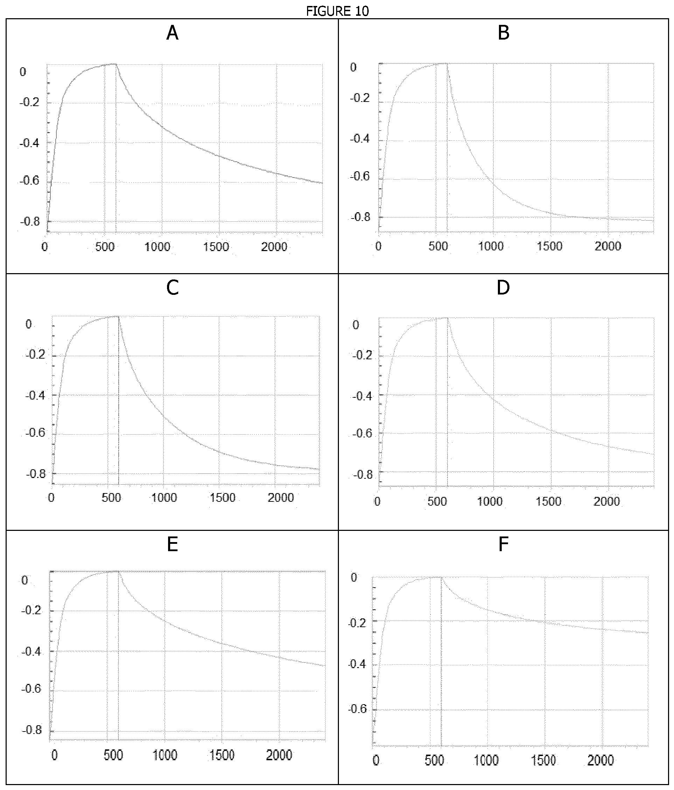

Figure **10****:** depicts BLI measurements showing displacement of LILRB1 from HLA-G interaction site and inhibition of new LILRB1 and HLA-G complex formation using antigen binding fragments obtained from antibodies of the invention (A) A-010, (B) A-045, (C) A-047 , (D) A-048, antigen binding fragment obtained from (E) reference molecule Ref062 [NGM707, cross-specific] or (F) assay buffer. Y-axes = wavelength shift (nm), x-axes = time (sec).

The present invention, and non-limiting aspects and/or embodiments thereof, can be described in more detail as follows:
In **a first aspect,** and as may be further described, defined, claimed or otherwise disclosed herein, the invention relates to **an antigen binding protein (ABP)** which specifically binds to LILRB1 and/or LILRB2 protein (eg to the extra cellular domain (ECD) of LILRB1 and/or LILRB2 protein) and, optionally, wherein the ABP and is able to inhibit (eg, inhibits) the interaction between a natural ligand of LILRB1 and/or LILRB2 (such as HLA-G) protein or a variant thereof and LILRB1 and/or LILRB2 protein or a variant thereof, for example, the ABP is optionally able to inhibit (eg, inhibits) the binding of LILRB1 and/or LILRB2 protein or a ligand of LILRB1 and/or LILRB2.In context of the present invention in all of its aspects and embodiments, whenever antigen binding proteins of the invention are referred to as binding to LILRB1 and/or LILRB2, in a most preferred variation thereof a binding of such antigen binding protein of the invention, such as an antibody, is preferably one which binds to both LILRB1 and LILRB2. Preferably such antigen-binding protein is one which does not bind to, or binds with less affinity to, LILRA1 and LILRA3.

The invention in further preferred embodiments relates to antigen binding proteins (ABPs) which specifically bind to LILRB1 and LILRB2 protein (eg to the extra cellular domain (ECD) of LILRB1 and LILRB2 protein) and, optionally, wherein the ABP and is able to inhibit (eg, inhibits) the interaction between a natural ligand of LILRB1 and the natural ligand of LILRB2 protein or (such as HLA-G). Preferably, the ABP of the invention binds to LILRB1 and LILRB2 with significantly higher affinity compared to the binding of the ABP to LILRA1 and LILRA2, optionally, wherein the ABP and is not able to inhibit (eg, does not inhibit) the interaction between a natural ligand of LILRA1 and the natural ligand of LILRA3 protein or (such as HLA-G).

### Antigen binding proteins targeting LILRB1 and/or LILRB2, preferably both LILRB1 and LILRB2

An "antigen binding protein" ("ABP") as used herein means a protein that specifically binds to a target antigen, such as to one or more epitope(s) displayed by or present on a target antigen. The antigen of the ABPs of the invention is LILRB1 and/or LILRB2; and the ABP can, optionally bind to one or more extracellular domains of said LILRB1 and/or LILRB2 (such as the epitope(s) can be displayed by or present on one or more extracellular domains of said LILRB1 and/or LILRB2). Typically, an antigen binding protein is an antibody (or a fragment thereof), however other forms of antigen binding protein are also envisioned by the invention. For example, the ABP may be another (non-antibody) receptor protein derived from small and robust non-immunoglobulin "scaffolds", such as those equipped with binding functions for example by using methods of combinatorial protein design (Gebauer & Skerra, 2009; Curr Opin Chem Biol, 13:245). Particular examples of such non-antibody ABPs include: Affibody molecules based on the Z domain of Protein A (Nygren, 2008; FEBS J 275:2668); Affilins based on gamma-B crystalline and/or ubiquitin (Ebersbach et al, 2007; J Mo Biol, 372:172); Affimers based on cystatin (Johnson et al, 2012; Anal Chem 84:6553); Affitins based on Sac7d from Sulfolobus acidcaldarius (Krehenbrink et al, 2008; J Mol Biol 383: 1058); Alphabodies based on a triple helix coiled coil (Desmet et al, 2014; Nature Comms 5:5237); Anticalins based on lipocalins (Skerra, 2008; FEBS J 275:2677); Avimers based on A domains of various membrane receptors (Silverman et al, 2005; Nat Biotechnol 23:1556); DARPins based on an ankyrin repeat motif (Strumpp et al, 2008; Drug Discov Today, 13:695); Fynomers based on an SH3 domain of Fyn (Grabulovski et al, 2007; J Biol Chem 282:3196); Kunitz domain peptides based on Kunitz domains of various protease inhibitors (Nixon et al, Curr opin Drug Discov Devel, 9:261) and Centyrins and Monobodies based on a 10th type III domain of fibronectin (Diem et al., 2014; Protein Eng Des Sel 27:419 doi: 10.1093/protein/gzu016; Koide & Koide, 2007; Methods Mol Biol 352:95). In the context of an ABP of the present invention that specifically binds LILRB1 and/or LILRB2 (and does not bind, or binds with significantly less affinity, LILRA1 and/or LILRA3), such an ABP is not a protein being a natural ligand of LILRB1 and/or LILRB2 (in particular, a ligand protein having more than 70%, 80% or 90% sequence identify to the amino acid sequence of HLA-G).

The term "epitope" includes any determinant capable of being bound by an antigen binding protein, such as an antibody. An epitope is a region of an antigen that is bound by an antigen binding protein that targets that antigen, and when the antigen is a protein, includes specific amino acids that bind the antigen binding protein (such as via an antigen binding domain of said protein). Epitope determinants can include chemically active surface groupings of molecules such as amino acids, sugar side chains, phosphoryl or sulfonyl groups, and can have specific three-dimensional structural characteristics, and/or specific charge characteristics. Generally, antigen binding proteins specific for a particular target antigen will preferentially recognise an epitope on the target antigen in a complex mixture of proteins and/or macromolecules.

The term "extracellular domain" ("ECD" or "EC" domain) as used herein refers to the region or regions of the protein which are exposed to the extracellular space and which are typically responsible for ligand binding. Immunoglobulin (Ig) superfamily genes typically have an Immunoglobulin-like ECD, such as a Ig-like C2-type domain.

An antigen binding protein is "specific" when it binds to one antigen (such as LILRB1 and/or LILRB2; eg human LILRB1 and/or LILRB2, orthologues and other variants thereof) more preferentially (eg, more strongly or more extensively) than it binds to another antigen, preferably an LILRA protein (such as LILRA1 and LILRA3). The term "specifically binds" (or "binds specifically" and the like) used herein in the context of an ABP means that said ABP will preferentially bind to the desired antigen (eg LILRB1 and/or LILRB2, in particular an ECD of LILRB1 and/or LILRB2) than to bind to other proteins (or other molecules), such as preferentially binding to such LILRB1 and/or LILRB2 compared to one or more of a leucocyte immunoglobulin-like subfamily A (LILRBA) proteins. Therefore, preferably, the binding affinity of the ABP to the LILRB antigen (e.g. LILRB1 and/or LILRB2) is at least 2-fold, 5-fold, at least 10-fold, at least 20-fold, at least 50-fold, at least 100-fold, at least 200-fold, at least 500-fold, at least 1000-fold, at least 2000-fold, at least 5000-fold, at least 10000-fold, at least 10⁵-fold or even at least 10⁶-fold, most preferably at least 2-fold, compared to its affinity to the other targets (e.g. unrelated proteins such as mouse or human Fc domain, or streptavidin).

Leukocyte Ig-like receptors (LIRs) are a family of immunoreceptors expressed predominantly on monocytes and B cells and at lower levels on dendritic cells and natural killer (NK) cells. All members of LIR subfamily B, such as LILRB2, contain a cytoplasmic immunoreceptor tyrosine-based inhibitory motif (ITIM) and have an inhibitory function. Upon engagement of members of LIR subfamily B by MHC class I or other ligands and tyrosine phosphorylation of the ΓΓIM, intracellular protein-tyrosine phosphatases, such as SHP1 (PTPN6), are recruited and an inhibitory signal cascade ensues. Most members of LIR subfamily A (e.g., LILRA1) have short cytoplasmic regions that lack ITIMs, have transmembrane regions that contain a charged arginine residue, and can initiate stimulatory cascades. One member of subfamily A, LILRA3, lacks a transmembrane region and is presumed to be a soluble receptor (summary by Borges et al., 1997).

The interaction between LILRB1 and/or LILRB2 and a natural ligand thereof (such as HLA-G) has subsequently been independently described (De Louche et al 2022 JCI Insight. 2022;7(2):e151553).

The human LILRB1 gene is located at chromosomal position 19q13.42, and has orthologues (eg, is conserved) in many species. The term LILRB1 in some embodiments of the invention may also pertain to variants of the human LILRB1 protein having an amino acid sequence that is substantially identical to, or of at least 70%, 75% or 80%, preferably 85%, more preferably at least 90%, 95%, 96%, 97%, 98%, 99%, or 100% sequence identity (such as at least 90% or 95% sequence identity) to, the amino acid sequence shown in any of SEQ ID NOs. 385-389, as determined using, e.g., the "Blast 2 sequences" algorithm described by Tatusova & Madden 1999 (FEMS Microbiol Lett 174: 247-250), and which (preferably) retain biological activity identical or substantially identical to the respective reference LILRB1 (eg to bind to HLA-G) protein and/or to modulate macrophage (or other immune cell) function/activity. The term LILRB1 can mean, as applicable to the context (if not more specifically indicated), an LILRB1 protein (such as one described above) or an mRNA molecule encoding such an LILRB1 protein.

The human LILRB2 gene is located at chromosomal position 19q13.42, and has orthologues (eg, is conserved) in many species. The term LILRB2 in some embodiments of the invention may also pertain to variants of the human LILRB2 protein having an amino acid sequence that is substantially identical to, or of at least 70%, 75% or 80%, preferably 85%, more preferably at least 90%, 95%, 96%, 97%, 98%, 99%, or 100% sequence identity (such as at least 90% or 95% sequence identity) to, the amino acid sequence shown in any of SEQ ID NOs. 390-393, as determined using, e.g., the "Blast 2 sequences" algorithm described by Tatusova & Madden 1999 (FEMS Microbiol Lett 174: 247-250), and which (preferably) retain biological activity identical or substantially identical to the respective reference LILRB1 (eg to bind to HLA-G) protein and/or to modulate macrophage (or other immune cell) function/activity. The term LILRB2 can mean, as applicable to the context (if not more specifically indicated), an LILRB2 protein (such as one described above) or an mRNA molecule encoding such an LILRB2 protein.

In certain of embodiments, an ABP of the invention that binds to the ECD of human LILRB1 and/or LILRB2 protein is cross reactive to the ECD of an orthologous protein, such as cross reactive to the ECD of cynomolgus LILRB1 and/or LILRB2 protein and/or to the ECD of mouse LILRB1 and/or LILRB2 protein and/or to the ECD of rat LILRB1 and/or LILRB2 protein.

The term "orthologue" as used herein means a variant that descends from the same ancestral gene but which is present in another organism due to a speciation event. Orthologues of LILRB1 and/or LILRB2 are typically expected to retain the same function as (or have a similar function to) human LILRB1 and/or LILRB2.

The term "variant" as used herein in the context of a protein means any natural or non-natural version of such protein which comprises one or more amino acid mutations compared to the reference protein, but which shares significant amino acid sequence identity with the reference protein, e.g. at least 70% or 75% amino acid sequence identity, preferably at least 80% amino acid sequence identity, more preferably at least 90% amino acid sequence identity and most preferably at least 95%, 96%, 97%, 98% or 99% amino acid sequence identity. Preferably, the variant of the protein possesses and/or maintains at least one function/activity that is the same, essentially the same or similar as the reference protein. Variants of LILRB1 and/or LILRB2 may include orthologues to and natural variants of human LILRB1 and/or LILRB2. A "functional variant" of LILRB1 and/or LILRB2 (such as a functional fragment of an LILRB1 and/or LILRB2 protein) is a variant of the protein of LILRB1 and/or LILRB2 that provides, possesses and/or maintains one or more of the herein described functions/activities of the non-variant protein of LILRB1 and/or LILRB2. For example, such functional variant may bind HLA-G protein and/or to suppress T cell (or other immune cell) function/activity as LILRB1 and/or LILRB2 protein, such as having the same, essentially the same or similar specificity and/or function as a receptor as LILRB1 and/or LILRB2 protein. In other embodiments, such a functional variant may possess other activities than those possessed by the non-variant LILRB1 and/or LILRB2 protein, as long as, preferably, it provides, possesses and/or maintains at least one function/activity that is the same, essentially the same or similar as LILRB1 and/or LILRB2 protein. In more preferred embodiments, a functional variant of LILRB1 and/or LILRB2 may act as an immune checkpoint inhibitor, such as by inhibiting one or more cell-based immune response(s) to a tumour or cancer cell that expresses such functional variant.

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

In calculating percent identity, the sequences being compared are typically aligned in a way that gives the largest match between the sequences. One example of a computer program that can be used to determine percent identity is the GCG program package, which includes GAP (Devereux et al., 1984, Nucl. Acid Res. 12:387; Genetics Computer Group, University of Wisconsin, Madison, WI). The computer algorithm GAP is used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm.

A standard comparison matrix (see, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSUM 62 comparison matrix) may also be used by the algorithm.

Examples of parameters that can be employed in determining percent identity for polypeptides or nucleotide sequences using the GAP program are the following: (i) Algorithm: Needleman et al., 1970, J. Mol. Biol. 48:443-453; (ii) Comparison matrix: BLOSUM 62 from Henikoff et al., 1992, *supra;* (iii) Gap Penalty: 12 (but with no penalty for end gaps); (iv) Gap Length Penalty: 4; (v) Threshold of Similarity: 0.

A preferred method of determining similarity between a protein or nucleic acid and (or between) human LILRB1 and/or LILRB2, a paralogue, orthologue or other variant thereof, is that provided by the Blast searches supported at Uniprot *supra* (e.g., http://www.uniprot.org/uniprot/Q8NHL6 for LILRB1 and http://www.uniprot.org/uniprot/Q8N423 for LILRB2); in particular for amino acid identity, those using the following parameters: Program: blastp; Matrix: blosum62; Threshold: 10; Filtered: false; Gapped: true; Maximum number of hits reported: 250.

Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least about 10, 15, 20, 25, 30, 35, 40, 45, 50 or other number of contiguous amino acids of the target polypeptide or region thereof.

In particular embodiments of the invention, the LILRB1 is human LILRB1, preferably a protein comprising an amino acid sequence selected from the group consisting of: SEQ ID NO: 385, SEQ ID NO: 386, SEQ ID NO: 387, SEQ ID NO: 388, and SEQ ID NO: 389 (in particular, SEQ ID NO. 385), or a protein having no more than two, four, six, eight, or ten, for example no more than one, two or three, such as no more than one, amino acid substitutions, insertions or deletions compared to these sequences.

In particular embodiments of the invention, the LILRB2 is human LILRB2, preferably a protein comprising an amino acid sequence selected from the group consisting of: SEQ ID NO: 390, SEQ ID NO: 391, SEQ ID NO: 392, and SEQ ID NO: 393 (in particular, SEQ ID NO. 390), or a protein having no more than two, four, six, eight, or ten, for example no more than one, two or three, such as no more than one, amino acid substitutions, insertions or deletions compared to these sequences.

In the context of variants of LILRB1 and/or LILRB2, the invention includes those embodiments where a variant of LILRB1 and/or LILRB2 is a protein comprising an amino acid sequence having at least 80%, 85%, 90%, 92% 95% or 97% sequence identity (in particular, at least 92% or 95% sequence identity) to any one of the sequences of SEQ ID NO: 385-393 (preferably of SEQ ID NO: 353 (LILRB1) or SEQ ID NO 358 (LILRB2)).

In the context of other variants of LILRB1 and/or LILRB2, the invention also includes those embodiments where a variant of LILRB1 and/or LILRB2 is selected from the group consisting of an ortholog (or paralog) of LILRB1 and/or LILRB2, and a functional fragment of an LILRB1 and/or LILRB2 protein. In certain of such embodiments, such functional fragment of an LILRB1 and/or LILRB2 protein binds to a natural ligand of LILRB1 and/or LILRB2 protein, such as a human leucocyte antigen (HLA) type proteins (such as one described elsewhere herein).

An ABP of the invention may, in particular embodiments, be able to inhibit (eg, inhibits) the interaction between a natural ligand of LILRB1 and/or LILRB2 protein and LILRB1 and/or LILRB2 protein (such as for example to HLA-G). For example, the ABP is optionally able to inhibit (eg, inhibits) the binding of LILRB1 and/or LILRB2 protein to a ligand of LILRB1 and/or LILRB2 (such as for example HLA-G).

The protein sequences of the canonical isoform, and further isoforms of the off targets LILRA1 and LILRA3 can also be derived from the UniProt database. For LILRA1, protein sequences and additional information is obtainable under the accession number: 075019. The canonical protein sequence is provided herein as SEQ ID NO: 394. For LILRA1, protein sequences and additional information is obtainable under the accession number: 075019. The canonical protein sequence is provided herein as SEQ ID NO: 395.

As used herein, the term "HLA-G" refers to a protein called human leukocyte antigen G or HLA-G histocompatibility antigen class G, etc. This protein was first discovered in extravillous trophoblasts (EVT) present at the maternal-fetal interface during pregnancy, and is a heterologous material (membrane-bound HLA-G, such as HLA-G1, G2, G3 and G4 are present) that is expressed only in the cellular membrane by selective conjugation of HLA-G mRNA, and has a single molecular form in a soluble state to be secreted to the outside of the cells (HLA-G5, G6 and G7 are present as soluble HLA-G). Human HLA-G amino acid sequences and further information can be derived from the UniProt database in the version of December 2022 with the accession number P17693.

An ABP of the invention may, in particular embodiments, be able to inhibit (eg, inhibits) the interaction between a natural ligand of LILRB1 and LILRB2 protein and LILRB1 and LILRB2 protein (such as for example to HLA-G). For example, the ABP is optionally able to inhibit (eg, inhibits) the binding of LILRB1 and LILRB2 protein to a ligand of LILRB1 and LILRB2 (such as for example HLA-G). The ABP of the invention further does not inhibit the interaction of LILRA1 and LILRA3 with a natural ligand of LILRA1 and/or LILRA3.

### Modulators of LILRB1 and/or LILRB2 expression, function, activity and/or stability

In particular embodiments of such aspect, the ABP is a modulator of the expression, function, activity and/or stability of LILRB1 and/or LILRB2, or the variant of LILRB1 and/or LILRB2, such as wherein the ABP inhibits the expression, function, activity and/or stability of LILRB1 and/or LILRB2, or the variant of LILRB1 and/or LILRB2, or in particular where the ABP is an inhibitor of the function and/or activity of said LILRB1 and/or LILRB2 or the variant of LILRB1 and/or LILRB2. In one of such embodiments, an ABP of the invention is an inhibitor of the interaction between LILRB1 and/or LILRB2, or the variant of LILRB1 and/or LILRB2, to its endogenous receptor or ligand, such as to a HLA type protein, in particular an ABP of the invention is capable of inhibiting (eg, inhibits or is an inhibitor of) the binding of a natural ligand of LILRB1 and/or LILRB2 protein to LILRB1 and/or LILRB2 protein. Accordingly, ABPs of the invention can be "modulators".

The term "modulator" as used herein, refers to a molecule that changes, modifies or alters one or more characteristics, properties and/or abilities of another molecule or, for example, that changes, modifies or alters an immune response ("immunomodulators"), such as a cell-mediated immune response. For example, a modulator (eg, an inhibiting or antagonistic modulator) can impair or interfere with, or cause a decrease in the magnitude of, expression, function, activity and/or stability, such as a certain activity or function, of a molecule compared to the magnitude of such characteristic, property or ability observed in the absence of the modulator. In an alternative example, a modulator (eg, an activating or agonistic modulator) can enhance or promote, or cause an increase in the magnitude of, expression, function, activity and/or stability, such as a certain activity or function, of a molecule compared to the magnitude of such characteristic, property or ability observed in the absence of the modulator. Certain exemplary characteristics, properties or abilities of a molecule include, but are not limited to, expression, function, activity and/or stability, such as binding ability or affinity, enzymatic activity, and signal transduction; for example, any of the functions or activities of LILRB1 and/or LILRB2 described herein.

Modulatory molecules (in particular, modulatory ABPs) can act as "inhibitors" ("antagonists") against a receptor such as LILRB1 and/or LILRB2, such as by impairing (e.g. blocking) ligand engagement to such receptor, eg by inhibiting the interaction between LILRB1 and/or LILRB2 and their ligand. Alternatively, modulatory molecules (in particular, modulatory ABPs) can act as "activators" ("agonists") for a receptor such as LILRB1 and/or LILRB2, such as by enhancing or promoting function and/or activity of such receptor, for example by triggering the receptor's signalling pathway, such as by mimicking the binding of the endogenous ligand for such receptor.

A particular embodiment of a modulator of LILRB1 and/or LILRB2 is an "inhibitor of LILRB1 and/or LILRB2" (or "LILRB1 and/or LILRB2 inhibitor"), which meaning includes any moiety that inhibits LILRB1 and/or LILRB2, which can mean inhibition of the expression (eg the amount), function, activity and/or stability of LILRB1 and/or LILRB2, especially of protein of LILRB1 and/or LILRB2. In one particular of such embodiments, an inhibitor of LILRB1 and/or LILRB2 can reduce the function (and/or activity) of LILRB1 and/or LILRB2 protein, and in another of such embodiments, an inhibitor of LILRB1 and/or LILRB2 can reduce the expression of LILRB1 and/or LILRB2 mRNA and/or protein.

General and specific examples of LILRB1 and/or LILRB2 inhibitors (including those that are ABPs of the present invention) are described elsewhere herein, including those as may be characterised by the applicable functional and/or structural features set out herein.

Accordingly, in particular embodiments of the present invention, an ABP of the invention is one that is capable of specifically binding to (eg which specifically binds to) LILRB1 and/or LILRB2, as well as being capable of inhibiting (eg reducing or blocking) the interaction between LILRB1 and/or LILRB2 protein (or a variant thereof, such as one described above) and its natural ligand (such as a HLA protein, or other ligand). In particular, such an ABP is able to inhibit (eg inhibits) the binding of HLA-G protein (or a variant thereof, such as one described above) to LILRB1 and/or LILRB2 protein (or a variant thereof, such as one described above).

Methodologies to determine the interaction (eg binding) between LILRB1 and/or LILRB2 and a HLA protein such as HLA-G protein (or between variants thereof) are known to the person of ordinary skill, and include ELISA assays (such as described in the examples below), and technologies such as *inter alia*: flow cytometry, surface plasmon resonance, surface acoustic waves and microscale thermophoresis. Such determination methodologies can be used (or adapted) to not only detect the presence of such interaction/binding, but also to measure (eg quantitatively) the degree of binding between the interacting partners LILRB1 and/or LILRB2 and their ligands such as HLA-G proteins (or variants thereof). Such (quantitative) measurement of interaction (binding) may be determined or measured in the presence of a competing (eg inhibiting) ABP of the invention, and hence the potential of an ABP of the present invention to inhibit (eg block) such interaction can be measured, and eg reported as an IC50.

Such IC50 values may be determined, such as using ELISA methodology (eg, using an assay correspond to, or substantially as, the ELISA described in **Example 3),** in the presence of a suitable concentration of HLA-G protein (or variant thereof) in solution and with surface-bound LILRB1 and/or LILRB2.

In certain of such embodiments of the invention, the ABP of the invention (eg one that binds to [one or more epitope(s) displayed by] an extracellular domain(s) of LILRB1 and/or LILRB2, or a paralogue, orthologue or other variant thereof) is capable of inhibiting (eg will inhibit) the binding of a natural ligand of LILRB1 and/or LILRB2 protein or a variant thereof to LILRB1 and/or LILRB2 protein or a variant thereof with an IC50 of 100nM, 50nM, or preferably 20nM or less, such as 15nM or less, 10nM or less, 5nM or less, 2nM or less, 1nM or less, 500pM or less, 250pM or less, or 100pM or less. In particular of such embodiments, an ABP of the invention is capable of inhibiting (eg will inhibit) the binding of HLA-G protein or a variant thereof to LILRB1 and/or LILRB2 protein or a variant thereof with an IC50 of 10nM or less, such as 5nM or less and preferably 2nM or less.

In other embodiments, a modulator of the invention (eg, an ABP that binds to LILRB1 and/or LILRB2) that is an *inhibitor or antagonist* may instead or also:
- inhibit, impair, reduce or reverse LILRB1 and/or LILRB2-mediated inhibition of a cell-mediated immune response (eg in an in-vitro assay or in a subject, such as one in need thereof); and/or
- inhibit, impair, reduce or reverse LILRB1 and/or LILRB2-mediated inhibition of humoral immunity (eg in an in-vitro assay or in a subject, such as one in need thereof).

The term "cell-mediated immune response", as used herein, may include, but is not limited to, a response in a host organism involving, utilising, and/or promoting any one or combinations of T cell maturation, proliferation, activation, migration, infiltration and/or differentiation, and/or the activation/modulation/migration/infiltration of a macrophage, a natural killer cell, a T lymphocyte (or T cell), a helper T lymphocyte, a memory T lymphocyte, a suppressor T lymphocyte, a regulator T lymphocyte, and/or a cytotoxic T lymphocyte (CTL), and/or the production, release, and/or effect of one or more cell-secretable or cell-secreted factor such as a cytokine or autocoid (in particular a pro-inflammatory cytokine), and/or one or more components of any of such processes (such as a cytokine or autocoid, particular a pro-inflammatory cytokine). The term "cell-mediated immune response," as used herein, may include a cellular response involving a genetically engineered, *in-vitro* cultured, autologous, heterologous, modified, and/or transferred T lymphocyte, or it may include a cell-secretable or cell-secreted factor (such as a cytokine or autocoid, in particular a pro-inflammatory cytokine) produced by genetic engineering. A cell-mediated immune response is preferably not a humoral immune response, such as an immune response involving the release of antibodies. In certain embodiments, in particular when the proliferative disorder is a cancer or tumour, the cell-mediated immune response is an anti-tumour cell-mediated immune response. For example, one that leads to a reduction in tumour (cell) growth, such as a cytotoxic cell-mediated immune response (such as a cytotoxic T cell exposure) that kills cells of the cancer or tumour.

In certain embodiments, the cell mediating the cell-mediated immune response may be mediated by a cell, such as an immune cell, capable of secreting (eg secreting) pro-inflammatory cytokine, such as one selected from the group consisting of: interleukin-1 (IL-1), IL-2, IL-12, IL-17 and IL-18, tumour necrosis factor (TNF) [alpha], interferon gamma (IFN-gamma), and granulocyte-macrophage colony stimulating factor.

In certain embodiments, the cell-mediated immune response can be mediated by a pro-inflammatory cytokine-secreting cell, such as a lymphocyte (eg a T cell), in particular a cytotoxic T lymphocyte (CTL), or a natural killer cell (NK cell).

In particular embodiments, the cell-mediated immune response may induce killing of cells associated or involved with a disease, disorder or condition, such as a proliferative disorder (eg a cancer).

The term "humoral immunity" (or "humoral immune response") will also be readily understood by the person of ordinary skill, and includes an aspect of an immune response that is mediated by macromolecules found in extracellular fluids such as secreted antibodies, complement proteins, and certain antimicrobial peptides. Humoral immunity is so named because it involves substances found in the humors, or body fluids. Its aspects involving antibodies can be termed antibody-mediated immunity.

As used herein, a "subject" includes all mammals, including without limitation humans, but also non-human primates such as cynomolgus monkeys. It also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

In further other embodiments, a modulator of the invention (eg, an ABP that binds to LILRB1 and/or LILRB2) that is an *inhibitor or antagonist,* preferably of an interaction of LILRB1 and/or LILRB2 with a natural ligand thereof, such as HLA-G, may instead or also:
- Activate and/or reduce suppression of immune signaling pathways, such as AKT and ERK signaling in a cell expressing LILRB1 and/or LILRB2;
- Reduce the polarization of, or repolarize, immune-suppressive macrophages, such as M2 macrophages;
- Reduce the polarization of, or repolarize, a tolerogenic dendritic cell (DC) phenotype;
- Promote adaptive immune responses, such as cellular or humoral immune responses elicited against an antigen or cell or therapeutic antibody (eg in an in-vitro assay or in a subject, such as one in need thereof);
- promote humoral immune responses elicited by a therapeutic or prophylactic vaccine (eg in an in-vitro assay or in a subject, such as one in need thereof);
- reduce tumour cell immune evasion mediated by HLA-G expression;
- mediate any one or combination of at least one of the following effects: (i) increases immune response, (ii) increases T cell activation, (iii) increases cytotoxic T cell activity, (iv) increases NK cell activity, (v) alleviates T-cell suppression, (vi) increases pro-inflammatory cytokine secretion, (vii) increases IL-2 secretion; (viii) increases interferon-gamma production, (ix) increases Th1 response, (x) decreases Th2 response, (xi) decreases or eliminates cell number and/or activity of at least one of regulatory T cells (Tregs), myeloid derived suppressor cells (MDSCs), iMCs, mesenchymal stromal cells, TIE2-expressing monocytes, (xii) reduces regulatory cell activity, and/or the activity of one or more of myeloid derived suppressor cells (MDSCs), iMCs, mesenchymal stromal cells, TIE2-expressing monocytes, (xiii) decreases or eliminates M2 macrophages, (xiv) reduces M2 macrophage pro-tumorigenic activity, (xv) decreases or eliminates N2 neutrophils, (xvi) reduces N2 neutrophils pro-tumorigenic activity, (xvii) reduces inhibition of T cell activation, (xviii) reduces inhibition of CTL activation, (xix) reduces inhibition of NK cell activation, (xx) reverses T cell exhaustion, (xxi) increases T cell response, (xxii) increases activity of cytotoxic cells, (xxiii) stimulates antigen-specific memory responses, (xxiv) elicits apoptosis or lysis of cancer cells, (xxv) stimulates cytotoxic or cytostatic effect on cancer cells, (xxvi) induces direct killing of cancer cells, (xxvii) increases Th17 activity and/or (xxviii) induces complement dependent cytotoxicity and/or antibody dependent cell-mediated cytotoxicity; (eg in an in-vitro assay or in a subject, such as one in need thereof) with the optional proviso that said modulator may elicit an opposite effect to one or more of (i)-(xxviii).

### ABPs of the invention comprising one or more complementarity determining regions

In particular embodiments, an ABP of the invention can preferentially comprise at least one complementarity determining region (CDR), such as one from an antibody (in particular from a human antibody), and in particular embodiments the ABP can comprise a CDR having an amino acid sequence with at least 80%, 85%, 90% or 95% sequence identity to (preferably, at least 90% sequence identity to), or having no more than three or two, preferably no more than one amino acid substitution(s), deletion(s) or insertion(s) compared to, a CDR sequence set forth in **Table 1** herein.

The term "complementarity determining region" (or "CDR" or "hypervariable region"), as used herein, refers broadly to one or more of the hyper-variable or complementarily determining regions (CDRs) found in the variable regions of light or heavy chains of an antibody. See, for example: "IMGT", Lefranc et al, 20003, Dev Comp Immunol 27:55; Honegger & Plückthun, 2001, J Mol Biol 309:657, Abhinandan & Martin, 2008, Mol Immunol 45:3832, Kabat, et al. (1987): Sequences of Proteins of Immunological Interest National Institutes of Health, Bethesda, Md. These expressions include the hypervariable regions as defined by Kabat et al (1983) Sequences of Proteins of Immunological Interest, US Dept of Health and Human Services, or the hypervariable loops in 3-dimensional structures of antibodies (Chothia and Lesk, 1987; J Mol Biol 196:901). The CDRs in each chain are held in close proximity by framework regions and, with the CDRs from the other chain, contribute to the formation of the antigen-binding site. Within the CDRs there are select amino acids that have been described as the selectivity determining regions (SDRs) which represent the critical contact residues used by the CDR in the antibody-antigen interaction. (Kashmiri, 2005; Methods 36:25).

In all of its aspects and embodiments, a framework sequence of an ABP of the invention may include one or more mutations, for example in order to improve the isoelectric point of the molecules. One preferred mutation of the ABP of the invention are at position E81, for example is a E81M mutation, in accordance with the Kabat nomenclature.

As described above, in particular embodiments of the invention, an ABP can comprise at least one complementarity determining region (CDR). In certain of such embodiments, an ABP of the invention comprises at least one complementarity determining region 3 (CDR3), such as one having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from those heavy and light chain CDR3 sequences shown in **Table 1** (eg, a sequence selected from the list consisting of SEQ ID Nos: 3, 7, 11, 15, 19, 23, 27, 31, 35, 39, 43, 47, 51, 55, 59, 63, 67, 71, 75, 79, 83, 87, 91, 95, 99, 103, 107, 111, 115, 119, 123, 127, 131, 135, 139, 143, 147, 151, 155, 159, 163, 167, 171, 175, 179, 183, 187, 191, 195, 199, 203, 207, 211, 215, 219, 223, 227, 231, 235, 239, 243, 247, 251, 255, 259, 263, 267, 271, 275, 279, 283, 287, 291, 295, 299, 303, 307, 311, 315, 319, 323, 327, 331, 335, 339, 343, 347, 351, 355, 359, 363, 367, 371, 375, 379, and 383).

An ABP of the invention may, alternatively or as well as a CDR3 sequence, comprise at least one CDR1, and/or at least one CDR2 (such as one from an antibody, in particular from a human antibody). Preferably, and ABP of the invention comprises at least one such CDR3, as well as at least one such CDR1 and at least one such CDR2, more preferably where each of such CDRs having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from the corresponding (heavy and light chain) CDR1, CD2 and CD3 sequences shown in **Table 1.**

In certain preferred embodiments, the antibodies of the invention are derived from three parental antibody sequences denoted as A-001, A-002 and A-003. As such, the invention shows an inventive binding profile for the parental sequences, but also for maturated derivative antibody heavy and/or light chain sequences. The invention therefore in specific embodiments also pertains to antibody chain combination of any light chain sequence with any heavy chain sequence, of an antibody of the invention.

In particular embodiments, an ABP of the invention can be an antibody or an antigen binding fragment thereof.

As used herein, the term "antibody" may be understood in the broadest sense as any immunoglobulin (Ig) that enables binding to its epitope. An antibody as such is a species of an ABP. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (C1q) of the classical complement system. Other forms of antibodies include heavy-chain antibodies, being those which consist only of two heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies.

Antibodies (or those from which fragments thereof can be isolated) can include, for instance, chimeric, humanized, (fully) human, or hybrid antibodies with dual or multiple antigen or epitope specificities, antibody fragments and antibody sub-fragments, e.g., Fab, Fab' or F(ab')2 fragments, single chain antibodies (scFv) and the like (described below), including hybrid fragments of any immunoglobulin or any natural, synthetic or genetically engineered protein that acts like an antibody by binding to a specific antigen to form a complex.

Accordingly, in certain embodiments an ABP of the invention can comprise an antibody heavy chain, or an antigen binding fragment thereof, and/or an antibody light chain, or an antigen binding fragment thereof.

In further embodiments, an ABP of the invention can comprise an antibody heavy chain variable region, or an antigen binding fragment thereof, and/or an antibody light chain variable region, or an antigen binding fragment thereof, and in yet further embodiments, an ABP of the invention can comprise an antibody heavy chain variable region CDR1, CDR2, and CDR3, and/or an antibody light chain variable region CDR1, CDR2, and CDR3.

In particular embodiments of the invention, when the ABP comprises an antibody heavy chain sequence and/or an antibody light chain sequence, or an antigen binding fragment thereof; the antibody heavy chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those heavy chain CDR3 sequences shown in **Table 1** (eg, a sequence selected from the list consisting of SEQ ID Nos: 3, 11, 19, 27, 35, 43, 51, 59, 67, 75, 83, 91, 99, 107, 115, 123, 131, 139, 147, 155, 163, 171, 179, 187, 195, 203, 211, 219, 227, 235, 243, 251, 259, 267, 275, 283, 291, 299, 307, 315, 323, 331, 339, 347, 345, 363, 371 and 379), and/or wherein antibody light chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those light chain CDR3 sequences shown in **Table 1** (eg, a sequence selected from the list consisting of SEQ ID Nos: 7, 15, 23, 31, 39, 47, 55, 63, 71, 79, 87, 95, 103, 111, 119, 127, 135, 143, 151, 159, 167, 175, 183, 191, 199, 207, 215, 223, 231, 239, 247, 255, 263, 271, 279, 287, 295, 303, 311, 319, 327, 335, 343, 351, 359, 367, 375, and 383).

In particular embodiments of the invention, when the ABP comprises an antibody heavy chain sequence and/or an antibody light chain sequence, or an antigen binding fragment thereof; the antibody heavy chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those heavy chain CDR3 sequences selected from the list consisting of SEQ ID Nos: 75, 115, and 195, and/or wherein antibody light chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to a CDR3 sequence selected from those light chain CDR3 sequences selected from the list consisting of SEQ ID Nos: 287, 311, and 319. Any combination of these heavy chain CDR3 and light chain CDR3 are included in ABP of the invention, in particular the combinations selected from SEQ ID NO: 75 and 287, SEQ ID NO: 115 and 287, SEQ ID NO: 195 and 311 and SEQ ID NO: 195 and 319.

In further embodiments of the invention, when the ABP comprises an antibody heavy chain, or an antigen binding fragment thereof, the antibody heavy chain sequence, or the fragment thereof, can further comprise a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 1, 9, 17, 25, 33, 41, 49, 57, 65, 73, 81, 89, 97, 105, 113, 121, 129, 137, 145, 153, 161, 169, 177, 185, 193, 201, 209, 217, 225, 233, 241, 249, 257, 265, 273, 281, 289, 297, 305, 313, 321, 329, 337, 345, 353, 361, 369, and 377(eg a heavy chain CDR1 sequence disclosed in **Table** 1); and/or a CDR2 having at 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 2, 10, 18, 26, 34, 42, 50, 58, 66, 74, 82, 90, 98, 106, 114, 122, 130, 138, 146, 154, 162, 170, 178, 186, 194, 202, 210, 218, 226, 234, 242, 250, 258, 266, 274, 282, 290, 298, 306, 314, 322, 330, 338, 346, 354, 362, 370, and 378(eg a CDR2 sequence disclosed in **Table 1).**

In yet further embodiments of the present invention, an ABP of the invention comprises an antibody light chain, or an antigen binding fragment thereof, wherein the antibody light chain sequence, or the fragment thereof, further comprises a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 5, 13, 21, 29, 37, 45, 53, 61, 69, 77, 85, 93, 101, 109, 117, 125, 133, 141, 149, 157, 165, 173, 181, 189, 197, 205, 213, 221, 229, 237, 245, 253, 261, 269, 277, 285, 293, 301, 309, 317, 325, 333, 341, 349, 357, 365, 373, and 381(eg a light chain CDR1 sequence disclosed in **Table 1)**; and/or a CDR2 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 6, 14, 22, 30, 38, 46, 54, 62, 70, 78, 86, 94, 102, 110, 118, 126, 134, 142, 150, 158, 166, 174, 182, 190, 198, 206, 214, 222, 230, 238, 246, 254, 262, 270, 278, 286, 294, 302, 310, 318, 326, 334, 342, 350, 358, 366, 374, and 382(eg a light chain CDR2 sequence disclosed in **Table 1).**

In other embodiments of the present invention, an ABP of the invention can comprise an antibody variable chain sequence having at least 80%, 85%, 90%; or 95% (preferably at least 90%) sequence identity to, or having no more than ten, nine, eight, seven, six, five, four, three, two or one, preferably no more than three, two or one amino acid substitution(s), deletion(s) or insertion(s) compared to, a sequence selected from SEQ ID NOs. 4, 8, 12, 16, 20, 24, 28, 32, 36, 40, 44, 48, 52, 56, 60, 64, 68, 72, 76, 80, 84, 88, 92, 96, 100, 104, 108, 112, 116, 120, 124, 128, 132, 136, 140, 144, 148, 152, 156, 160, 164, 168, 172, 176, 180, 184, 188, 192, 196, 200, 204, 208, 212, 216, 220, 224, 228, 232, 236, 240, 244, 248, 252, 256, 260, 264, 268, 272, 276, 280, 284, 288, 292, 296, 300, 304, 308, 312, 316, 320, 324, 328, 332, 336, 340, 344, 348, 352, 356, 360, 364, 368, 372, 376, 380, and 384(eg, a VH or VL sequence disclosed in **Table 1).**

In particular embodiments of the invention, an ABP of the invention comprises an antigen binding fragment of an antibody, wherein the antigen binding fragment comprises CDR1, CDR2 and CDR3. In certain of such embodiments, the CDR1 is selected from those disclosed in **Table 1,** the CDR2 is selected from those disclosed in **Table 1** and the CDR3 is selected from those disclosed in **Table 1** (eg, the CDR1, CDR2 and CDR3 are selected from the CDR1, CDR2 and CDR3 sequences having the respective amino acid sequences of SEQ ID Nos. 1, 2, 3; or 9, 10, 11; or 17, 18, 19; or 25, 26, 27; or 33, 34, 35; or 41, 42, 43; or 49, 50, 51; or 57, 58, 59; or 65, 66, 67; or 73, 74, 75; or 81, 82, 83; or 89, 90, 91; or 97, 98, 99; or 105, 106, 107; or 113, 114, 115; or 121, 122, 123; or 129, 130, 131; or 137, 138, 139; or 145, 146, 147; or 153, 154, 155; or 161, 162, 163; or 169, 170, 171; or 177, 178, 179; or 185, 186, 187; or 193, 194, 195; or 201, 202, 203; or 209, 210, 211; or 217, 218, 219; or 225, 226, 227; or 233, 234, 235; or 241, 242, 243; or 249, 250, 251; or 257, 258, 259; or 265, 266, 267; or 273, 274, 275; or 281, 282, 283; or 289, 290, 291; or 297, 298, 299; or 305, 306, 307; or 313, 314, 315; or 321, 322, 323; or 329, 330, 331; or 337, 338, 339; or 345, 346, 347; or 5, 6, 7; or 13, 14, 15; or 21, 22, 23; or 29, 30, 31; or 37, 38, 39; or 45, 46, 47; or 53, 54, 55; or 61, 62, 63; or 69, 70, 71; or 77, 78, 79; or 85, 86, 87; or 93, 94, 95; or 101, 102, 103; or 109, 110, 111; or 117, 118, 119; or 125, 126, 127; or 133, 134, 135; or 141, 142, 143; or 149, 150, 151; or 157, 158, 159; or 165, 166, 167; or 173, 174, 175; or 181, 182, 183; or 189, 190, 191; or 197, 198, 199; or 205, 206, 207; or 213, 214, 215; or 221, 222, 223; or 229, 230, 231; or 237, 238, 239; or 245, 246, 247; or 253, 254, 255; or 261, 262, 263; or 269, 270, 271; or 277, 278, 279; or 285, 286, 287; or 293, 294, 295; or 301, 302, 303; or 309, 310, 311; or 317, 318, 319; or 325, 326, 327; or 333, 334, 335; or 341, 342, 343; or 349, 350, 351; or 353, 354, 355; or 357, 358, 359; or 361, 362, 363; or 365, 366, 367;or 369, 370, 371; or 373; 374, 375; or 377, 378, 379; or 381, 382, 383); in each case independently, optionally with no more than three or two, preferably no more than one, amino acid substitution(s), insertion(s) or deletion(s) compared to these sequences.

In further particular embodiments of the present invention, an ABP of the invention can comprise an antibody heavy chain variable region CDR1, CDR2, and CDR3, and/or an antibody light chain variable region CDR1, CDR2, and CDR3, wherein the CDR1 has an amino acid sequence of a heavy or light chain CDR1 shown in **Table 1** (eg has an amino acid sequence selected from the list consisting of SEQ ID No 1, 5, 9, 13, 17, 21, 25, 29, 33, 37, 41, 45, 49, 53, 57, 61, 65, 69, 73, 77, 81, 85, 89, 93, 97, 101, 105, 109, 113, 117, 121, 125, 129, 133, 137, 141, 145, 149, 153, 157, 161, 165, 169, 173, 177, 181, 185, 189, 193, 197, 201, 205, 209, 213, 217, 221, 225, 229, 233, 237, 241, 245, 249, 253, 257, 261, 265, 269, 273, 277, 281, 285, 289, 293, 297, 301, 305, 309, 313, 317, 321, 325, 329, 333, 337, 341, 345, 349, 353, 357, 361, 365, 369, 373, 377, and 381), and wherein the CDR2 has an amino acid sequence of a heavy or light chain CDR2 shown in **Table 1** (eg has an amino acid sequence selected from the list consisting of SEQ ID No 2, 6, 10, 14, 18, 22, 26, 30, 34, 38, 42, 46, 50, 54, 58, 62, 66, 70, 74, 78, 82, 86, 90, 94, 98, 102, 106, 110, 114, 118, 122, 126, 130, 134, 138, 142, 146, 150, 154, 158, 162, 166, 170, 174, 178, 182, 186, 190, 194, 198, 202, 206, 210, 214, 218, 222, 226, 230, 234, 238, 242, 246, 250, 254, 258, 262, 266, 270, 274, 278, 282, 286, 290, 294, 298, 302, 306, 310, 314, 318, 322, 326, 330, 334, 338, 342, 346, 350, 354, 358, 362, 366, 370, 374, 378, 382), and wherein the CDR3 has an amino acid sequence of a heavy or light chain CDR3 shown in **Table 1** (eg has an amino acid sequence selected from the list consisting of SEQ ID No 3, 7, 11, 15, 19, 23, 27, 31, 35, 39, 43, 47, 51, 55, 59, 63, 67, 71, 75, 79, 83, 87, 91, 95, 99, 103, 107, 111, 115, 119, 123, 127, 131, 135, 139, 143, 147, 151, 155, 159, 163, 167, 171, 175, 179, 183, 187, 191, 195, 199, 203, 207, 211, 215, 219, 223, 227, 231, 235, 239, 243, 247, 251, 255, 259, 263, 267, 271, 275, 279, 283, 287, 291, 295, 299, 303, 307, 311, 315, 319, 323, 327, 331, 335, 339, 343, 347, 351, 355, 359, 363, 367, 371, 375, 379, and 383); in each case independently, optionally with no more than three or two, preferably no more than one, amino acid substitution(s), insertion(s) or deletion(s) compared to these sequences.

In preferred of such embodiments, the ABP may be an antibody, or an antigen binding fragment thereof, composed of at least one, preferably two, antibody heavy chain sequences, and at least one, preferably two, antibody light chain sequences, wherein at least one, preferably both, of the antibody heavy chain sequences and at least one, preferably both, of the antibody light chain sequences comprise CDR1 to CDR3 sequences in a combination selected from any of the combinations of heavy chain CDRs shown in **Table B** and/or selected from any of the combinations of light chain CDRs shown in **Table B** (in each case, combinations CDRs-A-001 to CDRs-A-044); in each case independently, optionally with no more than three or two, preferably no more than one, amino acid substitution(s), insertion(s) or deletion(s) compared to these sequences. Preferably, the combination of both the heavy chain CDRs and the light chain CDRs is one selected from a row marked by any one of the combinations CDRs-A-001 to CDRs-A-044, in each CDR independently optionally with no more than three or two, preferably no more than one, amino acid substitution(s), insertion(s) or deletion(s) compared to these sequences.

**Table B: preferred combinations of heavy chain CDRs and preferred combinations of light chain CDRs**

| **Combination (ID)** | **Heavy Chain CDR1 to CDR3 (SEQ ID NO)** | | | **Light Chain CDR1 to CDR3 (SEQ ID NO)** | | |
|---|---|---|---|---|---|---|
| CDRs-A-001 | 1 | 2 | 3 | 5 | 6 | 7 |
| CDRs-A-002 | 9 | 10 | 11 | 13 | 14 | 15 |
| CDRs-A-003 | 17 | 18 | 19 | 21 | 22 | 23 |
| CDRs-A-004 | 25 | 26 | 27 | 29 | 30 | 31 |
| CDRs-A-005 | 33 | 34 | 35 | 37 | 38 | 39 |
| CDRs-A-006 | 41 | 42 | 43 | 45 | 46 | 47 |
| CDRs-A-007 | 49 | 50 | 51 | 53 | 54 | 55 |
| CDRs-A-008 | 57 | 58 | 59 | 61 | 62 | 63 |
| CDRs-A-009 | 65 | 66 | 67 | 69 | 70 | 71 |
| CDRs-A-010 | 73 | 74 | 75 | 77 | 78 | 79 |
| CDRs-A-011 | 81 | 82 | 83 | 85 | 86 | 87 |
| CDRs-A-012 | 89 | 90 | 91 | 93 | 94 | 95 |
| CDRs-A-013 | 97 | 98 | 99 | 101 | 102 | 103 |
| CDRs-A-014 | 105 | 106 | 107 | 109 | 110 | 111 |
| CDRs-A-015 | 113 | 114 | 115 | 117 | 118 | 119 |
| CDRs-A-016 | 121 | 122 | 123 | 125 | 126 | 127 |
| CDRs-A-017 | 129 | 130 | 131 | 133 | 134 | 135 |
| CDRs-A-018 | 137 | 138 | 139 | 141 | 142 | 143 |
| CDRs-A-019 | 145 | 146 | 147 | 149 | 150 | 151 |
| CDRs-A-020 | 153 | 154 | 155 | 157 | 158 | 159 |
| CDRs-A-021 | 161 | 162 | 163 | 165 | 166 | 167 |
| CDRs-A-022 | 169 | 170 | 171 | 173 | 174 | 175 |
| CDRs-A-023 | 177 | 178 | 179 | 181 | 182 | 183 |
| CDRs-A-024 | 185 | 186 | 187 | 189 | 190 | 191 |
| CDRs-A-025 | 193 | 194 | 195 | 197 | 198 | 199 |
| CDRs-A-026 | 201 | 202 | 203 | 205 | 206 | 207 |
| CDRs-A-027 | 209 | 210 | 211 | 213 | 214 | 215 |
| CDRs-A-028 | 217 | 218 | 219 | 221 | 222 | 223 |
| CDRs-A-029 | 225 | 226 | 227 | 229 | 230 | 231 |
| CDRs-A-030 | 233 | 234 | 235 | 237 | 238 | 239 |
| CDRs-A-031 | 241 | 242 | 243 | 245 | 246 | 247 |
| CDRs-A-032 | 249 | 250 | 251 | 253 | 254 | 255 |
| CDRs-A-033 | 257 | 258 | 259 | 261 | 262 | 263 |
| CDRs-A-034 | 265 | 266 | 267 | 269 | 270 | 271 |
| CDRs-A-035 | 273 | 274 | 275 | 277 | 278 | 279 |
| CDRs-A-036 | 281 | 282 | 283 | 285 | 286 | 287 |
| CDRs-A-037 | 289 | 290 | 291 | 293 | 294 | 295 |
| CDRs-A-038 | 297 | 298 | 299 | 301 | 302 | 303 |
| CDRs-A-039 | 305 | 306 | 307 | 309 | 310 | 311 |
| CDRs-A-040 | 313 | 314 | 315 | 317 | 318 | 319 |
| CDRs-A-041 | 321 | 322 | 323 | 325 | 326 | 327 |
| CDRs-A-042 | 329 | 330 | 331 | 333 | 334 | 335 |
| CDRs-A-043 | 337 | 338 | 339 | 341 | 342 | 343 |
| CDRs-A-044 | 345 | 346 | 347 | 349 | 350 | 351 |
| CDRs-A-045 | 353 | 354 | 355 | 357 | 358 | 359 |
| CDRs-A-046 | 361 | 362 | 363 | 365 | 366 | 367 |
| CDRs-A-047 | 369 | 370 | 371 | 373 | 374 | 375 |
| CDRs-A-048 | 377 | 378 | 379 | 381 | 382 | 383 |

In other preferred embodiments of the invention, the ABP may be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown in **Table C** (eg, selected from any of the variable chain combinations Chains-A-001 to Chains-A-044); in each case independently, optionally with no more than ten, nine, eight, seven, six, five, four, preferably no more than three, two or one, amino acid substitution(s), insertion(s) or deletion(s) compared to these sequences.

**Table C: preferred combinations of heavy and light chain variably domains**

| **Combination (ID)** | **Heavy Chain Variable Domain (SEQ ID NO)** | **Light Chain Variable Domain (SEQ ID NO)** |
|---|---|---|
| Chains-A-001 | 4 | 8 |
| Chains-A-002 | 12 | 16 |
| Chains-A-003 | 20 | 24 |
| Chains-A-004 | 28 | 32 |
| Chains-A-005 | 36 | 40 |
| Chains-A-006 | 44 | 48 |
| Chains-A-007 | 52 | 56 |
| Chains-A-008 | 60 | 64 |
| Chains-A-009 | 68 | 72 |
| Chains-A-010 | 76 | 80 |
| Chains-A-011 | 84 | 88 |
| Chains-A-012 | 92 | 96 |
| Chains-A-013 | 100 | 104 |
| Chains-A-014 | 108 | 112 |
| Chains-A-015 | 116 | 120 |
| Chains-A-016 | 124 | 128 |
| Chains-A-017 | 132 | 136 |
| Chains-A-018 | 140 | 144 |
| Chains-A-019 | 148 | 152 |
| Chains-A-020 | 156 | 160 |
| Chains-A-021 | 164 | 168 |
| Chains-A-022 | 172 | 176 |
| Chains-A-023 | 180 | 184 |
| Chains-A-024 | 188 | 192 |
| Chains-A-025 | 196 | 200 |
| Chains-A-026 | 204 | 208 |
| Chains-A-027 | 212 | 216 |
| Chains-A-028 | 220 | 224 |
| Chains-A-029 | 228 | 232 |
| Chains-A-030 | 236 | 240 |
| Chains-A-031 | 244 | 248 |
| Chains-A-032 | 252 | 256 |
| Chains-A-033 | 260 | 264 |
| Chains-A-034 | 268 | 272 |
| Chains-A-035 | 276 | 280 |
| Chains-A-036 | 284 | 288 |
| Chains-A-037 | 292 | 296 |
| Chains-A-038 | 300 | 304 |
| Chains-A-039 | 308 | 312 |
| Chains-A-040 | 316 | 320 |
| Chains-A-041 | 324 | 328 |
| Chains-A-042 | 332 | 336 |
| Chains-A-043 | 340 | 344 |
| Chains-A-044 | 348 | 352 |
| Chains-A-045 | 356 | 360 |
| Chains-A-046 | 364 | 368 |
| Chains-A-047 | 372 | 376 |
| Chains-A-048 | 380 | 384 |

The ABP of the invention are all characterized by an advantageous and surprising on-target and off-target binding affinity profile. Hence, the present invention is some embodiments pertain to ABPs binding to LILRB1 and LILRB2, while not binding to LILRA1 and not binding to LILRA3. More specifically, the ABP of the invention is an ABP wherein the binding of the ABP to any of the on target LILRB1 or LILRB2 is at least 2 or 3 times higher than to any of the off targets LILRA1 or LILRA3, preferably wherein the binding affinity is measured by BLI in KD (monovalent) under conditions set out in the example section of this disclosure. More preferably the invention provides for the first time, advantageous binding profiles, wherein the binding of the ABP to any of the on target LILRB1 or LILRB2 is at least 5, or preferably 10 times higher than to any of the off targets LILRA1 or LILRA3, preferably wherein the binding affinity is measured by BLI in KD (monovalent) under conditions set out in the example section of this disclosure. In addition thereto, in some embodiments, the ABP has a variable heavy chain sequence that is at least 90%, preferably 95%, 96%, 97%, 98% or 99% identical to the variable heavy chain sequence shown in any one of ABPs A-001 to A-048; and/or wherein, the ABP has a variable light chain sequence that is at least 90%, preferably 95%, 96%, 97%, 98% or 99% identical to the variable light chain sequence shown in any one of ABPs A-001 to A-048.

In particularly preferred embodiment, an ABP of the invention can comprise a combination of heavy chain CDR1, CDR2 and CDR3 sequences and a combination of light chain CDR1, CDR2 and CDR3 sequences in the combination shown by antibody A-010, such as shown in **Table B** by row CDRs-A-010 (eg, heavy chain CDR1, CDR2 and CDR2 having a sequence shown by SEQ ID Nos, 73, 74 and 75, respectively, and light chain CDR1, CDR2 and CDR2 having a sequence shown by SEQ ID Nos, 77, 78 and 79, respectively), in each CDR independently, optionally with no more than three or two, preferably no more than one, amino acid substitution(s), insertion(s) or deletion(s) compared to these sequences. In another particularly preferred embodiment, an ABP of the invention can be an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-010 and at least one, preferably both, of the antibody light chain sequences each comprises light chain CDR1 to CDR3 sequences in the combination shown in the row of **Table B** marked by CDRs-A-010, in each CDR independently, optionally with no more than one amino acid substitution(s), insertion(s) or deletion(s) compared to these sequences. In yet another particularly preferred embodiment, an ABP of the invention can be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown the row of **Table B** marked by Chains-A-010. In each of such particularly preferred embodiments of the ABP, optionally, the ABP is able to inhibit the binding of HLA-G protein or a variant thereof to LILRB1 and/or LILRB2 protein or a variant thereof with an IC50 of 20nM or less or 10nM or less, such as 5nM or less, or preferably 2nM or less. Such IC50s can be determined using the methods described elsewhere herein.

In particularly preferred embodiment, an ABP of the invention can comprise a combination of heavy chain CDR1, CDR2 and CDR3 sequences and a combination of light chain CDR1, CDR2 and CDR3 sequences in the combination shown by antibody A-010, such as shown in **Table B** by row CDRs-A-026 (eg, heavy chain CDR1, CDR2 and CDR2 having a sequence shown by SEQ ID Nos, 201, 202 and 203, respectively, and light chain CDR1, CDR2 and CDR2 having a sequence shown by SEQ ID Nos 205, 206 and 207, respectively), in each CDR independently, optionally with no more than three or two, preferably no more than one, amino acid substitution(s), insertion(s) or deletion(s) compared to these sequences. In another particularly preferred embodiment, an ABP of the invention can be an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-026 and at least one, preferably both, of the antibody light chain sequences each comprises light chain CDR1 to CDR3 sequences in the combination shown in the row of **Table B** marked by CDRs-A-026, in each CDR independently, optionally with no more than one amino acid substitution(s), insertion(s) or deletion(s) compared to these sequences. In yet another particularly preferred embodiment, an ABP of the invention can be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown the row of **Table B** marked by Chains-A-026. In each of such particularly preferred embodiments of the ABP, optionally, the ABP is able to inhibit the binding of HLA-G protein or a variant thereof to LILRB1 and/or LILRB2 protein or a variant thereof with an IC50 of 20nM or less or 10nM or less, such as 5nM or less, or preferably 2nM or less. Such IC50s can be determined using the methods described elsewhere herein.

In particularly preferred embodiment, an ABP of the invention can comprise a combination of heavy chain CDR1, CDR2 and CDR3 sequences and a combination of light chain CDR1, CDR2 and CDR3 sequences in the combination shown by antibody A-045, such as shown in **Table B** by row CDRs-A-045 (eg, heavy chain CDR1, CDR2 and CDR2 having a sequence shown by SEQ ID Nos, 353, 354, and 355, respectively, and light chain CDR1, CDR2 and CDR2 having a sequence shown by SEQ ID Nos, 357, 358, and 359, respectively), in each CDR independently, optionally with no more than three or two, preferably no more than one, amino acid substitution(s), insertion(s) or deletion(s) compared to these sequences. In another particularly preferred embodiment, an ABP of the invention can be an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-045 and at least one, preferably both, of the antibody light chain sequences each comprises light chain CDR1 to CDR3 sequences in the combination shown in the row of **Table B** marked by CDRs-A-045, in each CDR independently, optionally with no more than one amino acid substitution(s), insertion(s) or deletion(s) compared to these sequences. In yet another particularly preferred embodiment, an ABP of the invention can be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown the row of **Table B** marked by Chains-A-045. In each of such particularly preferred embodiments of the ABP, optionally, the ABP is able to inhibit the binding of HLA-G protein or a variant thereof to LILRB1 and/or LILRB2 protein or a variant thereof with an IC50 of 20nM or less or 10nM or less, such as 5nM or less. Such IC50s can be determined using the methods described elsewhere herein (see Example 3). Preferably, the ABP of this embodiment has a binding affinity to LILRB1 and/or LILRB2 as measured in KD (monovalent) of less than 20nM, more preferably of less than 10nM more preferably of less than 5nM, wherein the binding affinity ranges are provided preferably for both binding to LILRB1 and LILRB2, as measured using BLI under conditions as described in the examples. Hence, the ABP of this embodiment is preferably a LILRB1 and LILRB2 ABP. In another preferred embodiment of the invention such antibody is further characterized by a significantly lower binding affinity to the counter targets LILRA1 and LILRA3, wherein the binding is with less affinity compared to the binding to LILRB1 and/or LILRB2. Preferably the binding affinity as measured in KD (monovalent) of such ABP of this embodiment to LILRA1 and LILRA3 is higher than 50 nM, more preferably higher 100nm, more preferably higher than 200 nM, and most preferably higher than 400 nM, also as measured using BLI under conditions described herein in the example section.

In particularly preferred embodiment, an ABP of the invention can comprise a combination of heavy chain CDR1, CDR2 and CDR3 sequences and a combination of light chain CDR1, CDR2 and CDR3 sequences in the combination shown by antibody A-046, such as shown in **Table B** by row CDRs-A-046 (eg, heavy chain CDR1, CDR2 and CDR2 having a sequence shown by SEQ ID Nos, 361, 362, and 363, respectively, and light chain CDR1, CDR2 and CDR2 having a sequence shown by SEQ ID Nos, 365,366, and 367, respectively), in each CDR independently, optionally with no more than three or two, preferably no more than one, amino acid substitution(s), insertion(s) or deletion(s) compared to these sequences. In another particularly preferred embodiment, an ABP of the invention can be an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-046 and at least one, preferably both, of the antibody light chain sequences each comprises light chain CDR1 to CDR3 sequences in the combination shown in the row of **Table B** marked by CDRs-A-046, in each CDR independently, optionally with no more than one amino acid substitution(s), insertion(s) or deletion(s) compared to these sequences. In yet another particularly preferred embodiment, an ABP of the invention can be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown the row of **Table B** marked by Chains-A-046. In each of such particularly preferred embodiments of the ABP, optionally, the ABP is able to inhibit the binding of HLA-G protein or a variant thereof to LILRB1 and/or LILRB2 protein or a variant thereof with an IC50 of 20nM or less or 10nM or less, such as 5nM or less. Such IC50s can be determined using the methods described elsewhere herein (see Example 3). Preferably, the ABP of this embodiment has a binding affinity to LILRB1 and/or LILRB2 as measured in KD (monovalent) of less than 100nM, more preferably of less than 50nM more preferably of less than 15nM, wherein the binding affinity ranges are provided preferably for both binding to LILRB1 and LILRB2, as measured using BLI under conditions as described in the examples. Hence, the ABP of this embodiment is preferably a LILRB1 and LILRB2 ABP. In another preferred embodiment of the invention such antibody is further characterized by a significantly lower binding affinity to the counter targets LILRA1 and LILRA3, wherein the binding is with less affinity compared to the binding to LILRB1 and/or LILRB2. Preferably the binding affinity as measured in KD (monovalent) of such ABP of this embodiment to LILRA1 and LILRA3 is higher than 100 nM, more preferably higher 500nm, more preferably higher than 1000 nM, and most preferably higher than 2000 nM, also as measured using BLI under conditions described herein in the example section.

In particularly preferred embodiment, an ABP of the invention can comprise a combination of heavy chain CDR1, CDR2 and CDR3 sequences and a combination of light chain CDR1, CDR2 and CDR3 sequences in the combination shown by antibody A-047, such as shown in **Table B** by row CDRs-A-047 (eg, heavy chain CDR1, CDR2 and CDR2 having a sequence shown by SEQ ID Nos, 369, 370, and 371, respectively, and light chain CDR1, CDR2 and CDR2 having a sequence shown by SEQ ID Nos, 373,374, and 375, respectively), in each CDR independently, optionally with no more than three or two, preferably no more than one, amino acid substitution(s), insertion(s) or deletion(s) compared to these sequences. In another particularly preferred embodiment, an ABP of the invention can be an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-047 and at least one, preferably both, of the antibody light chain sequences each comprises light chain CDR1 to CDR3 sequences in the combination shown in the row of **Table B** marked by CDRs-A-047, in each CDR independently, optionally with no more than one amino acid substitution(s), insertion(s) or deletion(s) compared to these sequences. In yet another particularly preferred embodiment, an ABP of the invention can be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown the row of **Table B** marked by Chains-A-047. In each of such particularly preferred embodiments of the ABP, optionally, the ABP is able to inhibit the binding of HLA-G protein or a variant thereof to LILRB1 and/or LILRB2 protein or a variant thereof with an IC50 of 20nM or less or 10nM or less, such as 5nM or less. Such IC50s can be determined using the methods described elsewhere herein (see Example 3). Preferably, the ABP of this embodiment has a binding affinity to LILRB1 and LILRB2 as measured in KD (monovalent) of less than 20nM, more preferably of less than 10nM more preferably of less than 5nM, wherein the binding affinity ranges are provided preferably for both binding to LILRB1 and LILRB2, as measured using BLI under conditions as described in the examples. Hence, the ABP of this embodiment is preferably a LILRB1 and LILRB2 ABP. In another preferred embodiment of the invention such antibody is further characterized by a significantly lower binding affinity to the counter targets LILRA1 and LILRA3, wherein the binding is with less affinity compared to the binding to LILRB1 and/or LILRB2. Preferably the binding affinity as measured in KD (monovalent) of such ABP of this embodiment to LILRA1 and LILRA3 is higher than 20 nM, more preferably higher 50nm, more preferably higher than 100 nM, also as measured using BLI under conditions described herein in the example section.

In particularly preferred embodiment, an ABP of the invention can comprise a combination of heavy chain CDR1, CDR2 and CDR3 sequences and a combination of light chain CDR1, CDR2 and CDR3 sequences in the combination shown by antibody A-048, such as shown in **Table B** by row CDRs-A-048 (eg, heavy chain CDR1, CDR2 and CDR2 having a sequence shown by SEQ ID Nos, 377, 378, and 379, respectively, and light chain CDR1, CDR2 and CDR2 having a sequence shown by SEQ ID Nos, 381,382, and 383, respectively), in each CDR independently, optionally with no more than three or two, preferably no more than one, amino acid substitution(s), insertion(s) or deletion(s) compared to these sequences. In another particularly preferred embodiment, an ABP of the invention can be an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-048 and at least one, preferably both, of the antibody light chain sequences each comprises light chain CDR1 to CDR3 sequences in the combination shown in the row of **Table B** marked by CDRs-A-048, in each CDR independently, optionally with no more than one amino acid substitution(s), insertion(s) or deletion(s) compared to these sequences. In yet another particularly preferred embodiment, an ABP of the invention can be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown the row of **Table B** marked by Chains-A-048. In each of such particularly preferred embodiments of the ABP, optionally, the ABP is able to inhibit the binding of HLA-G protein or a variant thereof to LILRB1 and/or LILRB2 protein or a variant thereof with an IC50 of 20nM or less or 10nM or less, such as 5nM or less. Such IC50s can be determined using the methods described elsewhere herein (see Example 3). Preferably, the ABP of this embodiment has a binding affinity to LILRB1 and LILRB2 as measured in KD (monovalent) of less than 100nM, more preferably of less than 50nM more preferably of less than 10nM, wherein the binding affinity ranges are provided preferably for both binding to LILRB1 and LILRB2, as measured using BLI under conditions as described in the examples. Hence, the ABP of this embodiment is preferably a LILRB1 and LILRB2 ABP. In another preferred embodiment of the invention such antibody is further characterized by a significantly lower binding affinity to the counter targets LILRA1 and LILRA3, wherein the binding is with less affinity compared to the binding to LILRB1 and/or LILRB2. Preferably the binding affinity as measured in KD (monovalent) of such ABP of this embodiment to LILRA1 and LILRA3 is higher than 50 nM, more preferably higher 100nm, more preferably higher than 500 nM, and most preferably higher than 1000 nM, also as measured using BLI under conditions described herein in the example section.

The ABP of the invention are preferably ABP that show the advantageous on target off target profile as shown in the example section. In particular, the invention pertains to an ABP that is cross-specific for a binding to LILRB1 and LILRB2, but that does not bind to, or binds significantly less to LILRA1 and/or LILRA2. Hence, the ABP of the invention are characterized by maintaining cross-specificity, while not being able to bind to their off targets in the LILRA receptor family, specifically not to, or significantly less to, LILRA1 and LILRA3.

Specific embodiments of the ABP of the invention also pertain to an ABP, wherein the ABP binds to the ECD of LILRB1 and LILRB2 with at least 5 times higher, or preferably 10 times higher binding affinity compared the binding of the ABP to an ECD of LILRA1 and/or LILRA3, preferably wherein the ABP binding affinity is determined by bio-layer interferometry (BLI).

Further, in some additional or alternative embodiments, the ABP of the invention binds to the ECD of LILRB1 and/or LILRB2 with a binding dissociation constant (KD) that is at least 2 times lower, preferably 3 times lower, more preferably 5 times lower, most preferably 10 times lower, compared to the binding KD of the ABP to an ECD of LILRA1 and/or LILRA3, wherein the KD is determined by bio-layer interferometry (BLI), preferably under the conditions set out in example 3 herein below.

Yet another embodiment of the invention pertains to an ABP, wherein the ABP binds to an ECD of LILRB1 and LILRB2 with a dissociation constant (KD) that is at least 2 times lower, preferably 3 times lower, more preferably 5 times lower, most preferably 10 times lower, compared to the KD of the ABP to an ECD of LILRA1 and LILRA3, wherein the KD is determined by bio-layer interferometry (BLI), preferably under the conditions set out in example 3.

Specific embodiments of the invention pertain to such antibodies with the above characterized binding profile to LILRB1 and LILRB2 as well as LILRA1 and LILRA3 (non-binging thereof, or lower binding), wherein such ABP of the invention comprises one, preferably two, an antibody heavy chain sequence and one, preferably two, antibody light chain sequence, and wherein the antibody heavy chain sequence and the antibody light chain sequence are derived from one or a combination of the antibody parental clones A-001 to A-003, preferably from A-001 and/or A-003. Further preferably, the ABP comprises comprises one, preferably two, an antibody heavy chain variable sequence and one, preferably two, antibody light chain variable sequence, wherein the antibody heavy and light chain variable sequence each comprise a sequence that is at least 90%, preferably at least 95%, more preferably at least 96%, 97%, 98%, 99% identical to an antibody heavy or light chain variable sequence shown for any of the parental antibody sequences A-001 to A-003, preferably A-001 and/or A-003 (see **Table 1** herein below).

In further embodiments of the invention, the ABP of the invention is preferred in embodiments, wherein the ABP competes for binding to an ECD of LILRB1 and/or LILRB2, or to ECD of the variant of LILRB1 and/or LILRB2, with an endogenous LILRB1 and/or LILRB2 ligand or receptor, preferably wherein said endogenous LILRB1 and/or LILRB2 ligand or receptor is a HLA-G protein (or a variant of HLA-G)

Specifically, such ABPs of the invention are preferred that are characterized in that the lowest affinity selected from the two binding affinities of the group consisting of the binding affinity of the ABP to the ECD of LILRB1 and the binding affinity of the ABP to the ECD ofLILRB2,such lowest binding affinity is still at least 2 times higher than, or preferably at least 3 times higher (in some embodiments at least 5 times higher, more preferably at least 10 times higher) than the highest binding affinity out of the binding affinities of the ABP to an ECD of the off targets such as LILRA1 and LILRA3. In other words, an ABP is preferred that is characterized in that the highest KD with respect to the KDs of such ABP to LILRB1 and LILRB2 is still 2 times lower, preferably 3 times lower (in some embodiments at least 5 times lower, more preferably at least 10 times lower) than the lowest KD out of the two KDs of such ABP to LILRA1 and LILRA3.

Further aspects and embodiments of ABPs of the invention

In **a second aspect,** the invention relates to **an ABP which competes with** an ABP of a first aspect for binding to LILRB1 and/or LILRB2 protein (eg to the ECD of LILRB1 and/or LILRB2 protein) or variant thereof, in particular can relate to an ABP that competes with one of the particularly preferred ABPs described above for binding to the LILRB1 and/or LILRB2 protein or variant.

The term "compete" when used in the context of ABPs (e.g., modulator ABPs) that compete for binding for the same antigen (or epitope displayed by such antigen) means competition between ABPs as may be determined by an assay in which the ABP (e.g., antibody or binding fragment thereof) being tested prevents or inhibits (e.g., reduces) binding of a reference ABP (e.g., a ligand, or a reference antibody) to a common antigen (e.g., LILRB1 and/or LILRB2 or a fragment thereof such as an ECD of LILRB1 and/or LILRB2).

In **a related aspect,** the invention relates to **an ABP which binds to the same epitope** as an ABP of a first aspect.

ABPs of a second aspect of the invention may include one or more features (or specific combinations thereof) of the ABPs described above. In particular, an ABP of a second aspect of the invention may be capable of inhibiting (eg inhibits) the binding of a natural ligand of LILRB1 and/or LILRB2 protein, such as described in more details above, and/or an ABP of a second aspect of the invention may modulate the expression, function, activity and/or stability of LILRB1 and/or LILRB2, or the variant of LILRB1 and/or LILRB2 (such as in anyway described elsewhere herein).

In particular embodiments of the invention, as well as (or instead of) an ABP of the invention's capability to inhibit (eg block) the interaction between LILRB1 and/or LILRB2 protein or a variant thereof, and binding of a natural ligand of LILRB1 and/or LILRB2 protein; an ABP of the invention (including those of a first or second aspect as above) may display, exhibit or otherwise possess other functional features, in particular those which are associated with their utility in sensitising cells to a cell-mediated immune response.

In further of such particular embodiments, an ABP of the invention is capable of antagonizing immune-suppressive macrophage polarization, such as a polarization into M2 macrophages, and thereby to enhance cell-mediated immune responses. Such enhancement can be assessed, for example, using a suitable assay such as one described in **Example 6** hereof.

The term "immune cell" is art recognised to describe any cell of an organism involved in the immune system of such organism, in particular of a mammal such as a human. Leukocytes (white blood cells) are immune cells that are involved in the innate immune system, and the cells of the adaptive immune system are special types of leukocytes, known as lymphocytes. B cells and T cells are the major types of lymphocytes and are derived from hematopoietic stem cells in the bone marrow. B cells are involved in the humoral immune response, whereas T cells are involved in cell-mediated immune response. In preferred embodiments of the invention, the immune cell can be a myeloid cell eg a T cell, and in particular (such as when an increase in cell-mediated immune response is required, such as to treat a cancer) the T cell can be a cytotoxic T cell (also known as TC, cytotoxic T lymphocyte, CTL, T-killer cell, cytolytic T cell, CD8+ T-cell or killer T cell). A CTL is a T-cell that is involved in the killing of cancer cells, cells that are infected (particularly with viruses), or cells that are damaged in other ways. Other preferred immune cells for such embodiments can include Tumour-Infiltrating Lymphocytes (TILs). TILs are white blood cells that have left the bloodstream and migrated into a tumour. Typically, TILs are a mix of different types of cells (i.e., T cells, B cells, NK cells) in variable proportions, T cells being the most abundant cells. TILs can often be found in the stroma and within the tumour itself, and are implicated in killing tumour cells. The presence of lymphocytes in tumours is often associated with better clinical outcomes.

Other particular functional characteristics of an ABP of the invention may be that of: (i) enhancing a cell-mediated immune response, such as that mediated by an activated cytotoxic T-cell (CTL), to a mammalian cell expressing said LILRB1 and/or LILRB2 or the variant of LILRB1 and/or LILRB2; and/or (ii) increasing immune cell, such as T-cell, activity and/or survival (and/or proliferation) in the presence of a mammalian cell expressing said LILRB1 and/or LILRB2 or the variant of LILRB1 and/or LILRB2. In some embodiments, the mammalian cell expressing the LILRB1 and/or LILRB2 may be a cell associated with a disease, disorder or condition such as a cancer cell being (directly) associated with the cancer. In other the mammalian cell expressing the LILRB1 and/or LILRB2 may be an immune cell, such as a T cell (see below), for example an immune cell that is directly or indirectly associated with the disease, disorder or condition.

Other particular functional characteristics of an ABP of the invention that is an *inhibitor or antagonist* of LILRB1 and/or LILRB2 expression, function, activity and/or stability can be any one, or a combination or at least one, functional characteristic of the inhibiting or antagonistic modulators described herein, in particular in the section above "Modulators of LILRB1 and/or LILRB2 expression, function, activity and/or stability".

Those particular functional characteristics of an ABP of the invention that is an *activator or agonist* of LILRB1 and/or LILRB2 expression, function, activity and/or stability can be any one, or a combination or at least one, functional characteristic of the activating or agonistic modulators described herein, in particular in the section above "Modulators of LILRB1 and/or LILRB2 expression, function, activity and/or stability".

In preferred embodiments of all ABPs of the invention, the ABP is isolated and/or substantially pure.

The term "isolated" as used herein in the context of a protein, such as an ABP (an example of which could be an antibody), refers to a protein that is purified from proteins or polypeptides or other contaminants that would interfere with its therapeutic, diagnostic, prophylactic, research or other use. An isolated ABP according to the invention may be a recombinant, synthetic or modified (non-natural) ABP. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated ABP or nucleic acid or cells is/are substantially pure. In this context, a "recombinant" protein or nucleic acid is one made using recombinant techniques. Methods and techniques for the production of recombinant nucleic acids and proteins are well known in the art.

The term "isolated" as used herein in the context of a protein, such as an ABP (an example of which could be an antibody), refers to a protein that is purified from proteins or polypeptides or other contaminants that would interfere with its therapeutic, diagnostic, prophylactic, research or other use. An isolated ABP according to the invention may be a recombinant, synthetic or modified (non-natural) ABP. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated ABP or nucleic acid or cells is/are substantially pure. In this context, a "recombinant" protein or nucleic acid is one made using recombinant techniques. Methods and techniques for the production of recombinant nucleic acids and proteins are well known in the art.

In some embodiments, an ABP of the invention may bind to (e.g., via one or more epitope(s) displayed by one or more EC domain(s) of) LILRB1 and/or LILRB2 or a paralogue, orthologue or other variant thereof (such as any LILRB1 and/or LILRB2 or variant described herein) with a KD that is less than 20nM, such as less than about 10nM, 5nM or 2nM (in particular, less than about 1 nM). In a preferred embodiment, the ABP of the invention will bind (e.g. said epitope(s) of) said LILRB1 and/or LILRB2 or variant with a KD that is less than 100 pM. In a more preferred embodiment, the ABP of the invention will bind said LILRB1 and/or LILRB2 or variant with a KD that is less than 10 pM. In a most preferred embodiment, the ABP of the invention will bind said LILRB1 and/or LILRB2 or variant with a KD that is less than 2 pM. Binding of an ABP of the invention, such as an antibody of the invention, to a human cell line expressing said LILRB1 and/or LILRB2 or variant may, in some embodiments, occur at an EC50 of less than about 10µg/mL, 5µg/mL, 2µg/mL, 1µg/mL, 0.5µg/mL or 0.2µg/mL, preferably with an EC50 of less than 2µg/mL. Binding of an ABP of the invention, such as an antibody of the invention, to a Cynomolgus cell line expressing an orthologue of said LILRB1 and/or LILRB2 or variant may, in some embodiments, occur at an EC50 of less than about 10µg/mL, 5µg/mL, 2µg/mL, 1µg/mL, 0.5µg/mL or 0.2µg/mL, preferably with an EC50 of less than 2µg/mL.

In other embodiments, an ABP of the invention may: (i) bind to the LILRB1 and/or LILRB2, or to the variant of LILRB1 and/or LILRB2, with a KD that is less than 20nM, such as less than about 10nM, 5nM or 2nM (in particular, less than about 1 nM), is less than 100 pM, or is less than 10 pM; and/or (ii) binds to a human cell line expressing the LILRB1 and/or LILRB2 or the variant of LILRB1 and/or LILRB2 with an EC50 of less than 2ug/mL.

In some embodiments, an ABP of the invention may not bind to LILRB counter targets LILRAs, such as specifically not to LILRA1 and LILRA3, or a orthologue or other variant thereof. In alternative embodiments, the ABP of the invention binds to one or more, or any of, a LILRA protein (preferably an ECD thereof) with a KD that at least 2 fold higher than the KD of its binding to LILRB1 and/or LILRB2, more preferably with a KD that is at least 3 fold, 4 fold 5 or 10 fold higher. In other such embodiments, the ABP of the invention binds to an LILRA protein with a KD of more than 100nM, preferably more than 50nm, even more preferably of more than 20nM.

The term "KD", as used herein, is intended to refer to the dissociation constant, which is obtained from the ratio of Kd to Ka (i. e., Kd/Ka) and is expressed as a molar concentration (M). KD values for antibodies can be determined using methods well established in the art such as plasmon resonance (BIAcore^{®}), ELISA and KINEXA. A preferred method for determining the KD of an antibody is by using surface plasmon resonance, preferably using a biosensor system such as a BIAcore^{®} system or by ELISA. Another preferred method of the present invention to determine KD values for ABPs of the invention is by bio-layer interferometry (BLI), such as using the Octet RedO system. Conditions for measuring binding affinities of the ABP of the invention can be derived from the example section herein. "Ka" (or "K-assoc"), as used herein, refers broadly to the association rate of a particular antibody-antigen interaction, whereas the term "Kd" (or "K-diss"), as used herein, refers to the dissociation rate of a particular antibody-antigen interaction.

In yet other embodiments, an ABP of the invention may compete for binding to LILRB1 and/or LILRB2, or to the variant of LILRB1 and/or LILRB2, with an endogenous LILRB1 and/or LILRB2 ligand or receptor, preferably wherein said endogenous LILRB1 and/or LILRB2 ligand or receptor is HLA-G (or a variant of HLA-G). For example, in certain of such embodiments, the ABP of the invention (eg one that binds to [one or more epitope(s) displayed by] an extracellular domain(s) of LILRB1 and/or LILRB2, or a paralogue, orthologue or other variant thereof) is capable of inhibiting (eg will inhibit) the binding of HLA-G protein or a variant thereof to LILRB1 and/or LILRB2 protein or a variant thereof with an IC50 of 100nM or less, 50nM or less, or preferably 20nM or less, such as 15nM or less, 10nM or less, 5nM or less, 2nM or less, 1nM or less. In particular of such embodiments, an ABP of the invention is capable of inhibiting (eg will inhibit) the binding of HLA-G protein or a variant thereof to LILRB1 and/or LILRB2 protein or a variant thereof with an IC50 of 10nM or less, such as 5nM or less and preferably 2nM or less.

In one embodiment, an ABP of the invention is a polyclonal antibody (mixture), or the antigen binding fragment is a fragment of a polyclonal antibody (mixture).

In an alternative, and preferred, embodiment of all ABPs of the invention, the ABP is an antibody or an antigen binding fragment thereof, and the antibody is a monoclonal antibody, or wherein the antigen binding fragment is a fragment of a monoclonal antibody.

The term "monoclonal antibody" or "mAb" as used herein refers to an antibody obtained from a population of substantially identical antibodies based on their amino acid sequence. Monoclonal antibodies are typically highly specific. Furthermore, in contrast to conventional (polyclonal) antibody preparations which typically include different antibodies directed against different determinants (e.g. epitopes) of an antigen, each mAb is typically directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized or human antibodies or antibody fragments.

Monoclonal antibodies in accordance with the present invention may be prepared by methods well known to those skilled in the art. For example, mice, rats or rabbits may be immunized with an antigen of interest together with adjuvant. Splenocytes are harvested as a pool from the animals that are administered several immunisations at certain intervals with test bleeds performed to assess for serum antibody titers. Splenocytes are prepared that are either used immediately in fusion experiments or stored in liquid nitrogen for use in future fusions. Fusion experiments are then performed according to the procedure of Stewart & Fuller, J. Immunol. Methods 1989, 123:45-53. Supernatants from wells with growing hybrids are screened by eg enzyme-linked immunosorbent assay (ELISA) for mAb secretors. ELISA-positive cultures are cloned either by limiting dilutions or fluorescence-activated cell sorting, typically resulting in hybridomas established from single colonies. The ability of an antibody, including an antibody fragment or sub-fragment, to bind to a specific antigen can be determined by binding assays known in the art, for example, using the antigen of interest as the binding partner.

In a further preferred embodiment, an ABP of the invention is an antibody or an antigen binding fragment thereof, wherein the antibody is a human antibody a humanised antibody or a chimeric-human antibody, or wherein the antigen binding fragment is a fragment of a human antibody a humanised antibody or a chimeric-human antibody.

Human antibodies can also be derived by in vitro methods. Suitable examples include but are not limited to phage display (CAT, Morphosys, Dyax, Biosite/Medarex, Xoma, Yumab, Symphogen, Alexion, Affimed) and the like. In phage display, a polynucleotide encoding a single Fab or Fv antibody fragment is expressed on the surface of a phage particle (see e.g., Hoogenboom et al., J. Mol. Biol., 227: 381 (1991); Marks et al., J Mol Biol 222: 581 (1991); U.S. Patent No. 5,885,793). Phage are "screened" to identify those antibody fragments having affinity for target. Thus, certain such processes mimic immune selection through the display of antibody fragment repertoires on the surface of filamentous bacteriophage, and subsequent selection of phage by their binding to target. In certain such procedures, high affinity functional neutralizing antibody fragments are isolated. A complete repertoire of human antibody genes may thus be created by cloning naturally rearranged human V genes from peripheral blood lymphocytes (see, e.g., Mullinax et al., Proc Natl Acad Sci (USA), 87: 8095-8099 (1990)) or by generating fully synthetic or semi-synthetic phage display libraries with human antibody sequences (see Knappik et al 2000; J Mol Biol 296:57; de Kruif et al, 1995; J Mol Biol 248):97).

The antibodies described herein may alternatively be prepared through the utilization of the XenoMouse^{®} technology. Such mice are capable of producing human immunoglobulin molecules and antibodies and are deficient in the production of murine immunoglobulin molecules and antibodies. In particular, a preferred embodiment of transgenic production of mice and antibodies is disclosed in U.S. Patent Application Serial No. 08/759,620, filed December 3, 1996 and International Patent Application Nos. WO 98/24893, published June 11, 1998 and WO 00/76310, published December 21, 2000. See also Mendez et al., Nature Genetics, 15:146-156 (1997). Through the use of such technology, fully human monoclonal antibodies to a variety of antigens have been produced. Essentially, XenoMouse^{®} lines of mice are immunized with an antigen of interest. e.g. LILRB1 and/or LILRB2, lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produce antibodies specific to the antigen of interest. Other "humanised" mice are also commercially available: eg, Medarex - HuMab mouse, Kymab - Kymouse, Regeneron - Velocimmune mouse, Kirin -TC mouse, Trianni -Trianni mouse, OmniAb - OmniMouse, Harbour Antibodies - H2L2 mouse, Merus - MeMo mouse. Also are available are "humanised" other species: rats: OmniAb - OmniRat, OMT - UniRat. Chicken: OmniAb - OmniChicken.

The term "humanised antibody" according to the present invention refers to immunoglobulin chains or fragments thereof (such as Fab, Fab', F(ab')2, Fv, or other antigen-binding sub-sequences of antibodies), which contain minimal sequence (but typically, still at least a portion) derived from non-human immunoglobulin. For the most part, humanised antibodies are human immunoglobulins (the recipient antibody) in which CDR residues of the recipient antibody are replaced by CDR residues from a non-human species immunoglobulin (the donor antibody) such as a mouse, rat or rabbit having the desired specificity, affinity and capacity. As such, at least a portion of the framework sequence of said antibody or fragment thereof may be a human consensus framework sequence. In some instances, Fv framework residues of the human immunoglobulin need to be replaced by the corresponding non-human residues to increase specificity or affinity. Furthermore, humanised antibodies can comprise residues which are found neither in the recipient antibody nor in the imported CDR or framework sequences. These modifications are made to further refine and maximise antibody performance. In general, the humanised antibody will comprise substantially all of at least one, and typically at least two, variable domains, in which all or substantially all of the CDR regions correspond to those of a non-human immunoglobulin and all or substantially all of the framework regions are those of a human immunoglobulin consensus sequence. The humanised antibody optimally also will comprise at least a portion of an immunoglobulin constant region, typically that of a human immunoglobulin, which (eg human) immunoglobulin constant region may be modified (eg by mutations or glycoengineering) to optimise one or more properties of such region and/or to improve the function of the (eg therapeutic) antibody, such as to increase or reduce Fc effector functions or to increase serum half-life. Exemplary such Fc modification (for example, Fc engineering or Fc enhancement) are described elsewhere herein.

The term "chimeric antibody" according to the present invention refers to an antibody whose light and/or heavy chain genes have been constructed, typically by genetic engineering, from immunoglobulin variable and constant regions which are identical to, or homologous to, corresponding sequences of different species, such as mouse and human. Alternatively, variable region genes derive from a particular antibody class or subclass while the remainder of the chain derives from another antibody class or subclass of the same or a different species. It covers also fragments of such antibodies. For example, a typical therapeutic chimeric antibody is a hybrid protein composed of the variable or antigen-binding domain from a mouse antibody and the constant or effector domain from a human antibody, although other mammalian species may be used.

In particular of such embodiments, an ABP of the invention comprises an antigen binding domain of an antibody wherein the antigen binding domain is of a human antibody. Preferably, ABP comprises an antigen binding domain of an antibody or an antigen binding fragment thereof, which is a human antigen binding domain; (ii) the antibody is a monoclonal antibody, or wherein the antigen binding fragment is a fragment of a monoclonal antibody; and (iii) the antibody is a human antibody or a humanised antibody, or wherein the antigen binding fragment is a fragment of a human antibody, a humanised antibody or a chimeric-human antibody.

Light chains of human antibodies generally are classified as kappa and lambda light chains, and each of these contains one variable region and one constant domain. Heavy chains are typically classified as mu, delta, gamma, alpha, or epsilon chains, and these define the antibody's isotype as IgM, IgD, IgG, IgA, and IgE, respectively. Human IgG has several subtypes, including, but not limited to, IgG1, IgG2, IgG3, and IgG4. Human IgM subtypes include IgM, and IgM2. Human IgA subtypes include IgA1 and IgA2. In humans, the IgA and IgD isotypes contain four heavy chains and four light chains; the IgG and IgE isotypes contain two heavy chains and two light chains; and the IgM isotype contains ten or twelve heavy chains and ten or twelve light chains. Antibodies according to the invention may be IgG, IgE, IgD, IgA, or IgM immunoglobulins.

In some embodiments, the ABP of the invention is an IgG antibody or fragment thereof. In some embodiments, the ABP of the invention is an IgE antibody or fragment thereof. In some embodiments, the ABP of the invention is an IgD antibody or fragment thereof. In some embodiments, the ABP of the invention is an IgA antibody or fragment thereof. In some embodiments, the ABP of the invention is an IgM antibody or fragment thereof. Preferably the ABP of the invention is, comprises or is derived from an IgG immunoglobulin or fragment thereof; such as a human, human-derived IgG immunoglobulin, or a rabbit- or rat-derived IgG, and/or an IgG2 immunoglobulin, or fragment thereof. When the ABP of the invention is, comprises or is derived from a rat-derived IgG, then preferably, the ABP is, comprises or is derived from, a rat IgG2a or IgG2b immunoglobulin. When the ABP of the invention is, comprises or is derived from a human-derived IgG, then more preferably, the ABP of the invention is, comprises or is derived from a human IgG1, IgG2 or IgG4, most preferably, the ABP of the invention is, comprises or is derived from a human IgG1 or IgG2

Accordingly, in particular embodiments of the invention, an ABP is an antibody wherein the antibody is an IgG, IgE, IgD, IgA, or IgM immunoglobulin; preferably an IgG immunoglobulin.

An ABP of the invention, where comprising at least a portion of an immunoglobulin constant region (typically that of a human immunoglobulin) may have such (eg human) immunoglobulin constant region modified - for example eg by glycoengineering or mutations - to optimise one or more properties of such region and/or to improve the function of the (eg therapeutic) antibody, such as to increase or reduce Fc effector functions or to increase serum half-life.

ABPs of the invention, in particular those useful in the present methods include antibodies that induce antibody-dependent cytotoxicity (ADCC) of LILRB1 and/or LILRB2-expressing cells. The ADCC of an anti-LILRB1 and/or LILRB2 antibody can be improved by using antibodies that have low levels of or lack fucose. Antibodies lacking fucose have been correlated with enhanced ADCC (antibody- dependent cellular cytotoxicity) activity, especially at low doses of antibody (Shields et ah, 2002, J. Biol. Chem. 277:26733-26740; Shinkawa et ah, 2003, J. Biol. Chem. 278:3466).

Methods of preparing fucose-less antibodies or antibodies with reduced fucose levels include growth in rat myeloma YB2/0 cells (ATCC CRL 1662). YB 2/0 cells express low levels of FUT8 mRNA, which encodes an enzyme (.alpha. 1,6- fucosyltransferase) necessary for fucosylation of polypeptides.

Alternatively, during the expression of such antibodies, an inhibitor against an enzyme relating to the modification of a sugar chain may be used, including: tunicamycin which selectively inhibits formation of GicNAc-P-P-Dol which is the first step of the formation of a core oligosaccharide which is a precursor of an N-glycoside-linked sugar chain, castanospermin and W-methyl-1-deoxynojirimycin which are inhibitors of glycosidase I, kifunensine which is an inhibitor of mannosidase I, bromocondulitol which is an inhibitor of glycosidase II, 1 - deoxynojirimycin and 1,4-dioxy-1,4-imino-D-mannitol which are inhibitors of mannosidase I, swainsonine which is an inhibitor of mannosidase II and the like. Examples of an inhibitor specific for a glycosyltransferase include deoxy derivatives of substrates against N-acetylglucosamine transferase V (GnTV) and the like. Also, it is known that 1 -deoxynojirimycin inhibits synthesis of a complex type sugar chain and increases the ration of high mannose type and hybrid type sugar chains (Glycobiology series 2 -Destiny of Sugar Chain in Cell, edited by Katsutaka Nagai, Senichiro Hakomori and Akira Kobata, 1993).

Based on these data, several cell lines have been genetically engineered to produce antibodies containing no or low levels of fucose (Mori et al, 2004; Yamane-Ohnuki et al., 2004) to engineer the glycosylation patterns of IgG in order to select therapeutic monoclonal antibodies exhibiting particular profiles of Fc-gamma-R engagement that could be used in various pathologies.

Umana et al. and Davis et al. showed that an IgG1 antibody engineered to contain increasing amounts of bisected complex oligosaccharides (bisecting A/-acetylglucosamine, GlcNAC) allows triggering a strong ADCC as compared to its parental counterpart (Umana et al., 1999; Davies et al., 2001 ). Second, a lack of fucose on human IgG1 N-linked oligosaccharides has been shown to improve FCGRIII binding and ADCC.

GLYCART BIOTECHNOLOGY AG (Zurich, CH) has expressed N-acetyl-glucosaminyltransferase III (GnTIII) which catalyses the addition of the bisecting GlcNac residue to the N-linked oligosaccharide, in a Chinese hamster ovary (CHO) cell line, and showed a greater ADCC of IgG1 antibody produced (WO 99/54342; WO 03/01 1878; WO 2005/044859).

WO20070166306 is related to the modification of an antibody anti-CD19 containing 60% N-acetylglucosamine bisecting oligosaccharides and 10% non-fucosylated N-acetylglucosamine bisecting oligosaccharides produced in a mammalian human 293T embryonal kidney cells transfected with (i) the cDNA for the anti-CD19 antibody and (ii) the cDNA for the GnTIII enzyme.

Recombinant human IgG1 produced in YB2/0 cells (Shinkawa et al., 2003; Siberil et al., 2006) or in CHO-Lec13 (Shields et al., 2002) which exhibited a low-fucose content or were deficient in fucose as compared to the same IgG1 produced in wild-type CHO cells, showed an enhanced ability to trigger cellular cytotoxicity. By contrast, a correlation between galactose and ADCC was not observed and the content of bisecting GlcNAC only marginally affected ADCC (Shinkawa et al., 2003).

By removing or supplanting fucose from the Fc portion of the antibody, KYOWA HAKKO KOGYO (Tokyo, Japan) has enhanced Fc binding and improved ADCC, and thus the efficacy of the MAb (US 6,946,292). This improved Fc-gamma-RIIIA-dependent effector functions of low-fucosylated IgG has been shown to be independent from Fc-gamma-RIII allelic form (Niwa et al., 2005). Moreover, it has been recently shown that the antigenic density required to induce an efficient ADCC is lower when the IgG has a low content in fucose as compared to a highly fucosylated IgG (Niwa et al., 2005)

The Laboratoire Francais du Fractionnement et des Biotechnologies (LFB) (France) showed that the ratio Fuc/Gal in MAb oligosaccharide should be equal or lower than 0.6 to get antibodies with a high ADCC (FR 2 861 080).

Cardarelli et al., 2019 produce an anti-CD19 antibody in Ms-704PF CHO cells deficient in the FUT8 gene which encodes alphal-1 ,6-fucosyltransferase. Non-fucosylation of the antibody in this paper requires the engineering of an enzyme-deficient cell line. This paper does not consider amino acid mutations.

Herbst et al. generated a humanized IgG1 MAb MEDI-551 expressed in a fucosyltransferase-deficient producer CHO cell line This paper does not consider amino acid mutations (Herbst et al., 2010). [0049] S. Siberil et al used the rat myeloma YB2/0 cell line to produce a MAb anti RhD with a low fucose content. Whereas the MAb produced in a wild type CHO exhibited a high fucose content (81 %), the same MAb produced in YB2/0 cell exhibiited a lower fucose content (32%). This paper does consider amino acid mutations (Siberil et al., 2006).

Accordingly, an ABP of the invention may be prepared and/or may have one or more of the characteristics of such glycoengineering (eg afucosylated) approaches/antibodies described above.

Alternative methods for increasing ADDC activity for an ABP of the invention include mutations in an Fc portion of such ABP, particularly mutations which increase antibody affinity for an Fc-gamma-R receptor.

Accordingly, any of the ABPs of the invention described above can be produced with different antibody isotypes or mutant isotypes to control the extent of binding to different Fc-gamma receptors. Antibodies lacking an Fc region (e.g., Fab fragments) lack binding to different Fc-gamma receptors. Selection of isotype also affects binding to different Fc-gamma receptors. The respective affinities of various human IgG isotypes for the three different Fc-gamma receptors, Fc-gamma-RI, Fc- gamma-RII, and Fc- gamma-RIII, have been determined. (See Ravetch & Kinet, Annu. Rev. Immunol. 9, 457 (1991)). Fc- gamma-RI is a high affinity receptor that binds to IgGs in monomeric form, and the latter two are low affinity receptors that bind IgGs only in multimeric form. In general, both IgG1 and IgG3 have significant binding activity to all three receptors, IgG4 to Fc-gamma-RI, and IgG2 to only one type of Fc-gamma-RII called IIaLR (see Parren et al., J. Immunol. 148, 695 (1992). Therefore, human isotype IgG1 is usually selected for stronger binding to Fc-gamma receptors, and IgG2 or IgG4 is usually selected for weaker binding.

A correlation between increased Fc-gamma-R binding with mutated Fc has been demonstrated using targeted cytoxicity cell-based assays (Shields et ah, 2001, J. Biol. Chem. 276:6591-6604; Presta et ah, 2002, Biochem Soc. Trans. 30:487-490). Methods for increasing ADCC activity through specific Fc region mutations include the Fc variants comprising at least one amino acid substitution at a position selected from the group consisting of: 234, 235, 239, 240, 241, 243, 244, 245, 247, 262, 263, 264, 265, 266, 267, 269, 296, 297, 298, 299, 313, 325, 327, 328, 329, 330 and 332, wherein the numbering of the residues in the Fc region is that of the EU index as in Kabat (Kabat et ah, Sequences of Proteins of Immunological Interest (National Institute of Health, Bethesda, Md. 1987).

In certain specific embodiments, said Fc variants comprise at least one substitution selected from the group consisting of L234D, L234E, L234N, L234Q, L234T, L234H, L234Y, L234I, L234V, L234F, L235D, L235S, L235N, L235Q, L235T, L235H, L235Y, L235I, L235V, L235F, S239D, S239E, S239N, S239Q, S239F, S239T, S239H, S239Y, V240I, V240A, V240T, V240M, F241W, F241L, F241Y, F241E, F241R, F243W, F243L, F243Y, F243R, F243Q, P244H, P245A, P247V, P247G, V262I, V262A, V262T, V262E, V263I, V263A, V263T, V263M, V264L, V264I, V264W, V264T, V264R, V264F, V264M, V264Y, V264E, D265G, D265N, D265Q, D265Y, D265F, D265V, D265I, D265L, D265H, D265T, V266I, V266A, V266T, V266M, S267Q, S267L, E269H, E269Y, E269F, E269R, Y296E, Y296Q, Y296D, Y296N, Y296S, Y296T, Y296L, Y296I, Y296H, N297S, N297D, N297E, A298H, T299I, T299L, T299A, T299S, T299V, T299H, T299F, T299E, W313F, N325Q, N325L, N325I, N325D, N325E, N325A, N325T, N325V, N325H, A327N, A327L, L328M, L328D, L328E, L328N, L328Q, L328F, L328I, L328V, L328T, L328H, L328A, P329F, A330L, A330Y, A330V, A330I, A330F, A330R, A330H, I332D, I332E, I332N, I332Q, I332T, I332H, I332Y and I332A, wherein the numbering of the residues in the Fc region is that of the EU index as in Kabat.

Fc variants can also be selected from the group consisting of V264L, V264I, F241W, F241L, F243W, F243L, F241L/F243L/V262I/V264I, F241W/F243W, F241W/F243W/V262A/V264A, F241L/V262I, F243L/V264I, F243L/V262I/V264W, F241Y/F243Y/V262T/V264T, F241E/F243R/V262E/V264R, F241 E/F24 3Q/V262T /V264E, F241R/F243Q/V262T/V264R, F241E/F243Y/V262T/V264R, L328M, L328E, L328F, I332E, L3238M/I332E, P244H, P245A, P247V, W313F, P244H/P245A/P247V, P247G, V264I/I332E, F241E/F243R/V262E/V264R/I332E, F241 E/F24 3Q/V262T /264E/I332E, F241R/F243Q/V262T/V264R/I332E, F241 E/F24 3Y /V262T /V264R/I332E, S298A/I332E, S239E/I332E, S239Q/I332E, S239E, D265G, D265N, S239E/D265G, S239E/D265N, S239E/D265Q, Y296E, Y296Q, T299I, A327N, S267Q/A327S, S267L/A327S, A327L, P329F, A330L, A330Y, I332D, N297S, N297D, N297S/I332E, N297D/I332E, N297E/I332E, D265Y/N297D/I332E, D265Y/N297D/T299L/I332E, D265F/N297E/I332E, L328I/I332E, L328Q/I332E, I332N, I332Q, V264T, V264F, V240I, V263I, V266I, T299A, T299S, T299V, N325Q, N325L, N325I, S239D, S239N, S239F, S239D/I332D, S239D/I332E, S239D/I332N, S239D/I332Q, S239E/I332D, S239^I332N, S239E/I332Q, S239N/I332D, S239N/I332E, S239N/I332N, S239N/I332Q, S239Q/I332D, S239Q/I332N, S239Q/I332Q, Y296D, Y296N, F241Y/F243Y/V262T/V264T/N297D/I332E, A330Y/I332E, V264I/A330Y/I332E, A330L/I332E, V264I/A330L/I332E, L234D, L234E, L234N, L234Q, L234T, L234H, L234Y, L234I, L234V, L234F, L235D, L235S, L235N, L235Q, L235T, L235H, L235Y, L235I, L235V, L235F, S239T, S239H, S239Y, V240A, V240T, V240M, V263A, V263T, V263M, V264M, V264Y, V266A, V266T, V266M, E269H, E269Y, E269F, E269R, Y296S, Y296T, Y296L, Y296I, A298H, T299H, A330V, A330I, A330F, A330R, A330H, N325D, N325E, N325A, N325T, N325V, N325H, L328D/I332E, L328E/I332E, L328N/I332E, L328Q/I332E, L328V/I332E, L328T/I332E, L328H/I332E, L328I/I332E, L328A, I332T, I332H, I332Y, I332A, S239E/V264I/I332E, S239Q/V264I/I332E, S239E/V264I/A330Y/I332E, S239E/V264I/S298A/A330Y/I332E, S239D/N297D/I332E, S239E/N297D/I332E, S239D/D265V/N297D/I332E, S239D/D265I/N297D/I332E, S239D/D265L/N297D/I332E, S239D/D265F/N297D/I332E, S239D/D265Y/N297D/I332E, S239D/D265H/N297D/I332E, S239D/D265T/N297D/I332E, V264E/N297D/I332E, Y296D/N297D/I332E, Y296E/N297D/I332E, Y296N/N297D/I332E, Y296Q/N297D/I332E, Y296H/N297D/I332E, Y296T/N297D/I332E, N297D/T299V/I332E, N297D/T299I/I332E, N297D/T299L/I332E, N297D/T299F/I332E, N297D/T299H/I332E, N297D/T299E/I332E, N297D/A330Y/I332E, N297D/S298A/A330Y/I332E, S239D/A330Y/I332E, S239N/A330Y/I332E, S239D/A330L/I332E, S239N/A330L/I332E, V264I/S298A/I332E, S239D/S298A/I332E, S239N/S298A/I332E, S239D/V264I/I332E, S239D/V264I/S298A/I332E, and S239D/264I/A330L/I332E, wherein the numbering of the residues in the Fc region is that of the EU index as in Kabat. See also WO2004029207, incorporated by reference herein.

In particular embodiments, mutations on, adjacent, or close to sites in the hinge link region (e.g., replacing residues 234, 235, 236 and/or 237 with another residue) can be made, in all of the isotypes, to reduce affinity for Fc-gamma receptors, particularly Fc-gamma-RI receptor (see, eg US6624821). Optionally, positions 234, 236 and/or 237 are substituted with alanine and position 235 with glutamate. (See, eg US5624821.) Position 236 is missing in the human IgG2 isotype. Exemplary segments of amino acids for positions 234, 235 and 237 for human IgG2 are Ala Ala Gly, Val Ala Ala, Ala Ala Ala, Val Glu Ala, and Ala Glu Ala. A preferred combination of mutants is L234A, L235E and G237A, or is L234A, L235A, and G237A for human isotype IgG1. A particular preferred ABP of the invention is an antibody having human isotype IgG1 and one of these three mutations of the Fc region. Other substitutions that decrease binding to Fc-gamma receptors are an E233P mutation (particularly in mouse IgG1) and D265A (particularly in mouse IgG2a). Other examples of mutations and combinations of mutations reducing Fc and/or C1q binding are E318A/K320A/R322A (particularly in mouse IgG1), L235A/E318A/K320A/K322A (particularly in mouse IgG2a). Similarly, residue 241 (Ser) in human IgG4 can be replaced, eg with proline to disrupt Fc binding.

Additional mutations can be made to a constant region to modulate effector activity. For example, mutations can be made to the IgG1 or IgG2 constant region at A330S, P331S, or both. For IgG4, mutations can be made at E233P, F234V and L235A, with G236 deleted, or any combination thereof. IgG4 can also have one or both of the following mutations S228P and L235E. The use of disrupted constant region sequences to modulate effector function is further described, eg in WO2006118,959 and WO2006036291.

Additional mutations can be made to the constant region of human IgG to modulate effector activity (see, e.g., WO200603291). These include the following substitutions: (i) A327G, A330S, P331S; (ii) E233P, L234V, L235A, G236 deleted; (iii) E233P, L234V, L235A; (iv) E233P, L234V, L235A, G236 deleted, A327G, A330S, P331S; and (v) E233P, L234V, L235A, A327G, A330S, P331S to human IgG1; or in particular, (vi) L234A, L235E, G237A, A330S and P331S (eg, to human IgG1), wherein the numbering of the residues in the Fc region is that of the EU index as in Kabat. See also WO2004029207, incorporated by reference herein.

The affinity of an antibody for the Fc-gamma-R can be altered by mutating certain residues of the heavy chain constant region. For example, disruption of the glycosylation site of human IgG1 can reduce Fc-gamma-R binding, and thus effector function, of the antibody (see, eg WO2006036291). The tripeptide sequences NXS and NXT, where X is any amino acid other than proline, are the enzymatic recognition sites for glycosylation of the N residue. Disruption of any of the tripeptide amino acids, particularly in the CH2 region of IgG, will prevent glycosylation at that site. For example, mutation of N297 of human IgG1 prevents glycosylation and reduces Fc-gamma-R binding to the antibody.

Although activation of ADCC and CDC is often desirable for therapeutic antibodies, there are circumstances in which an ABP of the invention unable to activate effector functions is preferential (eg, an ABP of the invention that is an agnostic modulator). For these purposes IgG4 has commonly been used but this has fallen out of favour in recent years due the unique ability of this sub-class to undergo Fab-arm exchange, where heavy chains can be swapped between IgG4 in vivo as well as residual ADCC activity. Accordingly, Fc engineering approaches can also be used to determine the key interaction sites for the Fc domain with Fc-gamma receptors and C1q and then mutate these positions, such as in an Fc of an ABP of the invention, to reduce or abolish binding. Through alanine scanning Duncan and Winter (1998; Nature 332:738) first isolated the binding site of C1q to a region covering the hinge and upper CH2 of the Fc domain. Researchers at Genmab identified mutants K322A, L234A and L235A, which in combination are sufficient to almost completely abolish Fc-gamma-R and C1q binding (Hezareh et al, 2001; J Virol 75:12161). In a similar manner MedImmune later identified a set of three mutations, L234F/L235E/P331S (dubbed TM), which have a very similar effect (Oganesyan et al, 2008; Acta Crystallographica 64:700). An alternative approach is modification of the glycosylation on asparagine 297 of the Fc domain, which is known to be required for optimal FcR interaction. A loss of binding to Fc-gammaRs has been observed in N297 point mutations (Tao et al, 1989; J Immunol 143:2595), enzymatically degylcosylated Fc domains (Mimura et al, 2001; J Biol Chem 276:45539), recombinantly expressed antibodies in the presence of a glycosylation inhibitor (Walker et al, 1989; Biochem J 259:347) and the expression of Fc domains in bacteria (Mazor et al 2007; Nat Biotechnol 25:563). Accordingly, the invention also includes embodiments of the ABPs in which such technologies or mutations have been used to reduce effector functions.

IgG naturally persists for a prolonged period in (eg human) serum due to FcRn-mediated recycling, giving it a typical half-life of approximately 21 days. Despite this there have been a number of efforts to engineer the pH dependant interaction of the Fc domain with FcRn to increase affinity at pH 6.0 while retaining minimal binding at pH 7.4. Researchers at PDL BioPharma identified the mutations T250Q/M428L, which resulted in an approximate 2-fold increase in IgG half-life in rhesus monkeys (Hinto et al, 2004; J Biol Chem 279:6213), and researchers at MedImmune have identified mutations M252Y/S254T/T256E (dubbed YTE), which resulted in an approximate 4-fold increase in IgG half-life in cynomolgus monkeys (Dall'Acqua, et al 2006; J Biol Chem 281:23514). A combination of the M252Y/S254T/T256E mutations with point mutations H433K/N434F lead to similar effects (Vaccaro et al., 2005, Nat Biotechnol. Oct;23(10):1283-8). ABPs of the invention may also be PEGylated. PEGylation, ie chemical coupling with the synthetic polymer poly-ethylene glycol (PEG), has emerged as an accepted technology for the development of biologics that exercise prolonged action, with around 10 clinically approved protein and peptide drugs to date (Jevsevar et al., 2010; Biotechnol J 5:113). ABPs of the invention may also be subjected to PASylation, a biological alternative to PEGylation for extending the plasma half-life of pharmaceutically active proteins (Schlapschy et al, 2013; Protein Eng Des Sel 26:489; XL-protein GmbH, Germany). Similarily, the XTEN half-life extension technology from Amunix provides another biological alternative to PEGylation (Schellenberger, 2009, Nat Biotechnol.;27(12):1186-90. doi: 10.1038/nbt.1588). Accordingly, the invention also includes embodiments of the ABPs in which such technologies or mutations have been used to prolong serum half-life, especially in human serum.

Antibody fragments include "Fab fragments", which are composed of one constant and one variable domain of each of the heavy and the light chains, held together by the adjacent constant region of the light chain and the first constant domain (CH1) of the heavy chain. These may be formed by protease digestion, e.g. with papain, from conventional antibodies, but similar Fab fragments may also be produced by genetic engineering. Fab fragments include Fab', Fab and "Fab-SH" (which are Fab fragments containing at least one free sulfhydryl group).

Fab' fragments differ from Fab fragments in that they contain additional residues at the carboxy terminus of the first constant domain of the heavy chain including one or more cysteines from the antibody hinge region. Fab' fragments include "Fab'-SH" (which are Fab' fragments containing at least one free sulfhydryl group).

Further, antibody fragments include F(ab')2 fragments, which contain two light chains and two heavy chains containing a portion of the constant region between the CH1 and CH2 domains ("hinge region"), such that an interchain disulphide bond is formed between the two heavy chains. A F(ab')2 fragment thus is composed of two Fab' fragments that are held together by a disulphide bond between the two heavy chains. F(ab')2 fragments may be prepared from conventional antibodies by proteolytic cleavage with an enzyme that cleaves below the hinge region, e.g. with pepsin, or by genetic engineering.

An "Fv region" comprises the variable regions from both the heavy and light chains, but lacks the constant regions. "Single-chain antibodies" or "scFv" are Fv molecules in which the heavy and light chain variable regions have been connected by a flexible linker to form a single polypeptide chain, which forms an antigen binding region.

An "Fc region" comprises two heavy chain fragments comprising the CH2 and CH3 domains of an antibody. The two heavy chain fragments are held together by two or more disulphide bonds and by hydrophobic interactions of the CH3 domains.

Accordingly, in some embodiments, the ABP of the invention is an antibody fragment selected from the list consisting of: Fab', Fab, Fab'-SH, Fab-SH, Fv, scFv and F(ab')2.

In those embodiments of ABPs that are fragments of immunoglobulins, such as an antibody fragment, preferred are those fragments capable of binding to (eg an epitope displayed by) the extracellular domain(s) of LILRB1 and/or LILRB2, or a paralogue, orthologue or other variant thereof, such as any epitope or other binding characteristic as described herein: and more preferably said fragment is a modulator (such as an inhibitor or antagonist) of the expression, function, activity and/or stability of LILRB1 and/or LILRB2 or a paralogue, orthologue or other variant of LILRB1 and/or LILRB2.

In a preferred embodiment, an ABP of the invention is an antibody wherein at least a portion of the framework sequence of said antibody or fragment thereof is a human consensus framework sequence, for example, comprises a human germline-encoded framework sequence.

In some embodiments, an ABP of the invention is modified or engineered to increase antibody-dependent cellular cytotoxicity (ADCC). As will now be understood by the person of ordinary skill, such ABPs of the invention will have particular utility in the therapy of diseases or disorders associated with cellular resistance against immune cells like CTLs (such as an LILRB1 and/or LILRB2-positive cancer); as the ADCC mechanism (a cell-mediated immune defence whereby an effector cell of the immune system actively lyses a target cell, whose membrane-surface antigens have been bound by specific antibodies) would be enhanced in respect of the cells having resistance against immune cells like CTLs, hence leading to an increase in attachment by and/or lysis of such cells by effector cells of the immune system.

As used herein, "therapy" is synonymous with treating a disease, disorder or condition, which includes reducing symptoms of the disease, disorder or condition, inhibiting progression of the disease, disorder or condition, causing regression of the disease, disorder or condition and/or curing the disease, disorder or condition.

Various techniques to modify or engineer an ABP of the invention to increase ADCC are known (Satoh et al, 2006; Expert Opin Biol Ther 6:1161; WO2009/135181), and hence such embodiments include those wherein an ABP of the invention may be afucosylated (GlycArt Biotechnology) e.g., in which antibodies are produced in CHO cells in which the endogenous FUT8 gene has been knocked out; or the ABP may be a "Sugar-Engineered Antibody" (Seattle Genetics), e.g. in which fucose analogues are added to antibody-expressing CHO cells, resulting in a significant reduction in fucosylation. Other afucosylation approaches that may be applied to an ABP of the invention are described elsewhere herein.

Other techniques to modify or engineer an ABP of the invention to increase ADCC include mutations in a Fc portion of the ABP, (such as described in more detail elsewhere herein), in particular where one or more of residues 234, 235, 236 and/or 237, and/or residues 330, 331 of human Fc are so mutated; wherein such numbering of the residues in the Fc region is that of the EU index as in Kabat (Kabat et ah, Sequences of Proteins of Immunological Interest (National Institute of Health, Bethesda, Md. 1987).

Accordingly, in certain embodiments, the ABP of the invention is modified or engineered to increase antibody-dependent cell-mediated cytotoxicity (ADCC), preferably wherein said ABP is afucosylated and/or an Fc of said ABP is mutated. In alternative embodiments, the ABP of the invention is modified or engineered to reduce ADCC (eg where an Fc is mutated using one or more of the following residue changes: L234A, L235E, G237A, A330S and/or P331S).

In other certain embodiments, the ABP of the invention is modified to prolong serum half-life, especially in human serum. For example, an ABP of the invention may be PEGylated and/or PASylated, or has an Fc region with a T250Q/M428L, H433K/N434F/Y436 or M252Y/S254T/T256E/H433K/N434F modification.

An ABP of the present invention may be mono-specific (i.e, it possesses antigen binding domain(s) that bind to only one antigen) or may be multi-specific (i.e, it possesses two or more different antigen binding domain(s) that bind to different antigens). For example, a "bi-specific", "dual-specific" or "bifunctional" ABP or antibody is a hybrid ABP or antibody, respectively, having two different antigen binding sites. Bi-specific antigen binding proteins and antibodies are a species of multi-specific antigen binding protein antibody and can be produced by a variety of methods including, but not limited to, fusion of hybridomas or linking of Fab' fragments (see, e.g., Songsivilai and Lachmann, 1990; Kostelny et al., 1992). The two binding sites of a bi-specific antigen binding protein or antibody will bind to two different epitopes, which can reside on the same or different protein targets.

In certain of such embodiments, the ABP may be a bi-specific, tri-specific, or tetra-specific antibody, in particular a bi-specific antibody is selected from: a bispecific T-cell engager (BiTE) antibody, a dual-affinity retargeting molecule (DART), a CrossMAb antibody, a DutaMab^{™} antibody, a DuoBody antibody; a Triomab, a TandAb, a bispecific NanoBody, Tandem scFv, a diabody, a single chain diabody, a HSA body, a (scFv)2 HSA Antibody, an scFv-IgG antibody, a Dock and Lock bispecific antibody, a DVD-IgG antibody, a TBTI DVD-IgG, an IgG-fynomer, a Tetravalent bispecific tandem IgG antibody, a dual-targeting domain antibody, a chemically linked bispecific (Fab')2 molecule, a crosslinked mAb, a Dual-action Fab IgG (DAF-IgG), an orthoFab-IgG, a bispecific CovX-Body, a bispecific hexavalent trimerbody, and an ART-Ig.

Accordingly, in certain embodiments, the ABP of the invention is a multi-specific antibody comprising at least two antigen binding domains, wherein each antigen binding domain specifically binds to a different antigen epitope.

In certain of such embodiments of such an ABP, at least two of the different antigen epitopes are epitopes displayed by the ECD of LILRB1 and/or LILRB2 protein, or wherein at least one of the different antigen epitopes is an epitope displayed by the ECD of LILRB1 and/or LILRB2 protein, and at least one of the different antigen epitopes is an epitope displayed by a protein other than LILRB1 and/or LILRB2, and preferably other than an epitope displayed by a protein other than HLA-G.

Accordingly, in some embodiments, the ABP of the invention binds (e.g. via one or more first antigen binding domain(s)) to an extracellular domain(s) of the LILRB1 and/or LILRB2, paralogue, orthologue or other variant when expressed on the surface of a mammalian cell, and in addition comprises one or more additional antigen binding domain(s) that bind(s) to antigen(s) other than said LILRB1 and/or LILRB2 or variant. Such other antigen may, in certain embodiments of the inventive ABP, be another immunoglobulin superfamily gene (preferably not LILRA); and/or such other antigen may be an antigen present on a mammalian T-cell. Antigens present on a mammalian T-cell, that may be bound by such an additional antigen binding domain, include CD3, CD40, OX-40, ICOS and 4-1BB. Such other antigen may, in certain embodiments of the inventive ABP, also be albumin, e.g., human albumin. It may also be another component of blood or blood serum the binding of which by the ABP will confer an extended serum half-life upon the ABP, e.g., a half-life similar to that when bound to albumin.

In other embodiments, an ABP of the invention can comprise two or more antigen binding regions, preferably comprising two, three or four antigen binding regions.

In yet other embodiments, an ABP of the invention can comprise a chimeric antigen receptor (CAR), and preferably comprises an extracellular antigen binding region, a membrane anchor such as a transmembrane domain, and an intracellular region, for example, an intracellular signalling region.

In preferred embodiments, an ABP of the invention can comprise at least one antibody constant domain, in particular wherein at least one antibody constant domain is a CH1, CH2, or CH3 domain, or a combination thereof.

In further of such embodiments, an ABP of the invention having antibody constant domain comprises a mutated Fc region, for example for increasing interaction of the Fc region with a Fc receptor (Fc receptor on an immune effector cell (eg Saxena & Wu, 2016; Front Immunol 7:580). Examples and embodiments thereof are described elsewhere herein.

In other embodiments, an ABP of the invention may comprises an effector group and/or a labelling group.

The term "effector group" means any group, in particular one coupled to another molecule such as an antigen binding protein, that acts as a cytotoxic agent. Examples for suitable effector groups are radioisotopes or radionuclides. Other suitable effector groups include toxins, therapeutic groups, or chemotherapeutic groups. Examples of suitable effector groups include calicheamicins, auristatins, geldanamycins, alpha-amanitine, pyrrolobenzodiazepines and maytansines.

The term "label" or "labelling group" refers to any detectable label. In general, labels fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

In some embodiments, an effector group or a labelling group is coupled to another molecule (such as the ABP) via spacer arms of various lengths to reduce potential steric hindrance.

In **another aspect,** the invention relates to **an antigen binding domain (ABD)** of an ABP of the invention, such as of any ABP as described above or elsewhere herein. In certain embodiments, an ABD of the invention is capable, when comprised in an applicable scaffold, of binding to the ECD of LILRB1 and/or LILRB2 (or variant thereof). An ABD of the invention may, in certain embodiments, be isolated and/or substantial pure.

### Nucleic acids, nucleic acid constructs and (host) cells

In **a third aspect,** the invention relates to **a nucleic acid** encoding for an ABP (or ABD) of the invention (such as one described above) or of components thereof. For example, the component encoded by a nucleic acid of the invention may be all or part of one chain of an antibody of the invention; or the component may be a scFV of said ABP. The component encoded by such a nucleic acid may be all or part of one or other of the chains of an antibody of the invention; for example, the component encoded by such a nucleic acid may be an ABD of the invention. The nucleic acids of the invention may also encode a fragment, derivative, mutant, or variant of an ABP of the invention, and/or represent components that are polynucleotides suitable and/or sufficient for use as hybridisation probes, polymerase chain reaction (PCR) primers or sequencing primers for identifying, analyzing, mutating or amplifying a polynucleotide encoding a polypeptide, anti-sense or inhibitory nucleic acids (such as RNAi/siRNA/shRNA or gRNA molecules) for inhibiting expression of a polynucleotide, and complementary sequences of the foregoing.

In particular embodiments of the invention, a nucleic acid of the invention comprises a nucleic acid having a sequence encoding a heavy or light chain CDR, a combination of heavy and/or light chain CDR1, CDR2 and CDR3 or a heavy or light chain variable domain, in each case as displayed in **Table 1,** or a functional fragment thereof. In other embodiments, a nucleic acid of the invention comprises a nucleic acid sequence at least 60%, 65%, 70%, 75%, 80%, 85%, 90%; or 95% (preferably at least 75%) sequence identity to (or having no more than fifty, forty, thirty, twenty, fifteen, ten or five, preferably no more than three, two or one, base substitution(s), insertion(s) or deletion(s), preferably at the third base of a codon of) a nucleic acid sequence selected from the list consisting of SEQ IDS Nos. 9, 10, 19, 20, 29, 30, 39, 40, 49, 50, 59, 60, 69, 70, 79, 80, 89, 90, 99, 100, 109, 110, 119, 120, 129, 130, 139, 140, 149, 150, 159 160, 169, 170, 179, 180, 189, 190, 199, 200, 209, 210, 219, 220, 229, 230, 239, 240, 249, 250, 259, 260, 269, 270, 279, 280, 289, 290, 299, 300, 309, 310, 319, 320, 329, 330, 339, 340, 349, 350, 359, 360, 369 and 370; preferably wherein such nucleic acid encodes a heavy or light chain variable domain of an ABP of the invention, such as encodes the corresponding heavy or light chain variable domain having the amino acid sequence set forth in **Table 1,** and optionally having no more than ten, nine, eight, seven, six, five, four, preferably no more than three, two or one, amino acid substitution(s), insertion(s) or deletion(s) compared to these sequences.

The nucleic acid according to the invention may be a DNA or RNA of genomic, mRNA, cDNA, or synthetic origin or some combination thereof, optionally linked to a polynucleotide to which it is not linked in nature. In some embodiments, such nucleic acid may comprise one or more (such as 2, 3, 4, 5, 6, 7, 8, 9, 10 or more than 20, in particular between 1 and about 5, or preferably all instances of a particular nucleotide in the sequence) unnatural (e.g. synthetic) nucleotides; and/or such nucleic acid may comprise (e.g. is conjugated to) another chemical moiety, such as a labelling group or an effector group; for example, a labelling group or an effector group as described elsewhere herein.

In one embodiment, the nucleic acid of the invention may be isolated or substantially pure. In another embodiment, the nucleic acid of the invention may be recombinant, synthetic and/or modified, or in any other way non-natural. For example, a nucleic acid of the invention may contain at least one nucleic acid substitution (or deletion) modification (such as 2, 3, 4, 5, 6, 7, 8, 9, 10 or more than 10 such modifications, in particular between 1 and about 5 such modifications, preferably 2 or 3 such modifications) relative to a product of nature, such as a human nucleic acid.

The nucleic acids can be any suitable length, such as about 10, 15, 20, 25, 30, 35, 40, 45, 50, 75, 100, 125, 150, 175, 200, 250, 300, 350, 400, 450, 500, 750, 1,000, 1,500, 3,000, 5,000 or more nucleotides in length. For example: siRNA nucleic acids may, preferably, be between about 15 to about 25 base pairs in length (preferably between about 19 and about 21 base pairs in length); shRNA nucleic acids may, preferably, comprise a 20-30 base pair stem, a loop of at least 4 nucleotides, and a dinucleotide overhang at the 3′ end; microRNA may, preferably, be about 22 base pairs in length; an mRNA or DNA sequence encoding an ABP or a component thereof (such as a heavy or light chain or an IgG antibody) of the invention may, preferably, be between about 500 and 1,500 nucleotides. More preferably, a nucleic acid encoding a mammalian light chain of an antibody may be between about 630 and about 650 nucleotides, and one encoding a mammalian heavy chain of an antibody may be between about 1,300 and about 1,650 nucleotides. A nucleic acid can comprise one or more additional sequences, for example, regulatory sequences, and/or be part of a larger nucleic acid. The nucleic acids can be single-stranded or double-stranded and can comprise RNA and/or DNA nucleotides, and artificial variants thereof (e.g., peptide nucleic acids).

Nucleic acids encoding antibody polypeptides (e.g., heavy or light chain, variable domain only, or full length) may be isolated from B-cells of mice, rats, llamas, alpacas, chicken or rabbits that have been immunized with an LILRB1 and/or LILRB2 antigen or fragment thereof, such as one or more EC domains (or a polynucleotide encoding and capable of expressing an LILRB1 and/or LILRB2 antigen or fragment thereof). The nucleic acid may be isolated by conventional procedures such as PCR.

Changes can be introduced by mutation into the sequence of a nucleic acid of the invention. Such changes, depending on their nature and location in a codon, can lead to changes in the amino acid sequence of a polypeptide (e.g., an antigen binding protein) that it encodes. Mutations can be introduced using any technique known in the art.

In one embodiment, one or more particular amino acid residues may be changed using, for example, a site-directed mutagenesis protocol. In another embodiment, one or more randomly selected residues may be changed using, for example, a random mutagenesis protocol. However, it is made, a mutant polypeptide can be expressed and screened for a desired property. Mutations can be introduced into a nucleic acid without significantly altering the biological activity of a polypeptide that it encodes. For example, one can make nucleotide substitutions leading to amino acid substitutions at non-essential amino acid residues.

Other changes that may be made (e.g. by mutation) to the sequence of a nucleic acid of the invention may not alter the amino acid sequence of the encoded polypeptide, but may lead to changes to its stability and/or effectiveness of expression of the encoded polypeptide. For example, by codon optimisation, the expression of a given polypeptide sequence may be improved by utilising the more common codons for a given amino acid that are found for the species in which the nucleotide is to be expressed. Methods of codon optimisation, and alternative methods (such as optimisation of CpG and G/C content), are described in, for example, Hass et al, 1996 (Current Biology 6:315); WO1996/09378; WO2006/015789 and WO 2002/098443).

In one **related aspect,** the invention relates to **a nucleic acid construct (NAC)** comprising at least one nucleic acid of the invention (such as described above). Such an NAC can comprise one or more additional features permitting the expression of the encoded ABP or component of said ABP (eg the ABD) in a cell (such as in a host cell). Examples of NACs of the invention include, but are not limited to, plasmid vectors, viral vectors, mRNA, non-episomal mammalian vectors and expression vectors, for example, recombinant expression vectors. The nucleic acid constructs of the invention can comprise a nucleic acid of the invention in a form suitable for expression of the nucleic acid in a cell, such as a host cell, (see below). The nucleic acid constructs of the invention will be, typically, recombinant nucleic acids, and/or may be isolated and/or substantially pure. Recombinant nucleic acids will, typically, be non-natural; particularly if they comprise portions that are derived from different species and/or synthetic, in-vitro or mutagenic methods.

In some embodiments, an NAC of the invention comprises one or more constructs either of which includes a nucleic acid encoding either a heavy or a light antibody chain. In some embodiments, the NAC of the invention comprises two constructs, one of which includes a nucleic acid encoding the heavy antibody chain, the other of which includes a nucleic acid encoding the light antibody chain, such that expression from both constructs can generate a complete antibody molecule. In some embodiments, the NAC of the invention comprises a construct which includes nucleic acids encoding both heavy and light antibody chains, such that a complete antibody molecule can be expressed from one construct. In other embodiments, an NAC of the invention can comprise a single construct that encodes a single chain which is sufficient to form an ABP of the invention; for example, if the encoded ABP is a scFv or a single-domain antibody (such as a camelid antibody).

In some embodiments, the NAC of the invention includes sequences encoding all or part of a constant region, enabling an entire, or a part of, a heavy and/or light chain to be expressed.

An NAC according to the invention may comprise (or consist of) a mRNA molecule which includes an open reading frame encoding an ABP of the invention, and for example together with upstream and downstream elements (such as 5' and/or 3' UTRs and/or poly-A stretch) that enables expression of the ABP, and preferably enhancing stability of the mRNA and/or expression of the ABP. The use of mRNA as NACs to introduce into and express polynucleotides in cells is described, for example, in Zangi et al in Nat. Biotechnol. vol. 31, 898-907 (2013), Sahin et al (2014) Nature Reviews Drug Discovery 13:759 and by Thess et al in Mol. Ther. vol. 23 no.9, 1456-1464 (2015). Particular UTRs that may be comprised in an mRNA NAC of the invention include: 5'UTR of a TOP gene (WO2013/143699), and/or a histone stem-loop (WO 2013/120629). An mRNA NAC of the invention may further comprise one or more chemical modifications (EP 1 685 844); including a 5'-cap, such as m7G(5')ppp, (5'(A,G(5')ppp(5')A or G(5')ppp(5')G and/or at least one nucleotide that is an analogue of naturally occurring nucleotides, such as phosphorothioates, phosphoroamidates, peptide nucleotides, methylphosphonates, 7-deaza-guanosine, 5-methylcytosine or inosine.

NACs, such as DNA-, retroviral- and mRNA-based NACs of the invention may be used in genetic therapeutic methods in order to treat or prevent diseases of the immune system (see Methods of Treatment below), whereby an NAC that comprises an expressible sequence encoding an ABP of the invention is administered to the cell or organism (e.g. by transfection). In particular, the use of mRNA therapeutics for the expression of antibodies is known from WO2008/083949.

In another **related aspect,** the invention relates to **a cell** (such as a host cell and/or a recombinant host cell) comprising one or more nucleic acid or NAC of the invention. Preferably, such cell is capable of expressing the ABP (or component thereof) encoded by said NAC(s). For example, if an ABP of the invention comprises two separate polypeptide chains (e.g. a heavy and light chain of an IgG), then the cell of the invention may comprise a first NAC that encodes (and can express) the heavy chain of such ABP as well as a second NAC that encodes (and can express) the light chain of such ABP; alternatively, the cell may comprise a single NAC that encodes both chains of such ABP. In these ways, such a cell of the invention would be capable of expressing a functional (e.g. binding and/or inhibitory) ABP of the invention. A (host) cell of invention may be one of the mammalian, prokaryotic or eukaryotic host cells as described elsewhere herein, in particularly where the cell is a Chinese hamster ovary (CHO) cell.

In certain embodiments of such aspect, the (host) cell is a human cell; in particular it may be a human cell that has been sampled from a specific individual (eg an autologous human cell). In such embodiments, such human cell can be propagated and/or manipulated in-vitro so as to introduce a NAC of the present invention. The utility of a manipulated human cell from a specific individual can be to produce an ABP of the invention, including to reintroduce a population of such manipulated human cells into a human subject, such as for use in therapy. In certain of such uses, the manipulated human cell may be introduced into the same human individual from which it was first sampled; for example, as an autologous human cell.

The human cell that is subject to such manipulation can be of any germ cell or somatic cell type in the body. For example, the donor cell can be a germ cell or a somatic cell selected from the group consisting of fibroblasts, B cells, T cells, dendritic cells, keratinocytes, adipose cells, epithelial cells, epidermal cells, chondrocytes, cumulus cells, neural cells, glial cells, astrocytes, cardiac cells, oesophageal cells, muscle cells, melanocytes, hematopoietic cells, macrophages, monocytes, and mononuclear cells. The donor cell can be obtained from any organ or tissue in the body; for example, it can be a cell from an organ selected from the group consisting of liver, stomach, intestines, lung, pancreas, cornea, skin, gallbladder, ovary, testes, kidneys, heart, bladder, and urethra.

### Pharmaceutical compositions

To be used in therapy, the ABPs, nucleic acids or NACs (or the cells, such as host cells) of the invention may be formulated into a pharmaceutical composition appropriate to facilitate administration to animals or humans. The term "pharmaceutical composition" means a mixture of substances including a therapeutically active substance (such as an ABP of the invention) for pharmaceutical use.

Accordingly, in **a fourth aspect,** the invention relates to **a pharmaceutical composition** comprising a compound that is modulator of the expression, function, activity and/or stability of immunoglobulin superfamily member 11 (LILRB1 and/or LILRB2), or of a variant of LILRB1 and/or LILRB2 and a pharmaceutically acceptable carrier, stabiliser and/or excipient. For example, the LILRB1 and/or LILRB2 modulator is an ABP of the invention, and/or at least one NAC of the invention, and/or a (host) cell of the invention. Accordingly, in **a related aspect,** herein provided is a **pharmaceutical composition** comprising an ABP of the invention, and/or at least one NAC of the invention, and/or a (host) cell of the invention, and a pharmaceutically acceptable excipient or carrier.

In a preferred embodiment, the pharmaceutical composition comprises an ABP of the invention, for example in such embodiment, the LILRB1 and/or LILRB2 modulator is an ABP of the invention (eg an LILRB1 and/or LILRB2-inhibitory ABP of the invention).

By way of example, the pharmaceutical composition of the invention may comprise between 0.1% and 100% (w/w) active ingredient (for example, an LILRB1 and/or LILRB2 modulator), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration.

Solutions or suspensions used for parenteral, intradermal, or subcutaneous application, as well as comprising a compound of (or for use with) the invention (eg an LILRB1 and/or LILRB2 modulator), can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulphate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Kolliphor^{®} EL (formerly Cremophor EL^{™}; BASF, Parsippany, N.J.) or phosphate buffered saline (PBS). In all cases, the injectable composition should, typically, be sterile and be fluid to the extent that easy syringability exists. It should, typically, be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminium monostearate and gelatin.

Sterile injectable solutions can be prepared by incorporating the compound of (or for use with) the invention (e.g., an LILRB1 and/or LILRB2 modulator) in the required amount in an appropriate solvent with one or a combination of ingredients described herein, as required, followed by filtered sterilisation. Generally, dispersions are prepared by incorporating the active compound into a sterile vehicle which contains a basic dispersion medium and the required other ingredients from those described herein. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions, as well as comprising a compound of (or for use with) the invention (eg an LILRB1 and/or LILRB2 inhibitor), generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavouring agent such as peppermint, methyl salicylate, or orange flavouring.

Furthermore, the compounds of (or for use with) the invention (eg an LILRB1 and/or LILRB2 modulator) can be administered rectally. A rectal composition can be any rectally acceptable dosage form including, but not limited to, cream, gel, emulsion, enema, suspension, suppository, and tablet. One preferred dosage form is a suppository having a shape and size designed for introduction into the rectal orifice of the human body. A suppository usually softens, melts, or dissolves at body temperature. Suppository excipients include, but are not limited to, theobroma oil (cocoa butter), glycerinated gelatin, hydrogenated vegetable oils, mixtures of polyethylene glycols of various molecular weights, and fatty acid esters of polyethylene glycol.

For administration by inhalation, the compounds of (or for use with) the invention (eg an LILRB1 and/or LILRB2 modulator) are typically delivered in the form of an aerosol spray from pressured container or dispenser which contains a suitable propellant, e.g., a gas such as carbon dioxide, or a nebuliser.

Cells, such as immune cells (eg CAR T cells) for use with the invention can be included in pharmaceutical formulations suitable for administration into the bloodstream or for administration directly into tissues or organs. A suitable format is determined by the skilled person (such as a medical practitioner) for each patient, tissue, and organ, according to standard procedures. Suitable pharmaceutically acceptable carriers and their formulation are known in the art (see, e.g. Remington's Pharmaceutical Sciences 16th edition, Osol, A. Ed., 1980). Such cells, when formed in a pharmaceutical composition, are preferably formulated in solution at a pH from about 6.5 to about 8.5. Excipients to bring the solution to isotonicity can also be added, for example, 4.5% mannitol or 0.9% sodium chloride, pH buffered with art-known buffer solutions, such as sodium phosphate. Other pharmaceutically acceptable agents can also be used to bring the solution to isotonicity, including, but not limited to, dextrose, boric acid, sodium tartrate, propylene glycol, polyols (such as mannitol and sorbitol) or other inorganic or organic solutes. In one embodiment, a media formulation is tailored to preserve the cells while maintaining cell health and identity. For example, a premixture including an aqueous solution of anticoagulant (ACD-A), an equal amount of dextrose (50%), and phosphate buffered saline (PBS), or the like is pre-mixed and aliquoted in a volume to typically match or approximate the cellular matrix or environment from which the cell was extracted from the tissue or organ.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions can be formulated into ointments, salves, gels, or creams as generally known in the art.

In certain embodiments, the pharmaceutical composition is formulated for sustained or controlled release of a compound of (or for use with) the invention (eg an LILRB1 and/or LILRB2 modulator). Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially (including liposomes targeted to infected cells with monoclonal antibodies to viral antigens) can also be used as pharmaceutically acceptable carriers. These can be prepared according to methods known to those skilled in the art.

It is especially advantageous to formulate oral, rectal or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

In some embodiments, the pharmaceutical composition comprising an LILRB1 and/or LILRB2 modulator is in unit dose form of between 10 and 1000mg LILRB1 and/or LILRB2 modulator. In some embodiments, the pharmaceutical composition comprising an LILRB1 and/or LILRB2 modulator is in unit dose form of between 10 and 200mg LILRB1 and/or LILRB2 modulator. In some embodiments, the pharmaceutical composition comprising an ABP is in unit dose form of between 200 and 400mg LILRB1 and/or LILRB2 modulator. In some embodiments, the pharmaceutical composition comprising an LILRB1 and/or LILRB2 modulator is in unit dose form of between 400 and 600mg LILRB1 and/or LILRB2 modulator. In some embodiments, the pharmaceutical composition comprising an LILRB1 and/or LILRB2 modulator is in unit dose form of between 600 and 800mg LILRB1 and/or LILRB2 modulator. In some embodiments, the pharmaceutical composition comprising an LILRB1 and/or LILRB2 modulator is in unit dose form of between 800 and 100 mg LILRB1 and/or LILRB2 modulator.

Exemplary unit dosage forms for pharmaceutical compositions comprising LILRB1 and/or LILRB2 modulators are tablets, capsules (eg as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

Toxicity and therapeutic efficacy (eg effectiveness) of such active ingredients can be determined by standard pharmaceutical procedures in cell cultures or experimental animals, eg, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Active agents which exhibit large therapeutic indices are preferred. While compounds that exhibit toxic side effects may be used, care should be taken to design a delivery system that targets such compounds to the site of affected tissue in order to minimise potential damage to uninfected cells and, thereby, reduce side effects.

The data obtained from the cell culture assays and animal studies can be used in formulating a range of dosage of the active ingredients (eg an LILRB1 and/or LILRB2 modulator), such as for use in humans. The dosage of such active ingredients lies preferably within a range of circulating concentrations that include the ED50 with little or no toxicity. The dosage may vary within this range depending upon the dosage form employed and the route of administration utilised. For any active ingredients used in the therapeutic approaches of the invention, the (therapeutically) effective dose can be estimated initially from cell culture assays. A dose may be formulated in animal models to achieve a circulating plasma concentration range that includes the IC50 (ie, the concentration of the active ingredients which achieves a half-maximal inhibition of symptoms) as determined in cell culture. Such information can be used to more accurately determine useful (eg effective) amounts or doses, such as for administration to humans. The pharmaceutical compositions can be included in a container, pack, or dispenser together with instructions for administration.

In the context of the invention, an effective amount of the LILRB1 and/or LILRB2 modulator or the pharmaceutical composition can be one that will elicit the biological, physiological, pharmacological, therapeutic or medical response of a cell, tissue, system, body, animal, individual, patient or human that is being sought by the researcher, scientist, pharmacologist, pharmacist, veterinarian, medical doctor, or other clinician, eg, lessening of the effects/symptoms of a disorder, disease or condition, such as a proliferative disorder, for example, a cancer or tumour, or killing or inhibiting growth of a cell involved with a proliferative disorder, such as a tumour cell. The effective amount can be determined by standard procedures, including those described below.

In accordance with all aspects and embodiments of the medical uses and methods of treatment provided herein, the effective amount administered at least once to a subject in need of treatment with a LILRB1 and/or LILRB2 modulator is, typically, between about 0.01mg/kg and about 100mg/kg per administration, such as between about 1mg/kg and about 10mg/kg per administration. In some embodiments, the effective amount administered at least once to said subject of a LILRB1 and/or LILRB2 modulator is between about 0.01mg/kg and about 0.1mg/kg per administration, between about 0.1mg/kg and about 1mg/kg per administration, between about 1mg/kg and about 5mg/kg per administration, between about 5mg/kg and about 10mg/kg per administration, between about 10mg/kg and about 50mg/kg per administration, or between about 50mg/kg and about 100mg/kg per administration.

For the prevention or treatment of disease, the appropriate dosage of a LILRB1 and/or LILRB2 modulator (or a pharmaceutical composition comprised thereof) will depend on the type of disease to be treated, the severity and course of the disease, whether the LILRB1 and/or LILRB2 modulator and/or pharmaceutical composition is administered for preventive or therapeutic purposes, previous therapy, the patient's clinical history, age, size/weight and response to the LILRB1 and/or LILRB2 modulator and/or pharmaceutical composition, and the discretion of the attending physician. The LILRB1 and/or LILRB2 modulator and/or pharmaceutical composition is suitably administered to the patient at one time or over a series of treatments. If such LILRB1 and/or LILRB2 inhibitor and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

The amount of the LILRB1 and/or LILRB2 modulator and/or pharmaceutical composition administered will depend on variables such as the type and extent of disease or indication to be treated, the overall health, age, size/weight of the patient, the in vivo potency of the LILRB1 and/or LILRB2 modulator and/or pharmaceutical composition, and the route of administration. The initial dosage can be increased beyond the upper level in order to rapidly achieve the desired blood-level or tissue level. Alternatively, the initial dosage can be smaller than the optimum, and the daily dosage may be progressively increased during the course of treatment. Human dosage can be optimised, e.g., in a conventional Phase I dose escalation study designed to run from relatively low initial doses, for example from about 0.01mg/kg to about 20mg/kg of active ingredient. Dosing frequency can vary, depending on factors such as route of administration, dosage amount and the disease being treated. Exemplary dosing frequencies are once per day, once per week and once every two weeks. Formulation of an LILRB1 and/or LILRB2 modulator of (or for use with) the present is within the ordinary skill in the art. In some embodiments of the invention such LILRB1 and/or LILRB2 modulator is lyophilised and reconstituted in buffered saline at the time of administration. The LILRB1 and/or LILRB2 modulator and/or pharmaceutical composition of may further result in a reduced relapsing of the disease to be treated or reduce the incidence of drug resistance or increase the time until drug resistance is developing; and in the case of cancer may result in an increase in the period of progression-free survival and/or overall survival.

### LILRB1 and/or LILRB2 modulating uses, medical uses and methods of treatment

Modulating compounds of LILRB1 and/or LILRB2, in particular inhibiting the interaction of LILRB1 and/or LILRB2 and a natural ligand thereof (such as HLA-G) or of a variant of LILRB1 and/or LILRB2, and/or the ABPs, NAC, (host) cells and the pharmaceutical compositions of the invention can be used in various ways to modulate the expression, function, activity and/or stability of the LILRB1 and/or LILRB2 (or variant thereof), including their use in therapy or for prophylaxis.

Accordingly, **in a further aspect,** herein provided is a **method of modulating** the expression, function, activity and/or stability of of LILRB1 and/or LILRB2, or of a variant of LILRB1 and/or LILRB2 comprising contacting a cell that expresses said LILRB1 and/or LILRB2 or variant with a modulating compound as described above, in particular an ABP of the invention or an NAC encoding said ABP. When such ABP is a modulator of the expression, function, activity and/or stability of said LILRB1 and/or LILRB2 or variant, thereby the expression, function, activity and/or stability of said LILRB1 and/or LILRB2 or variant is modulated. Such method may be practiced on cells that are present *ex-vivo,* that is where said cells are contained in receptacles or containers, such as those used in research facilities. Accordingly, in such embodiments such method of the invention can be described as an *in-vitro* method of modulating the expression, function, activity and/or stability of LILRB1 and/or LILRB2, or of a variant of LILRB1 and/or LILRB2. However, in alternative embodiments, the method may be practiced using cells within the body, for example an in-vivo method of modulating the expression, function, activity and/or stability of LILRB1 and/or LILRB2, or of a variant of LILRB1 and/or LILRB2.

In particular of such embodiments, such an in-vitro (or in-vivo) method comprises the *inhibition* of the function and/or activity of the LILRB1 and/or LILRB2 or variant, when such modulating compound (eg the ABP) is an *inhibitor of and*/*or antagonist of* such function and/or activity. In some embodiments of such method, it further comprises the step of contacting the cell with an immune cell, such as a CTL or TIL. Preferably, the ABP is an antibody, or an antibody fragment, and is an *inhibitor or antagonist* of the function and/or activity of the LILRB1 and/or LILRB2 or variant.

In particular of such embodiments, such an in-vitro (or in-vivo) method comprises the *activation* of the function and/or activity of the LILRB1 and/or LILRB2 or variant, when such modulating compound (eg the ABP) is an *activator of and*/*or agonist* of such function and/or activity. In some embodiments of such method, it further comprises the step of contacting the cell with an immune cell, such as a CTL or TIL. Preferably, the ABP is an antibody, or an antibody fragment, and is an *activator and*/*or agonist* of the function and/or activity of the LILRB1 and/or LILRB2 or variant. In certain embodiments of these aspects, the method of modulating comprises contacting a cell that expresses said LILRB1 and/or LILRB2 or variant with a modulating compound as described above that is an *activator and*/*or agonist* of the function and/or activity of the LILRB1 and/or LILRB2 or variant, and the method mediates any one or combination of at least one of the functional characteristic or effects of the activating or agonistic modulators described herein, in particular as set forth in the section above "Modulators of LILRB1 and/or LILRB2 expression, function, activity and/or stability.

In other certain embodiments of these aspects, the method of modulating comprises contacting a cell that expresses said LILRB1 and/or LILRB2 or variant with a modulating compound as described above that is an *inhibitor and*/*or antagonist* of the function and/or activity of the LILRB1 and/or LILRB2 or variant, and the method mediates any one or combination of at least one of the functional characteristic or effects of the inhibitor or antagonist modulators described herein, in particular as set forth in the section above "Modulators of LILRB1 and/or LILRB2 expression, function, activity and/or stability.

In particular embodiments, the modulating compound (in particular, an ABP) is an *inhibitor and*/*or antagonist* of the function and/or activity of the LILRB1 and/or LILRB2 or variant and inhibits the interaction between of a natural ligand of LILRB1 and/or LILRB2 (such as HLA-G) protein or a variant thereof and LILRB1 and/or LILRB2 protein or a variant thereof; that is, such a compound inhibits the binding function and/or activity of the LILRB1 and/or LILRB2 protein or variant thereof.

In preferred embodiments of the therapeutic aspects, the modulating compound (such as one that is an inhibitor or antagonist of expression, function, activity and/or stability of the LILRB1 and/or LILRB2 or variant) for example an ABP, or an NAC encoding said ABP, is capable of: (i) modulating the expression, function, activity and/or stability of the LILRB1 and/or LILRB2 or variant; and/or (ii) enhancing a cell-mediated immune response to a mammalian cell, decreases or reduces the resistance of cells (such as tumour cells that express a natural ligand of LILRB1 and/or LILRB2 (for example HLA-G or variant)), to an immune response. In other certain preferred embodiments of the invention, the ABP (such as one that is an inhibitor or antagonist of expression, function, activity and/or stability of the LILRB1 and/or LILRB2 or variant, in particular one that inhibits the binding function and/or activity of the LILRB1 and/or LILRB2 protein to a ligand of LILRB1 and/or LILRB2), or an NAC encoding said ABP, enhances or increases the sensitivity of cells (such as tumour cells that express a natural ligand of LILRB1 and/or LILRB2 (for example HLA-G or variant)), to an immune response.

The term "resistance" refers to an acquired or natural resistance of a cell involved with (eg of or affected by) a disease (eg a proliferative disorder), such as tumour or cancer cell, to a patient's own immune response (such as a cell-mediated immune response), or to immune responses aided by immune therapy such as adoptive T-cell transfer or treatment with checkpoint blockers. Therefore, a resistant cell (eg a resistant tumour or cancer cell) is more likely to escape and survive humoural and/or cellular immune defence mechanisms in a subject having the disorder (such as the tumour or cancer). A treatment of a resistant proliferative disease, such as tumour/cancer resistance, in context of the invention shall be effective if, compared to a non-treated control, the cell involved with the proliferative disease (such as a cell of the tumour of cancer) becomes more sensitive or susceptible to an immune response (such as a cell-mediated immune response) - in other words will be more likely to be recognised and/or neutralised (for example by cytotoxic processes such as apoptosis) by the subject's immune response.

Accordingly, in particular embodiments of the invention, cell(s) involved with the disease may be resistant against (to) a cell-mediated immune response; and/or such cell(s) may have or display a resistant phenotype.

In preferred embodiments of the invention, the terms "cellular resistance", "cell resistance" and the like refers to a resistance of the subject cell(s) (such as a tumour or cancer cell) to a cell-mediated immune response, such as a cytotoxic T lymphocyte (CTL) response (eg, the tumour or tumour cell being nonresponsive to, or having reduced or limited response to a CTL targeting a tumour cell). A tumour cell may show a reduced or limited response when contacted with a CTL specific for an antigen expressed on that tumour cell. A reduced or limited response is a reduction to a 90% cytotoxic T cell response, preferably a reduction to 80%, 70%, 60%, 50% or more preferably a reduction to 40%, 30%, 20% or even less. In this case, 100% would denote the state wherein the CTLs can kill all of the subject cells involved with the proliferative disorder in a sample. Whether or not a subject cell (eg a tumour cell) is resistant to a patient's (cell-mediated) immune response may be tested in-vitro by contacting a sample of the subjects such cells (eg autologous tumour cells) with (eg autologous) T-cells and thereafter quantifying the survival/proliferation rate of the (eg) tumour cells. As an alternative, the reduction in (cell-mediated) immune response is determined by comparing cancer samples of the same cancer before and after the resistance is acquired (for example induced by therapy), or by comparing with a cancer sample derived from a different cancer which is known to have no resistance to the CTL. On the other hand, the treatments of the present invention include the sensitisation of cells involved with the proliferative disorder against CTL and therefor to decrease resistance of such cells. A decrease of (eg tumour) cell resistance against CTL is preferably a significant increase of CTL toxicity, preferably a 10% increase, more preferably 20%, 30%, 40%, 50%, 60%, 70%, 80% or more, even more preferably 2 fold increase, 3 fold, 4 fold, 5 fold or more.

In particular embodiments, a resistant phenotype of the cells involved with the proliferative disorder is displayed by such cells when a subject suffering from the proliferative disorder (eg a cancer or tumour) has been previously treated with an (immune)therapy and, for example, such proliferative disorders has progressed despite such prior (immune)therapy. For example, a class of subject suitable for the various therapeutic methods of the invention can be those whose tumour (or cancer) has progressed (such as has relapsed or recurred, or has not responded to) after prior treatment with a cancer immunotherapy. In certain embodiments, such prior treatment may be any immunotherapy as described elsewhere herein, including adoptive immune cell transfer (eg TCR or CART cell therapy), an anti-tumour vaccine, an antibody binding to an immune checkpoint molecule (such as CTLA-4, PD-1 or PD-L1). In other embodiments, the subject may suffer from a tumour or cancer, and such cancer may have progressed (such as has relapsed or recurred, or has not responded to) after prior radiotherapy.

The immune response, is, in particular of such embodiments, a cell-mediated immune response such as one mediated by T-cells including cytotoxic T-cells and/or TILs; and/or the immune response is the lysis and/or killing of the cells, in particular those that express LILRB1 and/or LILRB2 or a variant thereof) that is mediated by cytotoxic T-cells and/or TILs. In other particular of such embodiments, the immune response is a cytotoxic immune response against cells (such as tumour cells), in particular a cell-mediated cytotoxic immune response such as one mediated by T-cells including cytotoxic T-cells and/or TILs. LILRB1 and/or LILRB2 expressing cells are found in the myeloid compartment and modulate cell-mediated immune responses.

Specifically, in certain preferred embodiments of such therapeutic aspects the modulating compound as disclosed herein, in particular the ABP (such as one that is an inhibitor or antagonist of expression, function, activity and/or stability of the LILRB1 and/or LILRB2 or variant thereof), or an NAC encoding said ABP, enhances or increases killing and/or lysis of cells associated with the proliferative disorder, such as tumor cells. Such effect is brought about via modulation of cells expressing LILRB1 and/or LILRB2 or variant thereof, (such as macrophage cells); which modulate a killing and/or lysis of tumor cells mediated by cytotoxic T-cells and/or TILs, and/or mediated by an enhancement of or increase in the sensitivity of the cells of the immune system, and/or mediated by a decrease in or reduction of the immune inhibitory effects of the cells expressing the LILRB1 and/or LILRB2 or variant thereof on a (cytotoxic) immune response, such an immune response described above. As such LILRB1 and/or LILRB2 expressing cells brought into contact with the agents of the invention are therefore less immune inhibitory and support a subject's immune response against a proliferative disease to be treated.

The cells that express LILRB1 and/or LILRB2 or variant thereof are, in certain of such preferred embodiments, cells of the myeloid compartment, such as macrophages, or alternatively in some instances may be cancer cells or are cells that originated from a tumor cell. Exemplary cancer or tumor cells can be those as described or exemplified elsewhere herein.

In other certain preferred embodiments of such therapeutic aspects, the modulating compounds, in particular the ABP (such as one that is an inhibitor or antagonist of expression, function, activity and/or stability of LILRB1 and/or LILRB2 or variant thereof, in particular that is an inhibitor of the HLA-G-binding function of the LILRB1 and/or LILRB2 or variant), or an NAC encoding said ABP, increases T-cell activity and/or survival (and/or increases T-cell proliferation), which in certain embodiments, may lead to an enhancement of a (cytotoxic) immune response mediated by such T-cells.

Accordingly, in **a fifth aspect,** the invention relates to **a method for the treatment** of a disease, disorder or condition in a mammalian subject by administering a product to the subject wherein the product is a modulator of the expression, function, activity and/or stability of immunoglobulin superfamily member 11 (LILRB1 and/or LILRB2), or of a variant of LILRB1 and/or LILRB2. In **a related aspect,** the invention relates to **a product for use in medicine,** wherein the product is a compound that is modulator of the expression, function, activity and/or stability of immunoglobulin superfamily member 11 (LILRB1 and/or LILRB2), or of a variant of LILRB1 and/or LILRB2. In particular embodiments, of these medical/treatment claims, the modulating compound (such as an ABP) is an inhibitor of the binding HLA-G-binding function of LILRB1 and/or LILRB2 or variant thereof; and/or wherein the product is selected from the list consisting of an ABP, ABD, nucleic acid, NAC or recombinant host cell of the invention, in particular an ABP of the invention.

In a **related aspect,** the invention also relates to **method of treating or preventing** a disease, disorder or condition in a mammalian subject in need thereof, comprising administering to said subject at least once an effective amount of modulating compound as desired above, or, and in particular administering to said subject at least once an effective amount of the ABP, the NAC, the (host) cells, or the pharmaceutical composition as described above.

In another **related aspect,** the invention also relates to the **use of a product** of the invention as describe above, or a modulating compound as described above (in particular an ABP of the invention) **for the manufacture of a medicament,** in particular for the treatment of a disease, disorder or condition in a mammalian subject, in particular where the disease, disorder or condition is one as set out herein.

The term "treatment" in the present invention is meant to include therapy, e.g. therapeutic treatment, as well as prophylactic or suppressive measures for a disease (or disorder or condition). Thus, for example, successful administration of a LILRB1 and/or LILRB2 inhibitor prior to onset of the disease results in treatment of the disease. "Treatment" also encompasses administration of a LILRB1 and/or LILRB2 inhibitor after the appearance of the disease in order to ameliorate or eradicate the disease (or symptoms thereof). Administration of a LILRB1 and/or LILRB2 inhibitor after onset and after clinical symptoms, with possible abatement of clinical symptoms and perhaps amelioration of the disease, also comprises treatment of the disease. Those "in need of treatment" include subjects (such as a human subject) already having the disease, disorder or condition, as well as those prone to or suspected of having the disease, disorder or condition, including those in which the disease, disorder or condition is to be prevented.

In particular embodiments of these aspects, the modulating compound is one described above, and/or is an ABP, NAC, a (host) cell, or a pharmaceutical composition of the present invention; in particular is an ABP of the invention, and/or is an inhibitory nucleic acid of the invention.

Such a compound can for example in preferred embodiments, be an inhibitor or antagonist of expression, function, activity and/or stability of LILRB1 and/or LILRB2, or of the variant of LILRB1 and/or LILRB2. In particular, the compound inhibits the binding of a natural ligand of LILRB1 and/or LILRB2 protein (such as HLA-G or a variant thereof) to LILRB1 and/or LILRB2 protein (or a variant thereof), in particular inhibits the binding of HLA-G protein (or a variant thereof) to human LILRB1 and/or LILRB2 protein (or a variant thereof), such as inhibits the binding between the ECDs of such proteins; preferably wherein such proteins (or variants) and the inhibitions is described as above.

Such a compound can, for example, be a compound (such as an ABP or inhibitors nucleic acid) that has the any one or any combination of the following characteristics
- specific binding to human LILRB1 and/or (preferably and) LILRB2 (e.g. comprising the amino acid sequence of SEQ ID NO: 353 (LILRB1) or SEQ ID NO: 358 (LILRB2), e.g., with a KD 50 nM or less, or of 20nM or less; more preferably of 10nM or less or 5nM or less
- lack of specific binding to a LILRA protein, such as preferably LILRA1 and/or LILRA3;
- stimulates T cell activation, e.g., in a mixed lymphocyte reaction (MLR) assay, as measured by increased T cell proliferation or IFN-gamma secretion, e.g.;
- stimulates differentiation or activation of monocytes into macrophages, e.g., stimulates differentiation of monocytes into pro-inflammatory macrophages, e.g., as shown in an assay described in the Examples;
- inhibits binding of LILRB1 and/or (preferably and) LILRB2 to HLA-A and HLA-B, preferably to HLA-G;
- has a binding profile as shown in **Table 2;**
- promotes pro-inflammatory polarization of macrophages towards M1 macrophages; and
- does not induce (or trigger) basophil activation;

In other aspects described elsewhere herein, are provided methods to detect and/or diagnose a disease, disorder or condition in a mammalian subject.

In one particular embodiment, the disease, disorder or condition that is characterised by a pathological immune response.

In a further particular embodiment, the disease, disorder or condition is characterised by expression of a natural ligand of LILRB1 and/or LILRB2, in particular by expression of HLA-G, by cells associated with the disease, disorder or condition, such as cancer cells. For example, the disease, disorder or condition can be associated with the undesired presence of a natural ligand of LILRB1 and/or LILRB2, such as a HLA-G positive cells or cells positive for such a ligand, in particular a disease or condition characterized by LILRB1 and/or LILRB2 positive monocytes and/or macrophages (in particular, TAMs).

In a yet further particular embodiment, a subject suffering from, or suspected of suffering from, a disease, disorder or condition is characterised as: (i) having a cancer positive for a natural ligand of LILRB1 and/or LILRB2, such as HLA-G and/or (ii) having LILRB1 and/or LILRB2 positive immune cells, in particular LILRB1 and/or LILRB2 positive monocytes and/or macrophages; and/or (iii) having LILRB1 and/or LILRB2 positive immune cells, in particular LILRB1 and/or LILRB2 positive monocytes (or macrophages); preferably wherein such LILRB1 and/or LILRB2 positive immune cells are present at or associated with the site of a cancer or tumour (such as being present in the tumour bed or tumour micro environment (TME) of such cancer or tumour, in particular with the presence of TAMs and/or MDSCs).

A disorder, disorder or condition treatable by the subject matter of the invention is, in certain alterative embodiments, one characterised by expression of a ligand of LILRB1 and/or LILRB2; in particular, one characterised by such expression that is aberrant, for example over- (or under-) expression or representation or activity of a ligand of LILRB1 and/or LILRB2 (in particular of HLA-G) in a given cell or tissue (such as those cells or tissues involved with the proliferative disease of the subject) compared to that in a healthy subject or a normal cell.

In yet a further particular embodiment, the disease, disorder or condition is characterised by expression and/or activity of LILRB1 and/or LILRB2 in an immune cell, such as a macrophage, in particular such cells express mRNA and/or protein of LILRB1 and/or LILRB2, and/or are positive for such LILRB1 and/or LILRB2 expression and/or activity.

In another particular embodiment, the disease, disorder or condition is a proliferative disorder (or a condition associated with such disorder or disease), in particular when the product or modulating compound (such as a ABP, ABD, nucleic acid, NAC or recombinant host cell of the invention, in particular an ABP of the invention) is an *inhibitor and*/*or antagonist* of the expression, function, activity and/or stability of LILRB1 and/or LILRB2 or a variant of LILRB1 and/or LILRB2.

A "proliferative disorder" refers to a disorder characterised by abnormal proliferation of cells. A proliferative disorder does not imply any limitation with respect to the rate of cell growth, but merely indicates loss of normal controls that affect growth and cell division. Thus, in some embodiments, cells of a proliferative disorder can have the same cell division rates as normal cells but do not respond to signals that limit such growth. Within the ambit of "proliferative disorder" is neoplasm or tumour, which is an abnormal growth of tissue or cells. Cancer is art understood, and includes any of various malignant neoplasms characterised by the proliferation of cells that have the capability to invade surrounding tissue and/or metastasise to new colonisation sites. Proliferative disorders include cancer, atherosclerosis, rheumatoid arthritis, idiopathic pulmonary fibrosis and cirrhosis of the liver. Non-cancerous proliferative disorders also include hyperproliferation of cells in the skin such as psoriasis and its varied clinical forms, Reiter's syndrome, pityriasis rubra pilaris, and hyperproliferative variants of disorders of keratinization (e.g., actinic keratosis, senile keratosis), scleroderma, and the like.

In more particular embodiments, the proliferative disorder is a cancer or tumour, in particular a solid tumour (or a condition associated with such cancer or tumour). Such proliferative disorders include, but are not limited to, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, any haemotological malignancy (e.g., chronic lymphoblastic leukemia, chronic myelomonocytic leukemia, acute lymphoblastic leukemia, acute lymphocytic leukemia, acute myelogenous leukemia, acute myeloblasts leukemia, chronic myeloblastic leukemia, Hodgkin's disease, non- Hodgkin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, marginal zone lymphoma, Burkitt Lymphoma, mycosis fungoides, seary syndrome, cutaneous T-cell lymphoma, peripheral T cell lymphoma, chronic myeloproliferative disorders, myelofibrosis, myeloid metaplasia, systemic mastocytosis), and cental nervous system tumors (e.g., brain cancer, glioblastoma, non- glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma), myeloproliferative disorders (e.g., polycythemia vera, thrombocythemia, idiopathic myelofibrosis), soft tissue sarcoma, thyroid cancer, endometrial cancer, carcinoid cancer, or liver cancer.

In one preferred embodiment, the various aspects of the invention relate to, for example the ABPs of the invention used to detect/diagnose, prevent and/or treat, such proliferative disorders that include but are not limited to carcinoma (including breast cancer, prostate cancer, gastric cancer, lung cancer, colorectal and/ or colon cancer, hepatocellular carcinoma, melanoma), lymphoma (including non-Hodgkin's lymphoma and mycosis fungoides), leukemia, sarcoma, mesothelioma, brain cancer (including glioma), germinoma (including testicular cancer and ovarian cancer), choriocarcinoma, renal cancer, pancreatic cancer, thyroid cancer, head and neck cancer, endometrial cancer, cervical cancer, bladder cancer, or stomach cancer.

Accordingly, in a preferred embodiment, the proliferative disease is a cancer, for example lung cancer, breast cancer, colorectal cancer, gastric cancer, hepatocellular carcinoma, pancreatic cancer, ovarian cancer, melanoma, myeloma, kidney cancer, head and neck cancer, Hodgkin lymphoma, bladder cancer or prostate cancer, in particular one selected from the list consisting of: melanoma, lung cancer (such as non-small cell lung cancer), bladder cancer (such as urothelial carcinoma), kidney cancer (such as renal cell carcinoma), head and neck cancer (such as squamous cell cancer of the head and neck) and Hodgkin lymphoma. Preferably, the proliferative disease is melanoma, or lung cancer (such as non-small cell lung cancer).

In a particularly preferred embodiment, the disease, disorder or condition is a cancer positive for the ligand of LILRB1 and/or LILRB2 (such as HLA-G) and/or is a cancer characterised by the presence of LILRB1 and/or LILRB2 positive immune cells, in particular LILRB1 and/or LILRB2 positive monocytes and/or macrophages and/or is a cancer (or other proliferative disorder) characterised by being resistant and/or refractory to blockade of an immune checkpoint molecule (eg resistant and/or refractory to therapy for blockade of an immune checkpoint molecule), such as blockade using a ligand to an immune checkpoint molecule (as further described below, such as blockade of PD1/CTLA4; analogous to Gao et al, 2017). For example, in one such embodiment, the disease, disorder or condition can be a proliferative disorder (such as cancer) resistant and/or refractory to PD1/CTLA4 blockade therapy.

In a further particular embodiment, the disease, disorder or condition is an infectious disease (or a condition associated with such disorder or disease), in particular when the product or modulating compound (such as a ABP, ABD, nucleic acid, NAC or recombinant host cell of the invention, in particular an ABP of the invention) is an *inhibitor and*/*or antagonist* of the expression, function, activity and/or stability of LILRB1 and/or LILRB2 or a variant of LILRB1 and/or LILRB2.

The term "infectious disease" is art recognized, and as used herein includes those diseases, disorders or conditions associated with (eg resulting from or caused by) by any pathogen or agent that infects mammalian cells, preferable human cells. Examples of such pathogens include bacteria, yeast, fungi, protozoans, mycoplasma, viruses, prions, and parasites. Examples of infectious disease include( a) viral diseases such as, for example, diseases resulting from infection by an adenovirus, a herpesvirus (e.g., HSV-I, HSV-II, CMV, or VZV), a poxvirus (e∼g-, an orthopoxvirus such as variola or vaccinia, or molluscum contagiosum), a picornavirus (e.g., rhinovirus or enterovirus), an orthomyxovirus (e.g., influenza virus), a paramyxovirus (e.g., parainfluenza virus, mumps virus, measles virus, and respiratory syncytial virus (RSV)), a cononavirus (e.g., SARS), a papovavirus (e.g., papillomaviruses, such as those that cause genital warts, common warts, or plantar warts), a hepadnavirus (e.g., hepatitis B virus), a flavi virus (e.g., hepatitis C virus or Dengue virus), or a retrovirus (e.g., a lentivirus such as HIV); (b) bacterial diseases such as, for example, diseases resulting from infection by bacteria of, for example, the genus Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Haemophilus, or Bordetella; (c) other infectious diseases, such chlamydia, fungal diseases including but not limited to candidiasis, aspergillosis, histoplasmosis, cryptococcal meningitis, parasitic diseases including but not limited to malaria, Pneumocystis camii pneumonia, leishmaniasis, cryptosporidiosis, toxoplasmosis, and trypanosome infection and prions that cause human disease such as Creutzfeldt-Jakob Disease (CJD), variant Creutzfeldt-Jakob Disease (vCJD), Gerstmann-Straeussler-Scheinker syndrome, Fatal Familial Insomnia and kuru.

In yet another particular embodiment, the disease, disorder or condition is one associated with an over-active or immune system or an immune system displaying undesired activity, such as autoimmunity, allergy or inflammatory conditions, in particular for allergy, autoimmunity, transplant rejection, inflammation, graft vs host disease or sepsis (or a condition associated with such diseases, disorders or conditions), in particular when the product or modulating compound (such as a ABP, ABD, nucleic acid, NAC or recombinant host cell of the invention, in particular an ABP of the invention) is an *activator and*/*or agonist* of the expression, function, activity and/or stability of LILRB1 and/or LILRB2 or a variant of LILRB1 and/or LILRB2.

According to the medical uses and methods of treatment disclosed herein, the subject is a mammal, and may include mice, rats, rabbits, monkeys and humans. In a preferred embodiment, the mammalian subject is a human patient.

In one embodiment, cells involved in the proliferative disorder are resistant to a humoral or cell-mediated immune response. For example, cells involved in the proliferative disorder (eg cells of a cancer or tumour) are resistant and/or refractory to blockade of an immune checkpoint molecule such as blockade using a ligand to an immune checkpoint molecule, in exemplary instances blockade of PD1/CTLA4 (analogous to Gao et al, 2017).

In particular, the treatment methods may be applied to a proliferative disorder that has been subjected to prior immunotherapy (such as therapy for blockade of an immune checkpoint molecule, eg blockade of PD1/CTLA4), in particular prior immunotherapy with a ligand to an immune checkpoint molecule. For example, in certain embodiments the LILRB1 and/or LILRB2 (eg antagonist) modulator, such as an ABP of the present invention, can be for use in the treatment of a proliferative disorder in a subject in need thereof, and the subject has been subjected to to prior immunotherapy, in particular prior administration of a ligand to an immune checkpoint molecule.

In other methods, the modulating (eg inhibiting) compound (eg an ABP, such as one of the present invention) may be used is in combination with a different anti-proliferative therapy, in particular a different anti-cancer therapy, in particular where the different anti-proliferative therapy is immunotherapy, in particular immunotherapy with a ligand to an immune checkpoint molecule. Accordingly, the composition can be for use in the treatment of a proliferative disorder in a subject in need thereof, where the subject is subjected to to co-treatment by immunotherapy, in particular co-therapy (eg combination treatment) with a ligand to an immune checkpoint molecule.

In such embodiments, the ligand is one that binds to an immune (inhibitory) checkpoint molecule. For example, such checkpoint molecule may be one selected from the group consisting of: A2AR, B7-H3, B7-H4, CTLA-4, IDO, KIR, LAG3, PD-1 (or one of its ligands PD-L1 and PD-L2), TIM-3 (or its ligand galectin-9), TIGIT and VISTA. In particular of such embodiments, the ligand binds to a checkpoint molecule selected from: CTLA-4, PD-1 and PD-L1. In other more particular embodiments, the ligand is an antibody selected from the group consisting of: ipilimumab, nivolumab, pembrolizumab, BGB-A317, atezolizumab, avelumab and durvaluma; in particular an antibody selected from the group consisting of: ipilimumab (YERVOY), nivolumab (OPDIVO), pembrolizumab (KEYTRUDA) and atezolizumab (TECENTRIQ).

When a method or use in therapy of the present invention (eg, one involving an ABP of the invention) is used in combination treatments together with any of such other procedures (eg, another agent or a cancer immunotherapy, such as a ligand that binds to an immune (inhibitory) checkpoint molecule), then such method or use being a combination treatment regimen may comprise embodiments where such exposures/administrations are concomitant. In alternative embodiments, such administrations may be sequential; in particular those embodiments where the LILRB1 and/or LILRB2 modulator (eg an ABP of the invention) is administered before such other procedure. For example, such LILRB1 and/or LILRB2 modulator may be sequentially administered within about 14 days of (eg before) the other procedure, such as within about 10 days, 7 days, 5 days, 2 days or 1 day of (eg before) the other procedure; and further including where the LILRB1 and/or LILRB2 modulator may be sequentially administered within about 48 hours, 24 hours, 12 hours, 8 hours, 6 hours, 4 hours, 2 hours, 1 hours, 30 mins, 15 mins or 5 mins of (eg before) the other procedure.

In certain embodiments, the medical uses or compositions are for use in enhancing an immune response in the subject, preferably for use in aiding a cell-mediated immune response in the subject such as the subject's T cell mediated immune response, for example for treating a proliferative disease such as a cancer disease.

In particular embodiments, the treatment can comprise a transfer of cells to the subject, preferably a transfer of immune cells to the subject, more preferably an adoptive T-cell transfer. For example, such cells can be autologous cells of the subject, for example autologous immune cells, such as T-cells, dendritic cells or Natural Killer (NK)-cells, of the subject.

In a preferred embodiment of the medical uses or compositions, the modulating compound (eg ABP of the invention) is an inhibitor or antagonist of expression, function, activity and/or stability of said LILRB1 and/or LILRB2, or the variant of LILRB1 and/or LILRB2, and wherein the inhibition of the expression, function, activity and/or stability of said LILRB1 and/or LILRB2, or the variant of LILRB1 and/or LILRB2, enhances an immune response, preferably enhances a cell-mediated immune response in the subject such as a T-cell mediated immune response in the subject, for example for treating an infectious disease or a proliferative disease such as a cancer disease, in particular where the composition is an ABP of the invention.

In such embodiments, the immune response can be enhanced by an increase in T cell activity, proliferation and/or survival, in particular wherein the increase in T cell activity comprises an increase in production of one or more pro-inflammatory cytokines by such T cells (such as TILs). Preferably in such embodiments, the cytokine is one selected from the group consisting of: interleukin-1 (IL-1), IL-2, IL-12, IL-17, and IL-18, tumour necrosis factor (TNF) [alpha], interferon gamma (IFN-gamma), and granulocyte-macrophage colony stimulating factor, such as IL-2 (and/or IL-17 or IFN-gamma).

Such an increase in T cell activity, proliferation and/or survival, can be associated with the inhibition of the interaction between LILRB1 and/or LILRB2 and their ligand(s), in particular mediated by LILRB1 and/or LILRB2-mediated signaling.

Administration of the modulating (eg inhibiting) compound (eg an ABP of the invention) is, in certain embodiments, associated with the inhibition of the interaction between LILRB1 and/or LILRB2 and their ligand(s), in particular mediated by LILRB1 and/or LILRB2-mediated signaling.

In other certain embodiments, administration of the modulating compound (eg an ABP of the invention), decreases or reduces the resistance of cells (such as tumour cells and/or cells that express LILRB1 and/or LILRB2, or the variant of LILRB1 and/or LILRB2), to an immune response, preferably wherein the compound enhances or increases the sensitivity of cells (such as tumour cells and/or cells that express LILRB1 and/or LILRB2, or the variant of LILRB1 and/or LILRB2), to an immune response.

In preferred embodiments, the medical uses are for the treatment of a proliferative disorder (such as a cancer described herein) in a mammalian subject in need thereof. In certain of such embodiments, the subject is a mouse, rat, guinea pig, rabbit, cat, dog, monkey, or preferably a human, for example a human patient.

### Cells and methods of producing the ABPs/NACs of the invention

As described above, in one aspect, herein provided is a cell, such as (recombinant) host cell or a hybridoma capable of expressing an ABP as described above. In an alternative aspect, herein provided is a cell, which comprises at least one NAC encoding an ABP or a component of an ABP as described above. Cells of the invention can be used in methods provided herein to produce the ABPs and/or NACs of the invention.

In certain embodiments, the cell is isolated or substantially pure, and/or is a recombinant cell and/or is a non-natural cell (i.e., it is not found in, or is a product of, nature), such as a hybridoma.

Accordingly, in **another aspect** the invention relates to **a method of producing a recombinant cell line** capable of expressing an ABP specific for LILRB1 and/or LILRB2, or for a LILRB1 and/or LILRB2 variant, the method comprising the steps of:
- providing a suitable host cell;
- providing at least one genetic construct comprising coding sequence(s) encoding the ABP of the present invention;
- introducing into said suitable host cell said genetic construct(s); and
- optionally, expressing said genetic construct(s) by said suitable host cell under conditions that allow for the expression of the ABP.

In **yet another aspect,** herein provided is a **method of producing an ABP** as described above, for example comprising culturing one or more cells of the invention under conditions allowing the expression of said ABP.

Accordingly, in **another aspect,** the invention relates to **a method of producing an ABP** specific for LILRB1 and/or LILRB2, or for a LILRB1 and/or LILRB2 variant, the method comprising the steps of:
- providing a hybridoma or (host) cell capable of expressing an ABP according to the invention, for example a recombinant cell line comprising at least one genetic construct comprising coding sequence(s) encoding said compound or ABP; and
- culturing said hybridoma or host cell under conditions that allow for the expression of the ABP.

For producing the recombinant ABPs of the invention, the DNA molecules encoding the proteins (e.g. for antibodies, light and/or heavy chains or fragments thereof) are inserted into an expression vector (or NAC) such that the sequences are operatively linked to transcriptional and translational control sequences. Alternatively, DNA molecules encoding the ABP can be chemically synthesized. Synthetic DNA molecules can be ligated to other appropriate nucleotide sequences, including, e.g., constant region coding sequences, and expression control sequences, to produce conventional gene expression constructs encoding the desired ABP. For manufacturing the ABPs of the invention, the skilled artisan may choose from a great variety of expression systems well known in the art, e.g. those reviewed by Kipriyanow and Le Gall, 2004. Expression vectors include, but are not limited to, plasmids, retroviruses, cosmids, EBV-derived episomes, and the like. The term "expression vector" or "NAC" comprises any vector suitable for the expression of a foreign DNA. Examples of such expression vectors are viral vectors, such as adenovirus, vaccinia virus, baculovirus and adeno-associated virus vectors. In this connection, the expression "virus vector" is understood to mean both a DNA and a viral particle. Examples of phage or cosmid vectors include pWE15, M13, λEMBL3, λEMBL4, λFIXII, λDASHII, λZAPII, λgT10, λgt11, Charon4A and Charon21A. Examples of plasmid vectors include pBR, pUC, pBluescriptII, pGEM, pTZ and pET groups. Various shuttle vectors may be used, e.g., vectors which may autonomously replicate in a plurality of host microorganisms such as E. coli and Pseudomonas sp. In addition, artificial chromosome vectors are considered as expression vectors. The expression vector and expression control sequences are selected to be compatible with the cell, such as a host cell. Examples of mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRepS, D H26S, D HBB, pNMT1, pNMT41, pNMT81, which are available from InvitrogenTM, pCI which is available from Promega, pMbac, pPbac, pBK-RSV and pBK-CMV which are available from Agilent Technologies, pTRES which is available from Clontech, and their derivatives.

For manufacturing antibodies, the antibody light chain gene and the antibody heavy chain gene can be inserted into separate vectors. In certain embodiments, both DNA sequences are inserted into the same expression vector. Convenient vectors are those that encode a functionally complete human CH or CL immunoglobulin sequence, with appropriate restriction sites engineered so that any VH or VL sequence can be easily inserted and expressed, as described above, wherein the CH1 and/or upper hinge region comprises at least one amino acid modification of the invention. The constant chain is usually kappa or lambda for the antibody light chain. The recombinant expression vector may also encode a signal peptide that facilitates secretion of the antibody chain from a (host) cell. The DNA encoding the antibody chain may be cloned into the vector such that the signal peptide is linked in-frame to the amino terminus of the mature antibody chain DNA. The signal peptide may be an immunoglobulin signal peptide or a heterologous peptide from a non-immunoglobulin protein. Alternatively, the DNA sequence encoding the antibody chain may already contain a signal peptide sequence.

In addition to the DNA sequences encoding the ABP (antibody) chains, the recombinant expression vectors carry regulatory sequences including promoters, enhancers, termination and polyadenylation signals and other expression control elements that control the expression of the antibody chains in a (host) cell. Examples for promoter sequences (exemplified for expression in mammalian cells) are promoters and/or enhancers derived from CMV (such as the CMV Simian Virus 40 (SV40) promoter/enhancer), adenovirus, (e.g., the adenovirus major late promoter (AdMLP)), polyoma and strong mammalian promoters such as native immunoglobulin and actin promoters. Examples for polyadenylation signals are BGH polyA, SV40 late or early polyA; alternatively, 3'UTRs of immunoglobulin genes etc. can be used.

The recombinant expression vectors may also carry sequences that regulate replication of the vector in (host) cells (e.g. origins of replication) and selectable marker genes. Nucleic acid molecules encoding the heavy chain or an antigen-binding portion thereof and/or the light chain or an antigen-binding portion thereof of an antibody of the present invention, and vectors comprising these DNA molecules can be introduced into (host) cells, e.g. bacterial cells or higher eukaryotic cells, e.g. mammalian cells, according to transfection methods well known in the art, including liposome-mediated transfection, polycation-mediated transfection, protoplast fusion, microinjections, calcium phosphate precipitation, electroporation or transfer by viral vectors.

For antibodies or fragments thereof, it is within ordinary skill in the art to express the heavy chain and the light chain from a single expression vector or from two separate expression vectors. Preferably, the DNA molecules encoding the heavy chain and the light chain are present on two vectors which are co-transfected into the (host) cell, preferably a mammalian cell

Mammalian cell lines available as hosts for expression are well known in the art and include, inter alia, Chinese hamster ovary (CHO, CHO-DG44, BI-HEX-CHO) cells, NSO, SP2/0 cells, HeLa cells, HEK293 cells, baby hamster kidney (BHK) cells, monkey kidney cells (COS), human carcinoma cells (e.g., Hep G2), A549 cells, 3T3 cells or the derivatives/progenies of any such cell line. Other mammalian cells, including but not limited to human, mice, rat, monkey and rodent cells lines, or other eukaryotic cells, including but not limited to yeast, insect and plant cells, or prokaryotic cells such as bacteria may be used. The antibody molecules of the invention are produced by culturing the host cells for a period of time sufficient to allow for expression of the antibody molecule in the host cells.

According to some embodiments of the method of producing an ABP, following expression, the intact antibody (or the antigen-binding fragment of the antibody) can be harvested and isolated using purification techniques well known in the art, e.g., Protein A, Protein G, affinity tags such as glutathione-S-transferase (GST) and histidine tags.

ABPs are preferably recovered from the culture medium as a secreted polypeptide or can be recovered from host cell lysates if for example expressed without a secretory signal. It is necessary to purify the ABP molecules using standard protein purification methods used for recombinant proteins and host cell proteins in a way that substantially homogenous preparations of the ABP are obtained. By way of example, state-of-the art purification methods useful for obtaining the ABP molecule of the invention include, as a first step, removal of cells and/or particulate cell debris from the culture medium or lysate. The ABP is then purified from contaminant soluble proteins, polypeptides and nucleic acids, for example, by fractionation on immunoaffinity or ion-exchange columns, ethanol precipitation, reverse phase HPLC, Sephadex chromatography, chromatography on silica or on a cation exchange resin. Preferably, ABPs are purified by standard protein A chromatography, e.g., using protein A spin columns (GE Healthcare). Protein purity may be verified by reducing SDS PAGE. ABP concentrations may be determined by measuring absorbance at 280nm and utilizing the protein specific extinction coefficient. As a final step in the process for obtaining an ABP molecule preparation, the purified ABP molecule may be dried, e.g. lyophilized, for therapeutic applications.

Accordingly, certain embodiments of such aspects, the method comprises a further step of isolation and/or purification of the ABP.

In another aspect, herein provided is a method of manufacturing a pharmaceutical composition comprising an ABP as described above, comprising formulating the ABP isolated by the methods described above into a pharmaceutically acceptable form.

In an alternative aspect, herein provided is a method of manufacturing a pharmaceutical composition comprising an NAC as described above, comprising formulating the NAC prepared by the methods described above into a pharmaceutically acceptable form.

According to some embodiments, the methods of manufacturing a pharmaceutical composition comprise a further step of combining said ABP and/or NAC with a pharmaceutically acceptable excipient or carrier.

In some embodiments of the method of manufacturing a pharmaceutical composition comprising an ABP, the ABP typically will be labelled with a detectable labelling group before being formulated into a pharmaceutically acceptable form. Various methods for labelling proteins are known in the art and may be used. Suitable labelling groups include, but are not limited to, the following: radioisotopes or radionuclides (e.g., 3H, 14C, 15N, 35S, 90Y, 99Tc, 1111n, 1251, 1311), fluorescent groups (e.g., FITC, rhodamine, lanthanide phosphors), enzymatic groups (e.g., horseradish peroxidase, β- galactosidase, luciferase, alkaline phosphatase), chemiluminescent groups, biotinyl groups, or predetermined polypeptide epitopes recognized by a secondary reporter (e.g., leucine zipper pair sequences, binding sites for secondary antibodies, metal binding domains, epitope tags). In some embodiments, the labelling group is coupled to the ABP via spacer arms of various lengths to reduce potential steric hindrance.

Accordingly, in certain embodiments of such aspects, the ABP is a modified antibody and the method comprises a further step of addition of a functional moiety selected from a detectable labelling group or a cytotoxic moiety.

### Detection/diagnostic/monitoring methods

LILRB1 and/or LILRB2 can be used for diagnostic purposes to detect, diagnose, or monitor diseases, disorders and/or conditions associated with the undesired presence of LILRB1 and/or LILRB2-positive cells or cells positive for a variant of LILRB1 and/or LILRB2 and/or associated with cellular resistance against a cell-mediated immune response; and in particular aberrant and/or localised expression/activity of LILRB1 and/or LILRB2 (in particular phosphorylated LILRB1 and/or LILRB2) can be so used. The disease, disorder and/or conditions so detected, diagnosed, or monitored, can be one of those described elsewhere herein.

In **a related aspect,** the invention relates to **a method for determining** the presence or an amount of LILRB1 and/or LILRB2 (or a variant thereof) in a biological sample from a subject, the method comprising the steps of:
- contacting said sample with an ABP capable of binding to LILRB1 and/or LILRB2 (or the variant); and
- detecting binding between the LILRB1 and/or LILRB2 (or the variant) in the biological sample and the ABP.

In a preferred embodiment, protein LILRB1 and/or LILRB2 (or a variant thereof) is detected with a ABP of the invention.

In certain embodiments, a biological sample will (preferably) comprise cells or tissue of the subject, or an extract of such cells or tissue, in particular where such cells are those involved with the proliferative disorder (eg cells of the tumour microenvironment, or immune cells present at the site of the tumour). The tumour or cell thereof, may be one or, or derived from, one of the tumours described elsewhere herein.

In particular embodiments of such aspect, the method will also comprise a step of:
- providing (such as by obtaining) the biological sample from the subject, in particular where such step is conducted prior to the detection step.

In particular embodiments, such detection and/or determination methods can be practiced as a method of diagnosis, such as a method of diagnosis whether a mammalian subject (such as a human subject or patient) has a disease, disorder or condition (such as one described above), in particular a proliferative disorder such as a cancer or tumour (or has a risk of developing such a disease, disorder or condition) that is associated with the undesired presence of LILRB1 and/or LILRB2-positive cells or cells positive for a variant of LILRB1 and/or LILRB2 and/or that is associated with cellular resistance against a cell-mediated immune response and/or that is associated with (eg aberrant) expression or activity of LILRB1 and/or LILRB2 (or a variant thereof); in particular an immune cell of a (solid) tumour, such as one having cellular resistance against a cell-mediated immune response.

In certain embodiments of these detection, determination and/or diagnostic methods, the cellular resistance against a cell-mediated immune response is cellular resistance against a T cell-mediated immune response.

In certain embodiments, the biological sample is one obtained from a mammalian subject like a human patient. The term "biological sample" is used in its broadest sense and can refer to a bodily sample obtained from the subject (eg, a human patient). For example, the biological sample can include a clinical sample, i.e., a sample derived from a subject. Such samples can include, but are not limited to: peripheral bodily fluids, which may or may not contain cells, e.g., blood, urine, plasma, mucous, bile pancreatic juice, supernatant fluid, and serum; tissue or fine needle biopsy samples; tumour biopsy samples or sections (or cells thereof), and archival samples with known diagnosis, treatment and/or outcome history. Biological samples may also include sections of tissues, such as frozen sections taken for histological purposes. The term "biological sample" can also encompass any material derived by processing the sample. Derived materials can include, but are not limited to, cells (or their progeny) isolated from the biological sample, nucleic acids and/or proteins extracted from the sample. Processing of the biological sample may involve one or more of, filtration, distillation, extraction, amplification, concentration, fixation, inactivation of interfering components, addition of reagents, and the like.

Such detection, determination and/or diagnosis methods can be conducted as an in-vitro method, and can be, for example, practiced using the kit of the present invention (or components thereof).

In some embodiments of these detection, determination and/or diagnosis methods, the biological sample is a tissue sample from the subject, such as a sample of a tumour or a cancer from the subject. Such a sample may contain tumour cells and/or blood cells (eg monocytes and T cells). As described above, such tissue sample may be a biopsy sample of the tumour or a cancer such as a needle biopsy samples, or a tumour biopsy sections or an archival sample thereof. Such a tissue sample may comprise living, dead or fixed cells, such as from the tumour or a cancer, and such cells may be suspected of expressing (e.g. aberrantly or localised) the applicable biomarker to be determined.

In other embodiments of these detection, determination and/or diagnosis methods, the biological sample is a blood sample from the subject, such as a sample of immune cells present in blood (eg monocytes and T cells).

In some embodiments, determination and/or diagnosis method of the invention can comprise, such as in a further step, comparing the detected amount (or activity of) of (eg protein or mRNA of) the applicable biomarker (ie LILRB1 and/or LILRB2 or a variant thereof) with a standard or cut-off value; wherein a detected amount greater than the standard or cut-off value indicates a phenotype (or a risk of developing a phenotype) that is associated with the undesired presence of LILRB1 and/or LILRB2-positive cells (or cells positive for a variant of LILRB1 and/or LILRB2) and/or that it associated with cellular resistance against the cell-mediated immune response in the subject and/or is associated with (eg aberrant) expression or activity of LILRB1 and/or LILRB2 (or the variant) in the subject. Such a standard or cut-off value may be determined from the use of a control assay, or may be pre-determined from one or more values obtained from a study or a plurality of samples having known phenotypes. For example, a cut-off value for a diagnostic test may be determined by the analysis of samples taken from patients in the context of a controlled clinical study, and determination of a cut-off depending on the desired (or obtained) sensitivity and/or specificity of the test.

Examples of methods useful in the detection of (such as the presence or absence of, or an amount of) the applicable biomarker (ie the LILRB1 and/or LILRB2 or variant thereof) include immunoassays, such as the enzyme linked immunosorbent assay (ELISA) and the radioimmunoassay (RIA), which employ ABP (eg of the present invention) such as an antibody or an antigen-binding fragment thereof, that specifically binds to such applicable biomarker.

For such methods, a monoclonal antibody or a polyclonal antibody may be employed. Examples of monoclonal antibodies are described elsewhere herein. The term "polyclonal antibody" as used herein refers to a mixture of antibodies which are genetically different since produced by plasma cells derived from multiple somatic recombination and clonal selection events and which, typically, recognise a different epitope of the same antigen.

Alternatively, the presence of the applicable biomarker (ie LILRB1 and/or LILRB2 or variant thereof) may be detected by detection of the presence of mRNA that encodes such applicable biomarker, or fragments of such mRNA. Methods to detect the presence of such mRNA (or fragments) can include, PCR (such as quantitative RT-PCR), hybridisation (such as to Illumina chips), nucleic-acid sequencing etc. Such methods may involve or comprise steps using one or more nucleic acids as described herein, such as PCR primers or PCR probes, or hybridisation probes, that bind (eg specifically) to such mRNA.

For such detection, determination or diagnostic applications, the ABP or nucleic acid, typically, will be labelled with a detectable labelling group. In general, labelling groups fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, beta-galactosidase, luciferase, alkaline phosphatase); e) biotinylated groups; and f) predetermined polypeptide epitopes recognised by a secondary reporter (e.g., leucine zipper pair sequences, binding sites for secondary antibodies, metal binding domains, epitope tags, etc.).Suitable labelling groups include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent groups (e.g., FITC, rhodamine, lanthanide phosphors), enzymatic groups (e.g., horseradish peroxidase, beta-galactosidase, luciferase, alkaline phosphatase), chemiluminescent groups, biotinyl groups, or predetermined polypeptide epitopes recognised by a secondary reporter (eg, leucine zipper pair sequences, binding sites for secondary antibodies, metal binding domains, epitope tags). In some embodiments, the labelling group is coupled to the ABP or nucleic acid via spacer arms of various lengths to reduce potential steric hindrance. Various methods for labelling proteins are known in the art and may be used. For example, the ABP or nucleic acid may be labelled with a secondary reporter (e.g., leucine zipper pair sequences, binding sites for secondary antibodies, metal binding domains, epitope tags, etc.).

Accordingly, in particular embodiments of the detection/diagnostic methods (or the kits therefor), the means (eg ABP or nucleic acid) for the detection (eg detector) of protein or mRNA of the applicable biomarker (eg LILRB1 and/or LILRB2), is labelled, for example is coupled to a detectable label. The term "label" or "labelling group" refers to any detectable label, including those described herein.

In certain embodiments, the detection/diagnostic methods of the invention involve an immunohistochemistry (IHC) assay or an immunocytochemistry (IC) assay. The terms "IHC" and "ICC" are art recognised, and include the meanings of techniques employed to localise antigen expression that are dependent on specific epitope-antibody interactions. IHC typically refers to the use of tissue sections, whereas ICC typically describes the use of cultured cells or cell suspensions. In both methods, positive staining is typically visualised using a molecular label (eg, one which may be fluorescent or chromogenic). Briefly, samples are typically fixed to preserve cellular integrity, and then subjected to incubation with blocking reagents to prevent non-specific binding of the antibodies. Samples are subsequently typically incubated with primary (and sometimes secondary) antibodies, and the signal is visualised for microscopic analysis.

### Detection/diagnostic/monitoring kits:

In **a seventh aspect,** herein provided is a **kit,** such as one for performing the diagnostic methods or the determination methods or the detection methods (or the monitoring or prognostic methods) of the invention, eg, for determining the presence, absence, amount, function, activity and/or expression of the applicable biomarker (ie LILRB1 and/or LILRB2 or a variant thereof) in a sample (eg a biological sample), such as on cells in a sample. The kit comprises an ABP and/or a nucleic acid as described above and, optionally one or more additional components.

In certain embodiments of the kit, an additional component may comprise instructions describing how to use the ABP or a nucleic acid or kit, for detecting the presence of the applicable biomarker in the sample, such as by detecting binding between the ABP and protein such applicable biomarker, and/or detecting binding between the nucleic acid and mRNA of such applicable biomarker. Such instructions may consist of a printed manual or computer readable memory comprising such instructions, or may comprise instructions as to identify, obtain and/or use one or more other components to be used together with the kit.

In other certain embodiments of the kit, the additional component may comprise one or more other claim, component, reagent or other means useful for the use of the kit or practice of a detection method of the invention, including any such claim, component, reagent or means disclosed herein useful for such practice. For example, the kit may further comprise reaction and/or binding buffers, labels, enzymatic substrates, secondary antibodies and control samples, materials or moieties etc.

The present invention in context of the complete disclosure also relates in certain embodiments to the following itemized embodiments:
**Item 1**: An isolated antigen binding protein (ABP) which specifically binds to the extra cellular domain (ECD) of leukocyte immunoglobulin-like receptor subfamily B1 (LILRB1) and/or LILRB2 protein and wherein the isolated ABP comprises at least one complementarity determining region (CDR) and is able to inhibit the binding of LILRB1 and/or LILRB2 to a natural ligand thereof (a natural ligand of LILRB1 and/or LILRB2, such as HLA-G), preferably wherein the ABP does not bind to, or binds with less affinity to, a leukocyte immunoglobulin-like receptor subfamily A (LILRA) protein, such as LILRA1 and/or LILRA3.
**Item 2:** The isolated ABP of item 1, comprising at least one complementarity determining region 3 (CDR3) having an amino acid sequence with at least 90% sequence identity to, or having no more than three or two, preferably no more than one amino acid substitution(s), deletion(s) or insertion(s) compared to, a sequence selected from SEQ ID Nos. 3, 7, 11, 15, 19, 23, 27, 31, 35, 39, 43, 47, 51, 55, 59, 63, 67, 71, 75, 79, 83, 87, 91, 95, 99, 103, 107, 111, 115, 119, 123, 127, 131, 135, 139, 143, 147, 151, 155, 159, 163, 167, 171, 175, 179, 183, 187, 191, 195, 199, 203, 207, 211, 215, 219, 223, 227, 231, 235, 239, 243, 247, 251, 255, 259, 263, 267, 271, 275, 279, 283, 287, 291, 295, 299, 303, 307, 311, 315, 319, 323, 327, 331, 335, 339, 343, 347, and 351.
**Item 3:** The isolated ABP of item 1 or 2, wherein the ABP is an antibody, or an antigen binding fragment thereof, composed of at least one, preferably two, antibody heavy chain sequences, and at least one, preferably two, antibody light chain sequences, wherein at least one, preferably both, of the antibody heavy chain sequences and at least one, preferably both, of the antibody light chain sequences comprise CDR1 to CDR3 sequences in a combination selected from any of the following combinations of heavy and/or light chain CDRs, CDRs-A-001 to CDRs-A-044:

| **Combination (ID)** | **Heavy Chain CDR1 to CDR3 (SEQ ID NO)** | | | **Light Chain CDR1 to CDR3 (SEQ ID NO)** | | |
|---|---|---|---|---|---|---|
| CDRs-A-001 | 1 | 2 | 3 | 5 | 6 | 7 |
| CDRs-A-002 | 9 | 10 | 11 | 13 | 14 | 15 |
| CDRs-A-003 | 17 | 18 | 19 | 21 | 22 | 23 |
| CDRs-A-004 | 25 | 26 | 27 | 29 | 30 | 31 |
| CDRs-A-005 | 33 | 34 | 35 | 37 | 38 | 39 |
| CDRs-A-006 | 41 | 42 | 43 | 45 | 46 | 47 |
| CDRs-A-007 | 49 | 50 | 51 | 53 | 54 | 55 |
| CDRs-A-008 | 57 | 58 | 59 | 61 | 62 | 63 |
| CDRs-A-009 | 65 | 66 | 67 | 69 | 70 | 71 |
| CDRs-A-010 | 73 | 74 | 75 | 77 | 78 | 79 |
| CDRs-A-011 | 81 | 82 | 83 | 85 | 86 | 87 |
| CDRs-A-012 | 89 | 90 | 91 | 93 | 94 | 95 |
| CDRs-A-013 | 97 | 98 | 99 | 101 | 102 | 103 |
| CDRs-A-014 | 105 | 106 | 107 | 109 | 110 | 111 |
| CDRs-A-015 | 113 | 114 | 115 | 117 | 118 | 119 |
| CDRs-A-016 | 121 | 122 | 123 | 125 | 126 | 127 |
| CDRs-A-017 | 129 | 130 | 131 | 133 | 134 | 135 |
| CDRs-A-018 | 137 | 138 | 139 | 141 | 142 | 143 |
| CDRs-A-019 | 145 | 146 | 147 | 149 | 150 | 151 |
| CDRs-A-020 | 153 | 154 | 155 | 157 | 158 | 159 |
| CDRs-A-021 | 161 | 162 | 163 | 165 | 166 | 167 |
| CDRs-A-022 | 169 | 170 | 171 | 173 | 174 | 175 |
| CDRs-A-023 | 177 | 178 | 179 | 181 | 182 | 183 |
| CDRs-A-024 | 185 | 186 | 187 | 189 | 190 | 191 |
| CDRs-A-025 | 193 | 194 | 195 | 197 | 198 | 199 |
| CDRs-A-026 | 201 | 202 | 203 | 205 | 206 | 207 |
| CDRs-A-027 | 209 | 210 | 211 | 213 | 214 | 215 |
| CDRs-A-028 | 217 | 218 | 219 | 221 | 222 | 223 |
| CDRs-A-029 | 225 | 226 | 227 | 229 | 230 | 231 |
| CDRs-A-030 | 233 | 234 | 235 | 237 | 238 | 239 |
| CDRs-A-031 | 241 | 242 | 243 | 245 | 246 | 247 |
| CDRs-A-032 | 249 | 250 | 251 | 253 | 254 | 255 |
| CDRs-A-033 | 257 | 258 | 259 | 261 | 262 | 263 |
| CDRs-A-034 | 265 | 266 | 267 | 269 | 270 | 271 |
| CDRs-A-035 | 273 | 274 | 275 | 277 | 278 | 279 |
| CDRs-A-036 | 281 | 282 | 283 | 285 | 286 | 287 |
| CDRs-A-037 | 289 | 290 | 291 | 293 | 294 | 295 |
| CDRs-A-038 | 297 | 298 | 299 | 301 | 302 | 303 |
| CDRs-A-039 | 305 | 306 | 307 | 309 | 310 | 311 |
| CDRs-A-040 | 313 | 314 | 315 | 317 | 318 | 319 |
| CDRs-A-041 | 321 | 322 | 323 | 325 | 326 | 327 |
| CDRs-A-042 | 329 | 330 | 331 | 333 | 334 | 335 |
| CDRs-A-043 | 337 | 338 | 339 | 341 | 342 | 343 |
| CDRs-A-044 | 345 | 346 | 347 | 349 | 350 | 351 |

in each case independently, optionally with no more than three or two, preferably no more than one, amino acid substitution(s), insertion(s) or deletion(s) compared to these sequences.
**Item 4:** The isolated ABP of any one of items 1 to 3, wherein the ABP is an antibody, or an antigen binding fragment thereof, composed of at least one, preferably two, antibody heavy chain sequences, and at least one, preferably two, antibody light chain sequences,
(i) wherein at least one, preferably both, of the antibody heavy chain sequences each comprises heavy chain CDR1 to CDR3 sequences in the combination CDRs-A-010 and at least one, preferably both, of the antibody light chain sequences each comprises light chain CDR1 to CDR3 sequences in the combination CDRs-A-010, in each case independently, optionally with no more than one amino acid substitution(s), insertion(s) or deletion(s) compared to these sequences, and preferably wherein the ABP specifically binds to LILRB1 and/or LILRB2 protein or a variant thereof with an IC50 of 10nM or less, preferably of about 5nM or less; or
(ii) wherein at least one, preferably both, of the antibody heavy chain sequences each comprises heavy chain CDR1 to CDR3 sequences in the combination CDRs-A-026 and at least one, preferably both, of the antibody light chain sequences each comprises light chain CDR1 to CDR3 sequences in the combination CDRs-A-026, in each case independently, optionally with no more than one amino acid substitution(s), insertion(s) or deletion(s) compared to these sequences, and preferably wherein the ABP specifically binds to LILRB1 and/or LILRB2 protein or a variant thereof with an IC50 of 10nM or less, preferably of about 5nM or less.
**Item 5:** An isolated ABP which competes with an ABP as recited in any one of items 1 to 4 for binding to the ECD of the LILRB1 and/or LILRB2 protein and is able to inhibit the binding of the LILRB1 and/or LILRB2 protein or the variant thereof to a natural ligand of LILRB1 and/or LILRB2, preferably wherein the isolated ABP.
**Item 6:** The isolated ABP of any one of items 1 to 5 that specifically binds to LILRB1 and/or LILRB2 with a KD of 100nM or less (preferably 50nM or less, more preferably 10nM or less); preferably wherein the isolated ABP binds to LILRA, such as LILRA1 and/or LILRA3, with a KD of 50nM or more (preferably of 100nM or more, more preferably of 200nM or more).
**Item 7:** The isolated ABP of any one of items 1 to 6 that (i) inhibits a binding of a natural ligand of LILRB1 and/or LILRB2 to LILRB1 and/or LILRB2, wherein such ligand is preferably HLA-G, and/or (ii) reduces development of immune-suppressive phenotypes, for example in a tumour microenvironment.
**Item 8:** The isolated ABP of any one of items 1 to 7that is an antibody or an antigen binding fragment thereof, wherein the antibody is a monoclonal antibody, or wherein the antigen binding fragment is a fragment of a monoclonal antibody.
**Item 9:** The isolated ABP of any one of items 1 to 8 that is an antibody or an antigen binding fragment thereof, wherein the antibody is a human antibody a humanised antibody or a chimeric-human antibody, or wherein the antigen binding fragment is a fragment of a human antibody a humanised antibody or a chimeric-human antibody.
**Item 10:** An isolated nucleic acid encoding for an ABP, or for an antigen binding fragment or a monomer of an ABP, wherein the ABP is one recited in any one of items 1 to 9.
**Item 11:** A recombinant host cell comprising a nucleic acid recited in item 10.
**Item 12:** A pharmaceutical composition comprising: (i) an ABP recited in any one of items 1 to 9, or (ii) a nucleic acid recited in item 10, or (iii) a recombinant host cell of item 11, and a pharmaceutically acceptable carrier, stabiliser and/or excipient.
**Item 13:** A product for use in medicine, wherein the product is selected from the list consisting of: (i) an isolated ABP recited in any one of items 1 to 9, and (ii) an isolated nucleic acid recited in item 10, (iii) a recombinant host cell of item 11, and (iv) a pharmaceutical composition of item 12.
**Item 14:** The product for use in medicine of item 13 wherein the product is for use in the treatment of an infective disorder or a proliferative disorder that is associated with a suppressed cell-mediated immune response due to the expression or activity of LILRB1 and/or LILRB2 or a variant thereof.
**Item 15:** The product for use in medicine of item 14, wherein cells involved in the proliferative disorder express a natural ligand of LILRB1 and/or LILRB2, and wherein such cells induce an immune-suppressive phenotype in immune cells, such as macrophages.
**Item 16:** The product for use in medicine of any one of items 13 to 15, wherein the product is for use in enhancing an immune response in a mammalian subject, preferably for use in promoting immune-activation of a humoral or cell-mediated immune response in the subject, such as promoting pro-inflammatory polarization of macrophages towards an M1 phenotype, for example for treating a proliferative disease, such as a cancer disease, of for treating an infectious disease.
**Item 17:** The product for use in medicine of any one of items 13 to 16, wherein the product is for use in the treatment of a proliferative disorder resistant and/or refractory to PD1/CTLA4 blockade therapy.
**Item 18:** The product for use of any one of items 13 to 17, wherein the product has any one or any combination of the following characteristics:
a. specific binding to human LILRB1 and/or (preferably and) LILRB2 (e.g. comprising the amino acid sequence of SEQ ID NO: 353 (LILRB1) and SEQ ID NO: 358 (LILRB2)), e.g., with a KD 50 nM or less, or of 20nM or less; more preferably of 10nM or less or 5nM or less
b. lack of specific binding to a LILRA protein, such as preferably LILRA1 and/or LILRA3;
c. stimulates T cell activation, e.g., in a mixed lymphocyte reaction (MLR) assay, as measured by increased T cell proliferation or IFN-gamma secretion, e.g.;
d. stimulates differentiation or activation of monocytes into macrophages, e.g., stimulates differentiation of monocytes into pro-inflammatory macrophages, e.g., as shown in an assay described in the Examples;
e. inhibits binding of LILRB1 and/or (preferably and) LILRB2 to HLA-A and HLA-B, preferably to HLA-G;
f. has a binding profile as shown in **Table 2;**
g. promotes pro-inflammatory polarization of macrophages towards M1 macrophages; and
h. does not induce (or trigger) basophil activation.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Generation and Maturation of antibodies that bind to human LILRB1 and/or LILRB2.

Affinity improved LILRB2 and LILRB1 cross-specific antibodies were selected by phage display from antibody gene libraries based on the parental V gene sequence with diversified CDR-H1/H2 and CDR-L3, respectively. For each parental sequence, two diversified libraries were constructed: (i) keeping the light chain constant and diversifying CDR-H1 and CDR-H2, and (ii) keeping the heavy chain constant and diversifying CDR-L3. CDR-H1/2 diversified libraries contained between 8e8 and 4e9 derivatives, CDR-L3 diversified libraries contained more than 2e7 derivatives of the respective parental sequence. The diversification of the CDR sequences was based on a rational design.

To select higher affinity binders and improve LILRB2/LILRB1 cross-specificity and simultaneously reducing LILRA1/LILRA3 binding, optimized selection conditions were applied. Briefly, the antibody phage libraries were blocked with 2x ChemiBLOCKER (Merck Millipore) and pre-adsorbed on streptavidin coated magnetic beads loaded with biotinylated LILRA1 and LILRA3. In the first panning round, the biotinylated recombinant target protein (LILRB1) was added at a concentration of 10 nM and incubated for 1 h at room temperature with 20-fold molar excess of non-biotinylated recombinant LILRA1 and LILRA3. Antibody phage bound to recombinant LILRB1 were separated using streptavidin magnetic beads (Dynabeads M-280, ThermoFisher) and washed with DPBST including an extended washing step for 30 min in presence of 100 nM non-biotinylated target protein and 500 nM non-biotinylated recombinant LILRA1 and LILRA3. The antibody phage particles were eluted using 10 ug/mL Trypsin and used to infect mid-logarithmic E. coli TG1 for phage amplification.

Panning rounds two and three were performed equivalently to panning round one with the following modifications to increase the selection pressure for higher affinity: The concentration of biotinylated recombinant target protein (LILRB2 in panning round two and LILRB1 in panning round three) was further limited (1 nM in panning round two and 0.5 nM in panning round three). After capturing the biotinylated antigen with the bound antibody phage on streptavidin magnetic beads, an initial washing step with DPBST was performed. To further increase the stringency of the washing and select for slower dissociation rates, the beads were suspended in 1.5 mL of PBST containing 100 nM of non-biotinylated recombinant target protein and 100 nM non-biotinylated LILRA1 and LILRA3 and incubated for up to 20 h at room temperature.

Enrichment of higher affinity binders and the optimal selection stringency was monitored by determining the phage titers in the selection output of each condition and panning round.

Parental and maturated scFv antibodies of the invention that selectively bind human LILRB1 and/or LILRB2 protein were identified and described in **Table 1,** showing for each such antibody the heavy chain and light chain CDR sequences and variable region sequences comprised in each such antibody.

Antibodies A-001, A-002 and A-003 constitute the selected parental antibodies. Antibodies A-004 to A-048 constitute derivative antibodies of a heavy and/or light chain sequence of one or two of the parental molecules. In particular, A-004 to A-021 are heavy chain (HC) variants of parental antibody A-001. A-022 to A-033 are HC variants of parental antibody A-003. A-034 to A-036 are light chain (LC) variants of parental antibody A-001, A-037 is an LC variant of A-002. A-038 to A-044 are LC variants of parental antibody A-003.

Antibody A-045 is a derivative of antibody A-001 and comprises the HC of A-010 and the LC of A-036. A-046 is a derivative of antibody A-001 and comprises the HC of A-015 and the LC of A-036. A-047 is combination of A-001 and A-003 derived HC and LC chains, and comprises the HC of A-025 and the LC of A-038. A-048 is combination of A-001 and A-003 derived HC and LC chains, and comprises the HC of A-025 and the LC of A-039.

**Table 1: Amino acid sequences of CDR and variable regions of ABPs of the invention, as well as nucleic acid sequences encoding variable regions of ABPs of the invention.**

| **Antibody-ID** | **Region** | **Sequence** | **SEQ ID NO.** |
|---|---|---|---|
| A-001 | H-CDR1 | SYAIS | 1 |
| | H-CDR2 | GIIPIFGTANYAQKFQG | 2 |
| | H-CDR3 | DLGTYSSGWPYYYGMDV | 3 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKDLGTY SSGWPYYYGMDVWGQGTTVTVSS | 4 |
| | L-CDR1 | SGDKLGDKYAS | 5 |
| | L-CDR2 | QDSKRPS | 6 |
| | L-CDR3 | QSTTHTSFV | 7 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 8 |
| A-002 | H-CDR1 | SYAIS | 9 |
| | H-CDR2 | GIIPIFGTANYAQKFQG | 10 |
| | H-CDR3 | VHLYYHFPASYFDY | 11 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCARVHLYY HFPASYFDYWGQGTLVTVSS | 12 |
| | L-CDR1 | SGDKLGDKYAS | 13 |
| | L-CDR2 | QDSKRPS | 14 |
| | L-CDR3 | QVTPGLGGVYV | 15 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQVTPGLGGVYVFGGGTK LTVL | 16 |
| A-003 | H-CDR1 | SYAIS | 17 |
| | H-CDR2 | GIIPIFGTANYAQKFQG | 18 |
| | H-CDR3 | GPPVGSSSWYTDWYFDL | 19 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 20 |
| | L-CDR1 | SGDKLGDKYAS | 21 |
| | L-CDR2 | QDSKRPS | 22 |
| | L-CDR3 | LSYGAHSHGW | 23 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 24 |
| A-004 | H-CDR1 | TKAIA | 25 |
| | H-CDR2 | GIIPIFSTANYAPKFQG | 26 |
| | H-CDR3 | DLGTYSSGWPYYYGMDV | 27 |
| | VH (aa) | QVQLVQSGAEVKKPGSSVKVSCKASGGTFRTKAIAWVRQAPGQGLEWMGG IIPIFSTANYAPKFQGRVTITADESTSTAYMELSSLRSEDTAVYYCAKDLGTYS SGWPYYYGMDVWGQGTTVTVSS | 28 |
| | L-CDR1 | SGDKLGDKYAS | 29 |
| | L-CDR2 | QDSKRPS | 30 |
| | L-CDR3 | QSTTHTSFV | 31 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 32 |
| A-005 | H-CDR1 | IYAIN | 33 |
| | H-CDR2 | GIIPGFHSANYAQKFQG | 34 |
| | H-CDR3 | DLGTYSSGWPYYYGMDV | 35 |
| | VH (aa) | QVQLVQSGAEVKKPGSSVKVSCKASGGTFTIYAINWVRQAPGQGLEWMGG IIPGFHSANYAQKFQGRVTITADESTSTAYMELSSLRSEDTAVYYCAKDLGTY SSGWPYYYGMDVWGQGTTVTVSS | 36 |
| | L-CDR1 | SGDKLGDKYAS | 37 |
| | L-CDR2 | QDSKRPS | 38 |
| | L-CDR3 | QSTTHTSFV | 39 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 40 |
| A-006 | H-CDR1 | QYAFS | 41 |
| | H-CDR2 | SIIPGFTTSNYAHKFQG | 42 |
| | H-CDR3 | DLGTYSSGWPYYYGMDV | 43 |
| | VH (aa) | QVQLVQSGAEVKKPGSSVKVSCKASGGTFSQYAFSWVRQAPGQGLEWMGS IIPGFTTSNYAHKFQGRVTITADESTSTAYMELSSLRSEDTAVYYCAKDLGTY SSGWPYYYGMDVWGQGTTVTVSS | 44 |
| | L-CDR1 | SGDKLGDKYAS | 45 |
| | L-CDR2 | QDSKRPS | 46 |
| | L-CDR3 | QSTTHTSFV | 47 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 48 |
| A-007 | H-CDR1 | EYAFS | 49 |
| | H-CDR2 | SIIPTFHTSNYAGKFQG | 50 |
| | H-CDR3 | DLGTYSSGWPYYYGMDV | 51 |
| | VH (aa) | QVQLVQSGAEVKKPGSSVKVSCKASGGSFREYAFSWVRQAPGQGLEWMGS IIPTFHTSNYAGKFQGRVTITADESTSTAYMELSSLRSEDTAVYYCAKDLGTY SSGWPYYYGMDVWGQGTTVTVSS | 52 |
| | L-CDR1 | SGDKLGDKYAS | 53 |
| | L-CDR2 | QDSKRPS | 54 |
| | L-CDR3 | QSTTHTSFV | 55 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 56 |
| A-008 | H-CDR1 | ESAIL | 57 |
| | H-CDR2 | GIVPSFYSANYAQKFQG | 58 |
| | H-CDR3 | DLGTYSSGWPYYYGMDV | 59 |
| | VH (aa) | QVQLVQSGAEVKKPGSSVKVSCKASGGTFAESAILWVRQAPGQGLEWMGG IVPSFYSANYAQKFQGRVTITADESTSTAYMELSSLRSEDTAVYYCAKDLGTY SSGWPYYYGMDVWGQGTTVTVSS | 60 |
| | L-CDR1 | SGDKLGDKYAS | 61 |
| | L-CDR2 | QDSKRPS | 62 |
| | L-CDR3 | QSTTHTSFV | 63 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 64 |
| A-009 | H-CDR1 | VTAIL | 65 |
| | H-CDR2 | GIIPSFTSANYASKFQG | 66 |
| | H-CDR3 | DLGTYSSGWPYYYGMDV | 67 |
| | VH (aa) | QVQLVQSGAEVKKPGSSVKVSCKASGGTFSVTAILWVRQAPGQGLEWMGG IIPSFTSANYASKFQGRVTITADESTSTAYMELSSLRSEDTAVYYCAKDLGTY SSGWPYYYGMDVWGQGTTVTVSS | 68 |
| | L-CDR1 | SGDKLGDKYAS | 69 |
| | L-CDR2 | QDSKRPS | 70 |
| | L-CDR3 | QSTTHTSFV | 71 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 72 |
| A-010 | H-CDR1 | SQAFA | 73 |
| | H-CDR2 | SIIPGFSTANYAQKFQG | 74 |
| | H-CDR3 | DLGTYSSGWPYYYGMDV | 75 |
| | VH (aa) | QVQLVQSGAEVKKPGSSVKVSCKASGGTFQSQAFAWVRQAPGQGLEWMG SIIPGFSTANYAQKFQGRVTITADESTSTAYMELSSLRSEDTAVYYCAKDLGT YSSGWPYYYGMDVWGQGTTVTVSS | 76 |
| | L-CDR1 | SGDKLGDKYAS | 77 |
| | L-CDR2 | QDSKRPS | 78 |
| | L-CDR3 | QSTTHTSFV | 79 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 80 |
| A-011 | H-CDR1 | HSAFS | 81 |
| | H-CDR2 | SIIPAFYTANYAQKFQG | 82 |
| | H-CDR3 | DLGTYSSGWPYYYGMDV | 83 |
| | VH (aa) | QVQLVQSGAEVKKPGSSVKVSCKASGGSFSHSAFSWVRQAPGQGLEWMGS IIPAFYTANYAQKFQGRVTITADESTSTAYMELSSLRSEDTAVYYCAKDLGTY SSGWPYYYGMDVWGQGTTVTVSS | 84 |
| | L-CDR1 | SGDKLGDKYAS | 85 |
| | L-CDR2 | QDSKRPS | 86 |
| | L-CDR3 | QSTTHTSFV | 87 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 88 |
| A-012 | H-CDR1 | EYAFL | 89 |
| | H-CDR2 | GIIPKFHTANYAQKFQG | 90 |
| | H-CDR3 | DLGTYSSGWPYYYGMDV | 91 |
| | VH (aa) | QVQLVQSGAEVKKPGSSVKVSCKASGGTISEYAFLWVRQAPGQGLEWMGG IIPKFHTANYAQKFQGRVTITADESTSTAYMELSSLRSEDTAVYYCAKDLGTY SSGWPYYYGMDVWGQGTTVTVSS | 92 |
| | L-CDR1 | SGDKLGDKYAS | 93 |
| | L-CDR2 | QDSKRPS | 94 |
| | L-CDR3 | QSTTHTSFV | 95 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 96 |
| A-013 | H-CDR1 | QEAFS | 97 |
| | H-CDR2 | SIIPIFYSSNYAQKFQG | 98 |
| | H-CDR3 | DLGTYSSGWPYYYGMDV | 99 |
| | VH (aa) | QVQLVQSGAEVKKPGSSVKVSCKASGGTFRQEAFSWVRQAPGQGLEWMGS IIPIFYSSNYAQKFQGRVTITADESTSTAYMELSSLRSEDTAVYYCAKDLGTY SSGWPYYYGMDVWGQGTTVTVSS | 100 |
| | L-CDR1 | SGDKLGDKYAS | 101 |
| | L-CDR2 | QDSKRPS | 102 |
| | L-CDR3 | QSTTHTSFV | 103 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 104 |
| A-014 | H-CDR1 | SYAIN | 105 |
| | H-CDR2 | GIIPIFHTSNYAQKFQG | 106 |
| | H-CDR3 | DLGTYSSGWPYYYGMDV | 107 |
| | VH (aa) | QVQLVQSGAEVKKPGSSVKVSCKASGGTFSSYAINWVRQAPGQGLEWMGG IIPIFHTSNYAQKFQGRVTITADESTSTAYMELSSLRSEDTAVYYCAKDLGTY SSGWPYYYGMDVWGQGTTVTVSS | 108 |
| | L-CDR1 | SGDKLGDKYAS | 109 |
| | L-CDR2 | QDSKRPS | 110 |
| | L-CDR3 | QSTTHTSFV | 111 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 112 |
| A-015 | H-CDR1 | SYAIN | 113 |
| | H-CDR2 | GIIPGFSTANYAQKFQG | 114 |
| | H-CDR3 | DLGTYSSGWPYYYGMDV | 115 |
| | VH (aa) | QVQLVQSGAEVKKPGSSVKVSCKASGGSFQSYAINWVRQAPGQGLEWMGG IIPGFSTANYAQKFQGRVTITADESTSTAYMELSSLRSEDTAVYYCAKDLGTY SSGWPYYYGMDVWGQGTTVTVSS | 116 |
| | L-CDR1 | SGDKLGDKYAS | 117 |
| | L-CDR2 | QDSKRPS | 118 |
| | L-CDR3 | QSTTHTSFV | 119 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 120 |
| A-016 | H-CDR1 | SYAFN | 121 |
| | H-CDR2 | SIIPIFKTANYAQKFQG | 122 |
| | H-CDR3 | DLGTYSSGWPYYYGMDV | 123 |
| | VH (aa) | QVQLVQSGAEVKKPGSSVKVSCKASGGTFSSYAFNWVRQAPGQGLEWMGS IIPIFKTANYAQKFQGRVTITADESTSTAYMELSSLRSEDTAVYYCAKDLGTY SSGWPYYYGMDVWGQGTTVTVSS | 124 |
| | L-CDR1 | SGDKLGDKYAS | 125 |
| | L-CDR2 | QDSKRPS | 126 |
| | L-CDR3 | QSTTHTSFV | 127 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 128 |
| A-017 | H-CDR1 | SYAIS | 129 |
| | H-CDR2 | GIIPYFSLANYAPKFQG | 130 |
| | H-CDR3 | DLGTYSSGWPYYYGMDV | 131 |
| | VH (aa) | QVQLVQSGAEVKKPGSSVKVSCKASGGTFSSYAISWVRQAPGQGLEWMGG IIPYFSLANYAPKFQGRVTITADESTSTAYMELSSLRSEDTAVYYCAKDLGTY SSGWPYYYGMDVWGQGTTVTVSS | 132 |
| | L-CDR1 | SGDKLGDKYAS | 133 |
| | L-CDR2 | QDSKRPS | 134 |
| | L-CDR3 | QSTTHTSFV | 135 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 136 |
| A-018 | H-CDR1 | TSAIS | 137 |
| | H-CDR2 | GIIPIFYTANYAQKFQG | 138 |
| | H-CDR3 | DLGTYSSGWPYYYGMDV | 139 |
| | VH (aa) | QVQLVQSGAEVKKPGSSVKVSCKASGGTFSTSAISWVRQAPGQGLEWMGG IIPIFYTANYAQKFQGRVTITADESTSTAYMELSSLRSEDTAVYYCAKDLGTY SSGWPYYYGMDVWGQGTTVTVSS | 140 |
| | L-CDR1 | SGDKLGDKYAS | 141 |
| | L-CDR2 | QDSKRPS | 142 |
| | L-CDR3 | QSTTHTSFV | 143 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 144 |
| A-019 | H-CDR1 | TSAMN | 145 |
| | H-CDR2 | GIIPYFSTANYAPKFQG | 146 |
| | H-CDR3 | DLGTYSSGWPYYYGMDV | 147 |
| | VH (aa) | QVQLVQSGAEVKKPGSSVKVSCKASGYTFTTSAMNWVRQAPGQGLEWMG GIIPYFSTANYAPKFQGRVTITADESTSTAYMELSSLRSEDTAVYYCAKDLGT YSSGWPYYYGMDVWGQGTTVTVSS | 148 |
| | L-CDR1 | SGDKLGDKYAS | 149 |
| | L-CDR2 | QDSKRPS | 150 |
| | L-CDR3 | QSTTHTSFV | 151 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 152 |
| A-020 | H-CDR1 | TYAIS | 153 |
| | H-CDR2 | SIIPKFYQSNYAYKFQG | 154 |
| | H-CDR3 | DLGTYSSGWPYYYGMDV | 155 |
| | VH (aa) | QVQLVQSGAEVKKPGSSVKVSCKASGGTFQTYAISWVRQAPGQGLEWMGS IIPKFYQSNYAYKFQGRVTITADESTSTAYMELSSLRSEDTAVYYCAKDLGTY SSGWPYYYGMDVWGQGTTVTVSS | 156 |
| | L-CDR1 | SGDKLGDKYAS | 157 |
| | L-CDR2 | QDSKRPS | 158 |
| | L-CDR3 | QSTTHTSFV | 159 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 160 |
| A-021 | H-CDR1 | SYAFA | 161 |
| | H-CDR2 | GIIPGFYAANYAQKFQG | 162 |
| | H-CDR3 | DLGTYSSGWPYYYGMDV | 163 |
| | VH (aa) | QVQLVQSGAEVKKPGSSVKVSCKASGGSITSYAFAWVRQAPGQGLEWMGG IIPGFYAANYAQKFQGRVTITADESTSTAYMELSSLRSEDTAVYYCAKDLGTY SSGWPYYYGMDVWGQGTTVTVSS | 164 |
| | L-CDR1 | SGDKLGDKYAS | 165 |
| | L-CDR2 | QDSKRPS | 166 |
| | L-CDR3 | QSTTHTSFV | 167 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQSTTHTSFVFGGGTKLT VL | 168 |
| A-022 | H-CDR1 | GSAIS | 169 |
| | H-CDR2 | GIVLAYSTSVYAQKFQG | 170 |
| | H-CDR3 | GPPVGSSSWYTDWYFDL | 171 |
| | VH (aa) | QVQLVQSGAEVKKPGSSVKVSCKASGGTFGGSAISWVRQAPGQGLEWMGG IVLAYSTSVYAQKFQGRVTITADESTSTAYMELSSLRSEDTAVYYCAKGPPVG SSSWYTDWYFDLWGRGTLVTVSS | 172 |
| | L-CDR1 | SGDKLGDKYAS | 173 |
| | L-CDR2 | QDSKRPS | 174 |
| | L-CDR3 | LSYGAHSHGW | 175 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 176 |
| A-023 | H-CDR1 | QYAMS | 177 |
| | H-CDR2 | GIIPQFSTSVYAQKFQG | 178 |
| | H-CDR3 | GPPVGSSSWYTDWYFDL | 179 |
| | VH (aa) | QVQLVQSGAEVKKPGSSVKVSCKASGYTFQQYAMSWVRQAPGQGLEWIGG IIPQFSTSVYAQKFQGRVTITADESTSTAYMELSSLRSEDTAVYYCAKGPPVG SSSWYTDWYFDLWGRGTLVTVSS | 180 |
| | L-CDR1 | SGDKLGDKYAS | 181 |
| | L-CDR2 | QDSKRPS | 182 |
| | L-CDR3 | LSYGAHSHGW | 183 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 184 |
| A-024 | H-CDR1 | GSAMS | 185 |
| | H-CDR2 | GILPIFGTSVYAQKFQG | 186 |
| | H-CDR3 | GPPVGSSSWYTDWYFDL | 187 |
| | VH (aa) | QVQLVQSGAEVKKPGSSVKVSCKASGYTFTGSAMSWVRQAPGQGLEWMG GILPIFGTSVYAQKFQGRVTITADESTSTAYMELSSLRSEDTAVYYCAKGPPV GSSSWYTDWYFDLWGRGTLVTVSS | 188 |
| | L-CDR1 | SGDKLGDKYAS | 189 |
| | L-CDR2 | QDSKRPS | 190 |
| | L-CDR3 | LSYGAHSHGW | 191 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 192 |
| A-025 | H-CDR1 | KSAMS | 193 |
| | H-CDR2 | GIVPKFHTSVYAQKFQG | 194 |
| | H-CDR3 | GPPVGSSSWYTDWYFDL | 195 |
| | VH (aa) | QVQLVQSGAEVKKPGSSVKVSCKASGYTFSKSAMSWVRQAPGQGLEWMG GIVPKFHTSVYAQKFQGRVTITADESTSTAYMELSSLRSEDTAVYYCAKGPP VGSSSWYTDWYFDLWGRGTLVTVSS | 196 |
| | L-CDR1 | SGDKLGDKYAS | 197 |
| | L-CDR2 | QDSKRPS | 198 |
| | L-CDR3 | LSYGAHSHGW | 199 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 200 |
| A-026 | H-CDR1 | SHGMS | 201 |
| | H-CDR2 | GILPIFHTATYAQKFQG | 202 |
| | H-CDR3 | GPPVGSSSWYTDWYFDL | 203 |
| | VH (aa) | QVQLVQSGAEVKKPGSSVKVSCKASGYTFTSHGMSWVRQAPGQGLEWMG GILPIFHTATYAQKFQGRVTITADESTSTAYMELSSLRSEDTAVYYCAKGPPV GSSSWYTDWYFDLWGRGTLVTVSS | 204 |
| | L-CDR1 | SGDKLGDKYAS | 205 |
| | L-CDR2 | QDSKRPS | 206 |
| | L-CDR3 | LSYGAHSHGW | 207 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 208 |
| A-027 | H-CDR1 | GYAIS | 209 |
| | H-CDR2 | GILPSFYTAVYAQKFQG | 210 |
| | H-CDR3 | GPPVGSSSWYTDWYFDL | 211 |
| | VH (aa) | QVQLVQSGAEVKKPGSSVKVSCKASGGSFTGYAISWVRQAPGQGLEWMGG ILPSFYTAVYAQKFQGRVTITADESTSTAYMELSSLRSEDTAVYYCAKGPPVG SSSWYTDWYFDLWGRGTLVTVSS | 212 |
| | L-CDR1 | SGDKLGDKYAS | 213 |
| | L-CDR2 | QDSKRPS | 214 |
| | L-CDR3 | LSYGAHSHGW | 215 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 216 |
| A-028 | H-CDR1 | EHAMS | 217 |
| | H-CDR2 | GIIPKFSTSVFAQKFQG | 218 |
| | H-CDR3 | GPPVGSSSWYTDWYFDL | 219 |
| | VH (aa) | QVQLVQSGAEVKKPGSSVKVSCKASGYSFSEHAMSWVRQAPGQGLEWMG GIIPKFSTSVFAQKFQGRVTITADESTSTAYMELSSLRSEDTAVYYCAKGPPV GSSSWYTDWYFDLWGRGTLVTVSS | 220 |
| | L-CDR1 | SGDKLGDKYAS | 221 |
| | L-CDR2 | QDSKRPS | 222 |
| | L-CDR3 | LSYGAHSHGW | 223 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 224 |
| A-029 | H-CDR1 | IYAMS | 225 |
| | H-CDR2 | GIIPSFRTAVYAQKFQG | 226 |
| | H-CDR3 | GPPVGSSSWYTDWYFDL | 227 |
| | VH (aa) | QVQLVQSGAEVKKPGSSVKVSCKASGYTFSIYAMSWVRQAPGQGLEWMGG IIPSFRTAVYAQKFQGRVTITADESTSTAYMELSSLRSEDTAVYYCAKGPPVG SSSWYTDWYFDLWGRGTLVTVSS | 228 |
| | L-CDR1 | SGDKLGDKYAS | 229 |
| | L-CDR2 | QDSKRPS | 230 |
| | L-CDR3 | LSYGAHSHGW | 231 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 232 |
| A-030 | H-CDR1 | SYAFS | 233 |
| | H-CDR2 | GIVPIYYTATYAQKFQG | 234 |
| | H-CDR3 | GPPVGSSSWYTDWYFDL | 235 |
| | VH (aa) | QVQLVQSGAEVKKPGSSVKVSCKASGGSFQSYAFSWVRQAPGQGLEWMGG IVPIYYTATYAQKFQGRVTITADESTSTAYMELSSLRSEDTAVYYCAKGPPVG SSSWYTDWYFDLWGRGTLVTVSS | 236 |
| | L-CDR1 | SGDKLGDKYAS | 237 |
| | L-CDR2 | QDSKRPS | 238 |
| | L-CDR3 | LSYGAHSHGW | 239 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 240 |
| A-031 | H-CDR1 | KTAMS | 241 |
| | H-CDR2 | GIIPSYYTAVYAQKFQG | 242 |
| | H-CDR3 | GPPVGSSSWYTDWYFDL | 243 |
| | VH (aa) | QVQLVQSGAEVKKPGSSVKVSCKASGYTFTKTAMSWVRQAPGQGLEWMG GIIPSYYTAVYAQKFQGRVTITADESTSTAYMELSSLRSEDTAVYYCAKGPPV GSSSWYTDWYFDLWGRGTLVTVSS | 244 |
| | L-CDR1 | SGDKLGDKYAS | 245 |
| | L-CDR2 | QDSKRPS | 246 |
| | L-CDR3 | LSYGAHSHGW | 247 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 248 |
| A-032 | H-CDR1 | SYAFS | 249 |
| | H-CDR2 | GIVPQYSTAVYAQKFQG | 250 |
| | H-CDR3 | GPPVGSSSWYTDWYFDL | 251 |
| | VH (aa) | QVQLVQSGAEVKKPGSSVKVSCKASGGTFQSYAFSWVRQAPGQGLEWMGG IVPQYSTAVYAQKFQGRVTITADESTSTAYMELSSLRSEDTAVYYCAKGPPV GSSSWYTDWYFDLWGRGTLVTVSS | 252 |
| | L-CDR1 | SGDKLGDKYAS | 253 |
| | L-CDR2 | QDSKRPS | 254 |
| | L-CDR3 | LSYGAHSHGW | 255 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 256 |
| A-033 | H-CDR1 | SHAMS | 257 |
| | H-CDR2 | GIIPTFKTAVFAQKFQG | 258 |
| | H-CDR3 | GPPVGSSSWYTDWYFDL | 259 |
| | VH (aa) | QVQLVQSGAEVKKPGSSVKVSCKASGYTFSSHAMSWVRQAPGQGLEWMG GIIPTFKTAVFAQKFQGRVTITADESTSTAYMELSSLRSEDTAVYYCAKGPPV GSSSWYTDWYFDLWGRGTLVTVSS | 260 |
| | L-CDR1 | SGDKLGDKYAS | 261 |
| | L-CDR2 | QDSKRPS | 262 |
| | L-CDR3 | LSYGAHSHGW | 263 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLSYGAHSHGVVFGGGTK LTVL | 264 |
| A-034 | H-CDR1 | SYAIS | 265 |
| | H-CDR2 | GIIPIFGTANYAQKFQG | 266 |
| | H-CDR3 | DLGTYSSGWPYYYGMDV | 267 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKDLGTY SSGWPYYYGMDVWGQGTTVTVSS | 268 |
| | L-CDR1 | SGDKLGDKYAS | 269 |
| | L-CDR2 | QDSKRPS | 270 |
| | L-CDR3 | QLYGHTITV | 271 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQLYGHTITVFGGGTKLT VL | 272 |
| A-035 | H-CDR1 | SYAIS | 273 |
| | H-CDR2 | GIIPIFGTANYAQKFQG | 274 |
| | H-CDR3 | DLGTYSSGWPYYYGMDV | 275 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKDLGTY SSGWPYYYGMDVWGQGTTVTVSS | 276 |
| | L-CDR1 | SGDKLGDKYAS | 277 |
| | L-CDR2 | QDSKRPS | 278 |
| | L-CDR3 | QLSHGTLLV | 279 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQLSHGTLLVFGGGTKLT VL | 280 |
| A-036 | H-CDR1 | SYAIS | 281 |
| | H-CDR2 | GIIPIFGTANYAQKFQG | 282 |
| | H-CDR3 | DLGTYSSGWPYYYGMDV | 283 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKDLGTY SSGWPYYYGMDVWGQGTTVTVSS | 284 |
| | L-CDR1 | SGDKLGDKYAS | 285 |
| | L-CDR2 | QDSKRPS | 286 |
| | L-CDR3 | QLTHLTIYV | 287 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQLTHLTIYVFGGGTKLTV L | 288 |
| A-037 | H-CDR1 | SYAIS | 289 |
| | H-CDR2 | GIIPIFGTANYAQKFQG | 290 |
| | H-CDR3 | VHLYYHFPASYFDY | 291 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCARVHLYY HFPASYFDYWGQGTLVTVSS | 292 |
| | L-CDR1 | SGDKLGDKYAS | 293 |
| | L-CDR2 | QDSKRPS | 294 |
| | L-CDR3 | LASLGTGGVYV | 295 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCLASLGTGGVYVFGGGTK LTVL | 296 |
| A-038 | H-CDR1 | SYAIS | 297 |
| | H-CDR2 | GIIPIFGTANYAQKFQG | 298 |
| | H-CDR3 | GPPVGSSSWYTDWYFDL | 299 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 300 |
| | L-CDR1 | SGDKLGDKYAS | 301 |
| | L-CDR2 | QDSKRPS | 302 |
| | L-CDR3 | SLYGAGHTW | 303 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCSLYGAGHTVVFGGGTKL TVL | 304 |
| A-039 | H-CDR1 | SYAIS | 305 |
| | H-CDR2 | GIIPIFGTANYAQKFQG | 306 |
| | H-CDR3 | GPPVGSSSWYTDWYFDL | 307 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 308 |
| | L-CDR1 | SGDKLGDKYAS | 309 |
| | L-CDR2 | QDSKRPS | 310 |
| | L-CDR3 | ALYGGGHTW | 311 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCALYGGGHTVVFGGGTKL TVL | 312 |
| A-040 | H-CDR1 | SYAIS | 313 |
| | H-CDR2 | GIIPIFGTANYAQKFQG | 314 |
| | H-CDR3 | GPPVGSSSWYTDWYFDL | 315 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 316 |
| | L-CDR1 | SGDKLGDKYAS | 317 |
| | L-CDR2 | QDSKRPS | 318 |
| | L-CDR3 | SLYGPAHTGV | 319 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCSLYGPAHTGVFGGGTKL TVL | 320 |
| A-041 | H-CDR1 | SYAIS | 321 |
| | H-CDR2 | GIIPIFGTANYAQKFQG | 322 |
| | H-CDR3 | GPPVGSSSWYTDWYFDL | 323 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 324 |
| | L-CDR1 | SGDKLGDKYAS | 325 |
| | L-CDR2 | QDSKRPS | 326 |
| | L-CDR3 | SSYGSGLTW | 327 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCSSYGSGLTVVFGGGTKL TVL | 328 |
| A-042 | H-CDR1 | SYAIS | 329 |
| | H-CDR2 | GIIPIFGTANYAQKFQG | 330 |
| | H-CDR3 | GPPVGSSSWYTDWYFDL | 331 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 332 |
| | L-CDR1 | SGDKLGDKYAS | 333 |
| | L-CDR2 | QDSKRPS | 334 |
| | L-CDR3 | GVYSSGLTW | 335 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCGVYSSGLTVVFGGGTKL TVL | 336 |
| A-043 | H-CDR1 | SYAIS | 337 |
| | H-CDR2 | GIIPIFGTANYAQKFQG | 338 |
| | H-CDR3 | GPPVGSSSWYTDWYFDL | 339 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 340 |
| | L-CDR1 | SGDKLGDKYAS | 341 |
| | L-CDR2 | QDSKRPS | 342 |
| | L-CDR3 | SAYGSGLAW | 343 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCSAYGSGLAVVFGGGTKL TVL | 344 |
| A-044 | H-CDR1 | SYAIS | 345 |
| | H-CDR2 | GIIPIFGTANYAQKFQG | 346 |
| | H-CDR3 | GPPVGSSSWYTDWYFDL | 347 |
| | VH (aa) | QVQLVQSGAEVKKPGSSVKVSCKASGGTFSSYAISWVRQAPGQGLEWMGG IIPIFGTANYAQKFQGRVTITADESTSTAYMELSSLRSEDTAVYYCAKGPPVG SSSWYTDWYFDLWGRGTLVTVSS | 348 |
| | L-CDR1 | SGDKLGDKYAS | 349 |
| | L-CDR2 | QDSKRPS | 350 |
| | L-CDR3 | SLYASGLIAV | 351 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCSLYASGLIAVFGGGTKLT VL | 352 |
| A-045 | H-CDR1 | SQAFA | 353 |
| | H-CDR2 | SIIPGFSTANYAQKFQG | 354 |
| | H-CDR3 | DLGTYSSGWPYYYGMDV | 355 |
| | VH (aa) | QVQLVQSGAEVKKPGSSVKVSCKASGGTFQSQAFAWVRQAPGQGLEWMGS IIPGFSTANYAQKFQGRVTITADESTSTAYMELSSLRSEDTAVYYCAKDLGTY SSGWPYYYGMDVWGQGTTVTVSS | 356 |
| | L-CDR1 | SGDKLGDKYAS | 357 |
| | L-CDR2 | QDSKRPS | 358 |
| | L-CDR3 | QLTHLTIYV | 359 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQLTHLTIYVFGGGTKLTV L | 360 |
| A-046 | H-CDR1 | SYAIN | 361 |
| | H-CDR2 | GIIPGFSTANYAQKFQG | 362 |
| | H-CDR3 | DLGTYSSGWPYYYGMDV | 363 |
| | VH (aa) | QVQLVQSGAEVKKPGSSVKVSCKASGGSFQSYAINWVRQAPGQGLEWMGG IIPGFSTANYAQKFQGRVTITADESTSTAYMELSSLRSEDTAVYYCAKDLGTY SSGWPYYYGMDVWGQGTTVTVSS | 364 |
| | L-CDR1 | SGDKLGDKYAS | 365 |
| | L-CDR2 | QDSKRPS | 366 |
| | L-CDR3 | QLTHLTIYV | 367 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCQLTHLTIYVFGGGTKLTV L | 368 |
| A-047 | H-CDR1 | KSAMS | 369 |
| | H-CDR2 | GIVPKFHTSVYAQKFQG | 370 |
| | H-CDR3 | GPPVGSSSWYTDWYFDL | 371 |
| | VH (aa) | QVQLVQSGAEVKKPGSSVKVSCKASGYTFSKSAMSWVRQAPGQGLEWMGG IVPKFHTSVYAQKFQGRVTITADESTSTAYMELSSLRSEDTAVYYCAKGPPVG SSSWYTDWYFDLWGRGTLVTVSS | 372 |
| | L-CDR1 | SGDKLGDKYAS | 373 |
| | L-CDR2 | QDSKRPS | 374 |
| | L-CDR3 | SLYGAGHTW | 375 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCSLYGAGHTVVFGGGTKLT VL | 376 |
| A-048 | H-CDR1 | KSAMS | 377 |
| | H-CDR2 | GIVPKFHTSVYAQKFQG | 378 |
| | H-CDR3 | GPPVGSSSWYTDWYFDL | 379 |
| | VH (aa) | QVQLVQSGAEVKKPGSSVKVSCKASGYTFSKSAMSWVRQAPGQGLEWMGG IVPKFHTSVYAQKFQGRVTITADESTSTAYMELSSLRSEDTAVYYCAKGPPVG SSSWYTDWYFDLWGRGTLVTVSS | 380 |
| | L-CDR1 | SGDKLGDKYAS | 381 |
| | L-CDR2 | QDSKRPS | 382 |
| | L-CDR3 | ALYGGGHTW | 383 |
| | VL (aa) | SYELTQPPSVSVSPGQTASITCSGDKLGDKYASWYQQKPGQSPVLVIYQDSK RPSGIPERFSGSNSGNTATLTISGTQAEDEADYYCALYGGGHTVVFGGGTKL TVL | 384 |

### Example 2: Target and off-target antibody binding affinity

To identify affinity-improved binders that were specific to LILRB2 and LILRB1 but not to LILRA1 and LILRA3, monoclonal Fabs were expressed in *E. coli* after the second and third panning round. After bacterial lysis, the Fabs were tested for their binding properties and cross-reactivity profile on recombinant LILRB2, LILRB1, LILRA1 and LILRA3 by standard ELISA in a 1:100 or 1:25 dilution for the maturation of the heavy chain and light chain, respectively. Briefly, biotinylated recombinant LILRB2, LILRB1, LILRA1, and LILRA3 were immobilized at 1 ug/mL on a streptavidin-coated 384-well Maxisorp plate. The surface was blocked with 2% (w/v) bovine serum albumin (BSA) in PBST. After three wash cycles with PBST, the bacterial lysates in 2% (w/v) BSA were applied to the immobilized antigens and incubated for 1.5 h. After removing all unbound antibodies by 3 wash cycles with PBST, bound Fab antibodies were detected with a goat anti-human Fab antibody conjugated with horseradish peroxidase. After three wash cycles with PBST, the ELISA was developed with TMB substrate.

ELISA-positive hits with desired cross-reactivity profile and improved binding over the parent clone were subsequently analyzed for their cell binding properties by standard multiplex flow cytometry. Briefly, ExpiCHO transiently transfected with a construct expressing the extracellular domain (ECD) of LILRB2, LILRB1, and LILRA1, or LILRA3 fused to an intracellular GFP-tag, were stained with different concentrations (50 nM and unstained) of CellTrace^{™} violet (Invitrogen) to perform multiplex flow cytometry analysis (LILRB2 vs. LILRA1 and LILRB1 vs. LILRA3). The differently stained cell lines were mixed in a 1:1 ratio and 30,000 cells were incubated with E. coli lysates containing the monoclonal Fabs in 384 well format. Unbound antibodies were removed by washing the cells three time with FACS buffer (DPBS + 3% (v/v) FCS). Bound antibodies were detected with a mouse anti-human Fab antibody conjugated with AlexaFluor647. Dead cells were excluded by Zombie Yellow Dye (Biolegend) staining and the median fluorescent intensity (MFI) of AlexaFluor647 was analyzed for the two differently CellTrace^{™} violet (Invitrogen) stained cell populations to determine specific binding to cellular expressed LILRB2 and LILRB1 and reduced binding to LILRA1 and LILRA3.

The dissociation rates (off-rate) of the best cell binders were analyzed by biolayer interferometry (BLI) on an OctetRED96e system in a standard kinetic experiment using recombinant LILRB2 and LILRB1 protein. Briefly, biotinylated recombinant LILRB2 or LILRB1 was immobilized on streptavidin coated biosensors. The sensors were dipped in E. coli lysates containing the monoclonal Fabs diluted 1:1 with 2x Kinetics Buffer supplemented with 20 ug/mL biocytin and 4% (w/v) BSA and antibodies were associated for 420 s. Subsequently, the sensors were dipped into 1x Kinetics Buffer supplemented with 10 ug/mL biocytin and 2% (w/v) BSA to measure the dissociation for 420 s. All sensorgrams were double referenced against 1x Kinetics Buffer supplemented with 10 ug/mL biocytin and 2% (w/v) BSA, and empty streptavidin sensors to subtract sensor drifts and potential reference binding of the bacterial lysates. Dissociation rates were fitted using a 1:1 binding model.

Affinities were determined by BLI on the Octet RED96e system using the ECDs of recombinant LILRB2, LILRB1, LILRA1, and LILRA3. Briefly, IgGs were captured on anti-human capture (AHC) biosensors (Sartorius). The sensors were dipped into the wells containing 100 nM of recombinant LILRB2 or LILRB1 ECDs, or 900 nM of LILRA1 ECD or LILRA3 and incubated for 300 s to measure association. Subsequently, the sensors were dipped into 1x Kinetics Buffer to measure the dissociation over 420 s. All sensorgrams were double referenced against 1x Kinetics Buffer and unloaded streptavidin sensors to subtract sensor drifts and potential reference binding of the analytes. The affinities were determined by applying a 1:1 binding model.

Binding curves of the antibody clones of the invention are provided in **Figure 2****.** Dissociation constants (KD) are shown for the tested antibodies in Table 2 below.

Surprisingly, all antibody clones of the invention, parental and maturated, show cross specificity between the "on" targets LILRB1 and LILRB2 while being significantly lower in binding to both "off" targets LILRA1 and LILRA3.

### Example 3: Inhibition of LILRB1 and LILRB2 interaction with HLA-G

HLA-G inhibition was tested by ELISA. 384-well Maxisorp plates were coated with 2ug/mL B2M-HLA-G fused to a mouse IgG2a-Fc and the plates were blocked with 2% (w/v) BSA in PBST. 30nM biotinylated LILRB2 ECD or LILRB1 ECD were mixed with a 1:1000 dilution of Avidin-HRP (Biolegend, 405103) in a 1:1 volumetric ratio and incubated for 1h at room temperature. 15ul of the antibody dilution series (300nM to 0.3nM) were mixed with 30uL of the pre-mixed LILRB/Avidin-HRP solution (final antibody concentration 100nM to 0.1nM) and incubated for 1h at room temperature. After three wash cycles with PBST, 20uL of the antibody/LILRB/Avidin-HRP solution was transferred to the plate with immobilized HLA-G and incubated for 15min at room temperature. After three wash cycles with PBST, residual LILRB binding to HLA-G was detected by developing the ELISA with TMB substrate. The respective binding curves are shown in Figures 3, 4 and 5. IC50 values of the inhibition LILRB1/2-HLA-G binding are provided in Table 2.

**Table 2: Results of the biochemical assays. Provided are dissociation constants (KD) for binding target or off target proteins and IC50 for ligand binding inhibition.**

| **Antibody-ID** | **KD, monovalent (nM)** | | | | **IC50, HLA-G inhibition (nM)** | |
|---|---|---|---|---|---|---|
| | LILRB2 | LILRB1 | LILRA1 | LILRA3 | LILRB2 | LILRB1 |
| A-001 | 36.4 | 157.2 | 1824.0 | 575.7 | 3.1 | 5.1 |
| A-004 | 7.1 | 12.8 | 569.3 | 232.1 | 4.5 | 4.4 |
| A-005 | 3.3 | 1.0 | 407.3 | 161.0 | 4.3 | 5.0 |
| A-006 | 2.3 | 0.5 | 190.0 | 182.4 | 4.8 | 6.1 |
| A-007 | 1.2 | 11.7 | 322.2 | 255.0 | 3.6 | 3.3 |
| A-008 | 8.8 | 7.4 | 1521.0 | 354.8 | 4.0 | 4.1 |
| A-009 | 3.4 | 3.7 | 193.5 | 92.8 | 5.5 | 5.5 |
| A-010 | 3.3 | 4.5 | 760.0 | 217.0 | 3.7 | 4.1 |
| A-011 | 4.4 | 9.3 | 470.0 | 136.8 | 5.5 | 6.1 |
| A-012 | 6.8 | 12.8 | 469.1 | 166.1 | 3.8 | 3.3 |
| A-013 | 4.2 | 6.3 | 1084.0 | 621.5 | 4.6 | 4.5 |
| A-014 | 2.3 | 4.4 | 470.9 | 58.8 | 4.2 | 3.8 |
| A-015 | 3.5 | 5.0 | 867.5 | 201.5 | 3.2 | 4.4 |
| A-016 | 1.4 | 6.5 | 402.3 | 115.5 | 5.1 | 4.4 |
| A-017 | 1.7 | 2.3 | 591.2 | 223.0 | 4.4 | 4.5 |
| A-018 | 2.7 | 6.2 | 808.0 | 211.5 | 5.1 | 5.0 |
| A-019 | 3.9 | 20.7 | 1027.0 | 144.0 | 4.9 | 4.0 |
| A-020 | 2.9 | 21.2 | 315.7 | 391.9 | 3.8 | 3.7 |
| A-021 | 3.1 | 4.9 | 540.8 | 157.1 | 5.0 | 4.2 |
| A-045 | 2.8 | 2.3 | 962.0 | 575.0 | 3.5 | 2.1 |
| A-046 | 9.4 | 12.6 | n.b. | 2600.0 | 3.7 | 2.0 |
| A-003 | 73.1 | 99.4 | 342.5 | 519.0 | 65.8 | 16.5 |
| A-022 | 4.4 | 5.2 | 126.1 | 317.3 | 3.1 | 3.9 |
| A-023 | 4.3 | 3.1 | 82.4 | 229.5 | 6.8 | 7.5 |
| A-024 | 3.6 | 1.2 | 31.8 | 64.8 | 3.3 | 3.9 |
| A-025 | 4.2 | 1.8 | 71.3 | 166.5 | 4.3 | 5.1 |
| A-026 | 3.9 | 4.4 | 119.9 | 79.3 | 5.3 | 6.1 |
| A-027 | 1.9 | 0.5 | 33.9 | 124.5 | 5.6 | 7.0 |
| A-028 | 1.6 | 1.8 | 48.3 | 187.0 | 3.9 | 3.9 |
| A-029 | 2.7 | 1.9 | 29.5 | 123.1 | 5.6 | 6.5 |
| A-030 | 3.2 | 3.2 | 63.8 | 123.5 | 4.4 | 6.7 |
| A-031 | 0.9 | 0.8 | 11.6 | 50.1 | 4.2 | 4.1 |
| A-032 | 2.5 | 2.2 | 79.5 | 253.1 | 7.6 | 10.5 |
| A-033 | 1.4 | 0.5 | 23.0 | 84.8 | 8.6 | 8.3 |
| A-047 | 1.7 | 2.6 | 128.0 | 318.0 | 4.6 | 1.9 |
| A-048 | 5.6 | 8.7 | 1070.0 | 1970.0 | 4.1 | 2.0 |
| A-034 | 82.7 | 16.4 | 2686.0 | 677.7 | 5.6 | 3.3 |
| A-035 | 30.8 | 12.9 | 734.1 | 1248.0 | 3.5 | 1.7 |
| A-036 | 18.6 | 35.8 | 42930.0 | 5475.0 | 2.7 | 1.2 |
| A-037 | 2.0 | 16.0 | 1266.0 | 30820.0 | 3.5 | 3.8 |
| A-038 | 4.8 | 6.7 | 208.6 | 447.5 | 3.4 | 2.2 |
| A-039 | 13.7 | 21.6 | 1033.0 | 1547.0 | 2.4 | 2.0 |
| A-040 | 0.3 | 6.6 | 51.6 | 345.4 | 7.0 | 2.1 |
| A-041 | 5.8 | 17.5 | 242.2 | 464.6 | 4.4 | 1.5 |
| Ref047 | 3.4 | n.b. | n.b. | n.b. | 3.1 | n.b. |
| Ref051 | n.b. | 6.9 | 15.1 | 39.4 | n.b. | 1.8 |
| Ref062 | 0.4 | 0.5 | 0.6 | 1.4 | 4.7 | 5.7 |

| | | | | | | |
|---|---|---|---|---|---|---|
| n.b.=non-binding | | | | | | |

### Example 4.1: Cross-specific LILRB1/2 antibodies of the invention decrease M2-like polarization of monocyte derived macrophages

Functional myeloid assay was performed to characterize the effects of cross-specific LILRB1/2 antibodies and a reference monospecific LILRB2 antibody on macrophage phenotype and function. M0 Macrophages were generated by culture of isolated monocytes in media (RPMI with 10%FBS + 1%P/S) containing 50ng/ml recombinant human M-CSF (Peprotech) and indicated concentrations of in-house cross-specific LILRB1/2 antibodies, reference cross-specific LILRB1/2 antibody (Ref062 [NGM707]), reference monospecific LILRB2 antibody (Ref047 [Merck MK-4830]), or appropriate isotype control (Ref001) for 6 days at 37°C in 6-well tissue culture plates. M0 macrophages were removed from the plate with a cell scraper and polarized to M2-like macrophages in media (RPMI with 10%FBS + 1%P/S) containing 20ng/ml recombinant human IL-4 (Peprotech), 20ng/ml recombinant human IL-10 (Peprotech), and 20ng/ml recombinant human TGF-β (Peprotech) and indicated concentrations of in-house cross-specific LILRB1/2 antibodies, reference cross-specific LILRB1/2 antibody (Ref062 [NGM707]), reference monospecific LILRB2 antibody (Ref047 [Merck MK-4830]), or appropriate isotype control (Ref001) for 2 days at 37°C in 6-well tissue culture plates. On day 8, supernatant was taken for analysis of cytokines via Luminex Assay (R&D Systems) and M2-like macrophages were analyzed by flow cytometry for various surface markers indicative of an M2-like phenotype.

As shown in Figure 6A, cross-specific LILRB1/2 antibodies as well as monospecific LILRB2 antibody induced a significant decrease in M2-like macrophage phenotype marker CD163, consistent with a less suppressive M2-like polarization of the macrophages. In line with this, cross-specific LILRB1/2 antibodies as well as monospecific LILRB2 antibody also significantly decrease the secretion of immunosuppressive CCL13 and CCL23 cytokines of macrophages after M2-like polarization (see Figure 6B and C). Strikingly, all cross-specific LILRB1/2 antibodies are decreasing M2 marker CD163 and immunosuppressive cytokines at a much lower concentration than the monospecific LILRB2 antibody, indicating superior potency when both LILRB1 and 2 is blocked. Furthermore, comparing the cross-specific LILRB1/2 antibodies, A-010 even shows higher efficacy than reference molecule Ref062.

Such results further demonstrate the ability of antibodies of the present invention to selectively bind to both LILRB1 and LILRB2 while not binding to their counter targets LILRA1 and LILRA3. Furthermore, the cross-specific antibodies of the invention effectively decrease M2 like macrophage polarization and thereby provide means to reduce immune-suppressive functions of the LILRB-HLA-G axis, for example in the tumour microenvironment.

### Example 4.2: Cross-specific LILRB1/2 antibodies of the invention decrease M2-like polarization of monocyte derived macrophages and rescue cytotoxic T cell activation

The myeloid suppression assay was performed as the functional myeloid assay described above. However, on day 8, after supernatant was taken for analysis of myeloid cytokines, isolated autologous T cells were added to the macrophages at a ratio of 1:5 for a co-culture for an additional 3 days. On day 11, supernatant was again taken for analysis of T cell cytokines via Luminex Assay (R&D Systems) and T cells were analyzed by flow cytometry for surface activation markers.

Cross-specific LILRB1/2 antibody A-045 and reference molecule Ref-062 as well as monospecific LILRB2 antibody Ref-047 induced a significant decrease in M2-like macrophage phenotype marker CD163 (Figure 6D), consistent with a less suppressive M2-like polarization of the macrophages. In line with this, all antibodies also significantly decrease the secretion of immunosuppressive CCL18 after M2-like polarization (Figure 6E). Looking at rescue of T cell activation, all antibodies lead to upregulation of early activation marker CD69 on T cells and increased secretion of pro-inflammatory cytokines GM-CSF and IFNg (Figure 6 F - H). Compared to the reference molecules, A-045 shows superior potency specifically in T cell rescue.

### Example 5: Cellular binding of cross-specific LILRB1/2 antibodies of the invention

Cellular binding of A-045 was analyzed in Expi293 cells, transfected with targets of interest, and in primary T cells and monocytes.

Antibody binding to transfected Expi293 cells was performed as follows: Expi293 cells were transiently transfected to express proteins of interest LILRB1, LILRB2, LILRA1, LILRA2, and LILRA3. Non-transfected Expi293 cells were used as negative control. Cells were harvested and labeled with CellTraceViolet for multiplexing. Three cell lines each were mixed and incubated in FACS buffer (DPBS +3%FBS) with a titration of our cross-specific A-045, LILRB2 mono-specific reference antibody (Ref047 [Merck]), cross-specific LILRB1/2 reference antibody (Ref062 [NGM]), or an appropriate isotype control in a 384-well plate for 1 hour at 4°C. Subsequently, cells were washed and stained with a secondary anti-human IgG labeled with AF647 for 30 minutes at 4°C in the dark. Cells were washed again and mixed with 7-AAD viability dye before flow acquisition to test for surface binding.

Antibody binding to primary T cells and monocytes was tested similarly. Briefly, primary monocytes from healthy donors were isolated from whole blood using Ficoll and Percoll isolation. T cells were isolated from healthy donor whole blood using CD3 Fab-TACS Agarose human kit. Isolated cells were counted and incubated with human FC block for 20 minutes at 4°C.

Cells were then incubated in FACS buffer with a titration of our cross-specific A-045, LILRB2 mono-specific reference antibody (Ref047 [Merck]), cross-specific LILRB1/2 reference antibody (Ref062 [NGM]), or an appropriate isotype control in a 96-well plate for 1 hour at 4°C. Subsequently, cells were washed and stained with a secondary anti-human IgG labeled with AF647 for 30 minutes at 4°C in the dark. T cells were additionally stained with CD3 and CD8 antibodies for selection of CD8+ T cells subpopulation. Cells were then washed again and mixed with 7-AAD viability dye before flow acquisition to test for surface binding.

Interestingly, and to support the cross-specificity of A-045 showed specific, dose-dependent, high affinity binding to LILRB1 (1.5nM) and LILRB2 (1.3nM), but only marginal binding to unwanted counter-targets LILRA3 at high antibody concentrations. No binding could be detected for LILRA1 and LILRA2 (Figure 7A). Additionally, mono-specific reference molecule Ref047 showed binding only to LILRB2 (1.7 nM) and none of the other targets (Figure 7B), while LILRB1/2 cross-specific reference molecule Ref062 has high affinity for LILRB1 (9.1nM) and LILRB2 (17.3nM) but also showed binding to LILRA1 (3.7nM) and, surprisingly also to LILRA3 (7.8nM) (Figure 7C).

In addition, A-045 showed high affinity binding to primary T cells (1nM) and monocytes (0.9nM) (Figure 7D and 7E). However, the affinity of cross-specific reference molecule Ref062 is slightly lower on both cell types with 12 nM and 4.6 nM on T cells and monocytes, respectively. Further, LILRB2 mono-specific reference antibody Ref047 only binds on monocytes (1nM) due to the lack of LILRB2 expression on primary T cells, highlighting superiority of the ABP of the invention.

### Example 6: Reversal of Macrophage-Mediated T Cell Suppression Translates into Tumor Cell Killing

T cell suppression and tumor cell killing was analyzed by tri-culture assay, briefly describe as follows (Figure 8a): CD14+ monocytes and CD3+ T-cells from leukapheresis donors were isolated, frozen, and processed independently. Monocytes were thawed and differentiated to macrophages in medium with M-CSF, in presence of either hIgG1s_A-010 or negative control, hIgG1s_Ref-001. Differentiated macrophages were scraped from flasks and polarized to M2-like macrophages with IL-4, IL-10 and TGF-β for 2 days. Autologous anti-CD3/anti-CD28 activated T cells were added to the assay and cultured with or without polarized macrophages in a co-culture setting, in presence of either hIgG1s_A-010 or negative control, hIgG1s_Ref-001. After 3 days of co-culture with stimulated T cells, supernatants were harvested for the analysis of T cell activation. The effector T cell activity was measured by ELISA, wherein, secretion of IL-2 (Figure 8B) and IFN-γ (data not shown) correlated with T cell stimulation. Finally, MDA-MB-231/Luc-2A-GFP tumor cell line (which co-expresses the luciferase gene and GFP fluorescent markers) was added for a tri-culture (tumor killing assay) in presence of either hIgG1s_A-010 or negative control, hIgG1s_Ref001. Post 3 days of tri-culture, residual luciferase activity was measured by luciferase reporter assay (Figure 8C) and % confluency mask of GFP expressing tumor cell was evaluated using Incucyte Opti-Green software (data not shown) which directly correlates with tumor cell viability.

T cell activation, as assessed by IL-2 secretion measured in ELISA, is fully suppressed when T cells are co-cultured with M2-polarized macrophages in the presence of an isotype control antibody, Ref-001. This macrophage-mediated T cell suppression is significantly reverted by the addition of a functional anti-LILRB2/1 antibody, A-010 (Figure 8B). Assay controls include IL-2 secretion levels of either unstimulated T cells (negative control) or stimulated T cells (positive control) in the absence of macrophages.

Luciferase reporter positive tumor cells were added to the co-cultures and luciferase signal was assessed after 3 days of tri-culture to quantify the remaining tumor cell viability. As shown in Figure 8C, when A-010 was present throughout the experiment, the consequential reversal of T cell suppression resulted in a strong tumor cell killing. Indeed, this effect was to a similar extent as the positive assay control (addition of stimulated T cells to tumor cells in the absence of suppressive, M2-polarized macrophages). In contrast, Ref-001 isotype control (with full macrophage-mediated T cell suppression) does not translate into significant tumor cell killing compared to negative assay controls (tumor cells only or tumor cells in the presence of stimulated T cells but in the absence of suppressive, M2-polarized macrophages).

### Example 7: Displacement of LILRB1 or LILRB2 from HLA-G interaction site and inhibition of new complex formation between LILRB1 or LILRB2 with HLA-G

To identify the ability of the binders to inhibit the complex formation between LILRB1 and HLA-G or LILRB2 and HLA-G, respectively, biolayer interferometry (BLI) experiments were performed on an Octet RED96e system. Briefly, biotinylated recombinant HLA-G was immobilized on streptavidin coated biosensors to 1.5nm using a fixed loading threshold. To block unoccupied biotin binding sites on the streptavidin coated biosensors, biocytin was included in the assay buffer at a concentration of 10µg/mL for all further steps. To obtain tetrameric complexes of biotinylated LILRB1 or LILRB2, an excess of 100nM biotinylated recombinant LILRB1 or LILRB2 was premixed with 12.5nM streptavidin tetramer (ThermoFisher, 434302) in 1x kinetics buffer and incubated for 25 minutes. These samples were 2-fold concentrated and mixed 1:1 with 1x kinetics buffer containing 20 µg/mL biocytin in the sample plate to ensure blocking of biotin binding sites on the streptavidin coated biosensors in all steps after loading.

The sensors were dipped in wells containing the streptavidin premixed with either LILRB1 or LILRB2 to allow the tetrameric complexes of LILRB1 or LILRB2 to bind to the HLA-G immobilized on the biosensors. Subsequently, the sensors were dipped into 1x kinetics buffer containing 100nM of antigen binding fragment (Fab) to measure the displacement of the tetrameric complexes of LILRB1 or LILRB2 from the HLA-G immobilized on the biosensors over the course of 1800 seconds. As reference control, the dissociation of the tetrameric complexes of LILRB1 or LILRB2 from the HLA-G immobilized on the biosensors was analyzed by dipping the sensor in 1x kinetics buffer not containing antigen binding fragment.

To correct for sensor drift, all sensorgrams were referenced using the signal from one biosensor, with HLA-G immobilized, in 1x kinetics buffer supplemented with 10µg/mL biocytin.

Sensorgrams of this displacement and inhibition assay for antigen binding fragments of selected antibody clones of the invention are shown for LILRB2 (Figure 9) and LILRB1 (Figure 10).

Surprisingly, A-045, A-047, and A-048 showed stronger inhibition of complex formation between HLA-G and LILRB1 or LILRB2, respectively, than cross-specific reference molecule (Ref062 [NGM]) or antibody A-010, which both showed comparable inhibition. In general, displacement of LILRB2 from HLA-G interaction site and inhibition of new complex formation with HLA-G was more pronounced than displacement of LILRB1.

### Example 8 [prophetic]: In-vivo anti-tumor properties of cross-specific LILRB1/2 antibodies of the invention

Antibody A-045 is investigated together with the corresponding cross-specific reference molecule (Ref062 [NGM]) in a proof-of-concept efficacy *in vivo* study*.* Mice are humanized using human hematopoietic stem cells (CD34+) isolated from human cord blood following Transcure's proprietary humanization protocol. To evaluate the best mouse strain concerning humanization, two different mouse strains are run in parallel. Different Donors are used, and the humanization rate (human CD45 + human CD33+) is tested. Moreover, LILRB1 and LILRB2 expression is checked post-humanization. Mice are randomized for human HSC donors and the selected tumor cell line is inoculated (e.g. MDA-MB-231, MSTO-211H, NCI-H1975). Treatment starts when the average tumor volume reaches ∼100mm3. Treatment schedule is provided in Table 3.The humanized mice are randomized based on tumor volume, body weight, CD34+ donors and humanization rate into the following groups: A-045, Ref001 (isotype control; vehicle), and cross-specific reference molecule (Ref062 [NGM]). Animal health is monitored daily for unexpected signs of distress. A clinical score is determined 3 times per week. The tumor volume (TV) is measured 3 times per week using a caliper. A TV exceeding 1500 mm3 is considered as an endpoint and the mouse will be sacrificed. The tumor is then collected at sacrifice, weighed, and analyzed for immune cells (Flowcytometry) and Cytokines (Milliplex). If the tumor size is big enough, a part will be processed for FFPE for subsequent immunohistochemistry. Treatments are done according to **Table** .Such results further demonstrate the ability of antibodies of the present invention to inhibitit tumor growth in an in vivo setting by their advantageous on target binding compared to off target binding profile.

**Table 3: In vivo study schedule for testing LILRB1/2 antibodies**

| **Group** | **Treatment** | **Mouse strain** | **Tumor model** | **Dose (mg/kg)** | **Admini stratio n route** | **Dosing frequency** | **Number of animals** |
|---|---|---|---|---|---|---|---|
| 1 | Ref001 | NOG-EXL | MDA-MB-231 | 20.0 | i.p. | 2 times per week | 12 |
| 2 | A-045 | NOG-EXL | MDA-MB-231 | 20.0 | i.p. | 2 times per week | 12 |
| 3 | Ref062 | NOG-EXL | MDA-MB-231 | 20.0 | i.p. | 2 times per week | 12 |
| 4 | Ref001 | NOG-EXL | MSTO-211H | 20.0 | i.p. | 2 times per week | 12 |
| 5 | A-045 | NOG-EXL | MSTO-211H | 20.0 | i.p. | 2 times per week | 12 |
| 6 | Ref062 | NOG-EXL | MSTO-211H | 20.0 | i.p. | 2 times per week | 12 |
| 7 | Ref001 | NOG-EXL | NCI-H1975 | 20.0 | i.p. | 2 times per week | 12 |
| 8 | A-045 | NOG-EXL | NCI-H1975 | 20.0 | i.p. | 2 times per week | 12 |
| 9 | Ref062 | NOG-EXL | NCI-H1975 | 20.0 | i.p. | 2 times per week | 12 |
| 10 | Ref001 | NCG boosted | MDA-MB-231 | 20.0 | i.p. | 2 times per week | 12 |
| 11 | A-045 | NCG boosted | MDA-MB-231 | 20.0 | i.p. | 2 times per week | 12 |
| 12 | Ref062 | NCG boosted | MDA-MB-231 | 20.0 | i.p. | 2 times per week | 12 |
| 13 | Ref001 | NCG boosted | MSTO-211H | 20.0 | i.p. | 2 times per week | 12 |
| 14 | A-045 | NCG boosted | MSTO-211H | 20.0 | i.p. | 2 times per week | 12 |
| 15 | Ref062 | NCG boosted | MSTO-211H | 20.0 | i.p. | 2 times per week | 12 |
| 16 | Ref001 | NCG boosted | NCI-H1975 | 20.0 | i.p. | 2 times per week | 12 |
| 17 | A-045 | NCG boosted | NCI-H1975 | 20.0 | i.p. | 2 times per week | 12 |
| 18 | Ref062 | NCG boosted | NCI-H1975 | 20.0 | i.p. | 2 times per week | 12 |

The sequences show:
**SEQ ID NOs. 1 to 384** (Amino acid sequences of CDR and variable regions of ABPs of the invention, as well as nucleic acid sequences encoding variable regions of ABPs of the invention, see **Table 1):**
**SEQ ID NO. 385** (Human LILRB1 protein isoform 1; UniProt identifier Q8NHL6-1): 
MTPILTVLICLGLSLGPRTHVQAGHLPKPTLWAEPGSVITQGSPVTLRCQGGQETQEYRLYREKKTALWITRIPQELVKKGQFPIPSIT
WEHAGRYRCYYGSDTAGRSESSDPLELVVTGAYIKPTLSAQPSPVVNSGGNVILQCDSQVAFDGFSLCKEGEDEHPQCLNSQPHAR
GSSRAIFSVGPVSPSRRWWYRCYAYDSNSPYEWSLPSDLLELLVLGVSKKPSLSVQPGPIVAPEETLTLQCGSDAGYNRFVLYKDGER
DFLQLAGAQPQAGLSQANFTLGPVSRSYGGQYRCYGAHNLSSEWSAPSDPLDILIAGQFYDRVSLSVQPGPTVASGENVTLLCQSQ
GWMQTFLLTKEGAADDPWRLRSTYQSQKYQAEFPMGPVTSAHAGTYRCYGSQSSKPYLLTHPSDPLELVVSGPSGGPSSPTTGPTS
TSGPEDQPLTPTGSDPQSGLGRHLGVVIGILVAVILLLLLLLLLFLILRHRRQGKHWTSTQRKADFQHPAGAVGPEPTDRGLQWRSSP
AADAQEENLYAAVKHTQPEDGVEMDTRSPHDEDPQAVTYAEVKHSRPRREMASPPSPLSGEFLDTKDRQAEEDRQMDTEAAASEAP
 QDVTYAQLHSLTLRREATEPPPSQEGPSPAVPSIYATLAIH
**SEQ ID NO. 386** (Human LILRB1 protein isoform 2; UniProt identifier Q8NHL6-2): 
MTPILTVLICLGLSLGPRTHVQAGHLPKPTLWAEPGSVITQGSPVTLRCQGGQETQEYRLYREKKTALWITRIPQELVKKGQFPIPSIT
WEHAGRYRCYYGSDTAGRSESSDPLELVVTGAYIKPTLSAQPSPVVNSGGNVILQCDSQVAFDGFSLCKEGEDEHPQCLNSQPHAR
GSSRAIFSVGPVSPSRRWWYRCYAYDSNSPYEWSLPSDLLELLVLGVSKKPSLSVQPGPIVAPEETLTLQCGSDAGYNRFVLYKDGER
DFLQLAGAQPQAGLSQANFTLGPVSRSYGGQYRCYGAHNLSSEWSAPSDPLDILIAGQFYDRVSLSVQPGPTVASGENVTLLCQSQ
GWMQTFLLTKEGAADDPWRLRSTYQSQKYQAEFPMGPVTSAHAGTYRCYGSQSSKPYLLTHPSDPLELVVSGPSGGPSSPTTGPTS
TSAGPEDQPLTPTGSDPQSGLGRHLGVVIGILVAVILLLLLLLLLFLILRHRRQGKHWTSTQRKADFQHPAGAVGPEPTDRGLQWRSS
PAADAQEENLYAAVKHTQPEDGVEMDTRSPHDEDPQAVTYAEVKHSRPRREMASPPSPLSGEFLDTKDRQAEEDRQMDTEAAASEA
PQDVTYAQLHSLTLRREATEPPPSQEGPSPAVPSIYATLAIH
**SEQ ID NO. 387** (Human LILRB1 protein isoform 3; UniProt identifier Q8NHL6-3): 
MTPILTVLICLGLSLGPRTHVQAGHLPKPTLWAEPGSVITQGSPVTLRCQGGQETQEYRLYREKKTALWITRIPQELVKKGQFPIPSIT
WEHAGRYRCYYGSDTAGRSESSDPLELVVTGAYIKPTLSAQPSPVVNSGGNVILQCDSQVAFDGFSLCKEGEDEHPQCLNSQPHAR
GSSRAIFSVGPVSPSRRWWYRCYAYDSNSPYEWSLPSDLLELLVLGVSKKPSLSVQPGPIVAPEETLTLQCGSDAGYNRFVLYKDGER
DFLQLAGAQPQAGLSQANFTLGPVSRSYGGQYRCYGAHNLSSEWSAPSDPLDILIAGQFYDRVSLSVQPGPTVASGENVTLLCQSQ
GWMQTFLLTKEGAADDPWRLRSTYQSQKYQAEFPMGPVTSAHAGTYRCYGSQSSKPYLLTHPSDPLELVVSGPSGGPSSPTTGPTS
TSAGPEDQPLTPTGSDPQSGLGRHLGVVIGILVAVILLLLLLLLLFLILRHRRQGKHWTSTQRKADFQHPAGAVGPEPTDRGLQWRSS
PAADAQEENLYAAVKHTQPEDGVEMDTRQSPHDEDPQAVTYAEVKHSRPRREMASPPSPLSGEFLDTKDRQAEEDRQMDTEAAAS
EAPQDVTYAQLHSLTLRREATEPPPSQEGPSPAVPSIYATLAIH
**SEQ ID NO. 388** (Human LILRB1 protein isoform 4; UniProt identifier Q8NHL6-4): 
MTPILTVLICLGLSLGPRTHVQAGHLPKPTLWAEPGSVITQGSPVTLRCQGGQETQEYRLYREKKTALWITRIPQELVKKGQFPIPSIT
WEHAGRYRCYYGSDTAGRSESSDPLELVVTGAYIKPTLSAQPSPVVNSGGNVILQCDSQVAFDGFSLCKEGEDEHPQCLNSQPHAR
GSSRAIFSVGPVSPSRRWWYRCYAYDSNSPYEWSLPSDLLELLVLGVSKKPSLSVQPGPIVAPEETLTLQCGSDAGYNRFVLYKDGER
DFLQLAGAQPQAGLSQANFTLGPVSRSYGGQYRCYGAHNLSSEWSAPSDPLDILIAGQFYDRVSLSVQPGPTVASGENVTLLCQSQ
GWMQTFLLTKEGAADDPWRLRSTYQSQKYQAEFPMGPVTSAHAGTYRCYGSQSSKPYLLTHPSDPLELVVSGPSGGPSSPTTGPTS
TSGPEDQPLTPTGSDPQSGLGRHLGVVIGILVAVILLLLLLLLLFLILRHRRQGKHWTSTQRKADFQHPAGAVGPEPTDRGLQWRSSP
AADAQEENLYAAVKHTQPEDGVEMDTRQSPHDEDPQAVTYAEVKHSRPRREMASPPSPLSGEFLDTKDRQAEEDRQMDTEAAASE
APQDVTYAQLHSLTLRREATEPPPSQEGPSPAVPSIYATLAIH
**SEQ ID NO. 389** (Human LILRB1 protein isoform 5; UniProt identifier Q8NHL6-5): 
MTPILTVLICLGLSLGPRTHVQAGHLPKPTLWAEPGSVITQGSPVTLRCQGGQETQEYRLYREKKTALWITRIPQELVKKGQFPIPSIT
WEHAGRYRCYYGSDTAGRSESSDPLELVVTGAYIKPTLSAQPSPVVNSGGNVILQCDSQVAFDGFSLCKEGEDEHPQCLNSQPHAR
GSSRAIFSVGPVSPSRRWWYRCYAYDSNSPYEWSLPSDLLELLVLGVSKKPSLSVQPGPIVAPEETLTLQCGSDAGYNRFVLYKDGER
DFLQLAGAQPQAGLSQANFTLGPVSRSYGGQYRCYGAHNLSSEWSAPSDPLDILIAGQFYDRVSLSVQPGPTVASGENVTLLCQSQ
GWMQTFLLTKEGAADDPWRLRSTYQSQKYQAEFPMGPVTSAHAGTYRCYGSQSSKPYLLTHPSDPLELVVSGPSGGPSSPTTGPTS
TSAGPEDQPLTPTGSDPQSGE
**SEQ ID NO. 390** (Human LILRB2 protein isoform 2; UniProt identifier Q8N423-2): 
MTPIVTVLICLGLSLGPRTRVQTGTIPKPTLWAEPDSVITQGSPVTLSCQGSLEAQEYRLYREKKSASWITRIRPELVKNGQFHIPSIT
WEHTGRYGCQYYSRARWSELSDPLVLVMTGAYPKPTLSAQPSPVVTSGGRVTLQCESQVAFGGFILCKEGEDEHPQCLNSQPHARG
SSRAIFSVGPVSPNRRWSHRCYGYDLNSPYVWSSPSDLLELLVPGVSKKPSLSVQPGPVMAPGESLTLQCVSDVGYDRFVLYKEGER
DLRQLPGRQPQAGLSQANFTLGPVSRSYGGQYRCYGAHNLSSECSAPSDPLDILITGQIRGTPFISVQPGPTVASGENVTLLCQSWR
QFHTFLLTKAGAADAPLRLRSIHEYPKYQAEFPMSPVTSAHAGTYRCYGSLNSDPYLLSHPSEPLELVVSGPSMGSSPPPTGPISTPGP
EDQPLTPTGSDPQSGLGRHLGVVIGILVAVVLLLLLLLLLFLILRHRRQGKHWTSTQRKADFQHPAGAVGPEPTDRGLQWRSSPAAD
AQEENLYAAVKDTQPEDGVEMDTRAAASEAPQDVTYAQLHSLTLRRKATEPPPSQEREPPAEPSIYATLAIH
**SEQ ID NO. 391** (Human LILRB2 protein isoform 1; UniProt identifier Q8N423-1): 
MTPIVTVLICLGLSLGPRTRVQTGTIPKPTLWAEPDSVITQGSPVTLSCQGSLEAQEYRLYREKKSASWITRIRPELVKNGQFHIPSIT
WEHTGRYGCQYYSRARWSELSDPLVLVMTGAYPKPTLSAQPSPVVTSGGRVTLQCESQVAFGGFILCKEGEDEHPQCLNSQPHARG
SSRAIFSVGPVSPNRRWSHRCYGYDLNSPYVWSSPSDLLELLVPGVSKKPSLSVQPGPVMAPGESLTLQCVSDVGYDRFVLYKEGER
DLRQLPGRQPQAGLSQANFTLGPVSRSYGGQYRCYGAHNLSSECSAPSDPLDILITGQIRGTPFISVQPGPTVASGENVTLLCQSWR
QFHTFLLTKAGAADAPLRLRSIHEYPKYQAEFPMSPVTSAHAGTYRCYGSLNSDPYLLSHPSEPLELVVSGPSMGSSPPPTGPISTPAG
PEDQPLTPTGSDPQSGLGRHLGVVIGILVAVVLLLLLLLLLFLILRHRRQGKHWTSTQRKADFQHPAGAVGPEPTDRGLQWRSSPAAD
AQEENLYAAVKDTQPEDGVEMDTRAAASEAPQDVTYAQLHSLTLRRKATEPPPSQEREPPAEPSIYATLAIH
**SEQ ID NO. 392** (Human LILRB2 protein isoform 3; UniProt identifier Q8N423-3): 
MTPIVTVLICLGLSLGPRTRVQTGTIPKPTLWAEPDSVITQGSPVTLSCQGSLEAQEYRLYREKKSASWITRIRPELVKNGQFHIPSIT
WEHTGRYGCQYYSRARWSELSDPLVLVMTGAYPKPTLSAQPSPVVTSGGRVTLQCESQVAFGGFILCKEGEDEHPQCLNSQPHARG
SSRAIFSVGPVSPNRRWSHRCYGYDLNSPYVWSSPSDLLELLVPGVSKKPSLSVQPGPVMAPGESLTLQCVSDVGYDRFVLYKEGER
DLRQLPGRQPQAGLSQANFTLGPVSRSYGGQYRCYGAHNLSSECSAPSDPLDILITGQIRGTPFISVQPGPTVASGENVTLLCQSWR
QFHTFLLTKAGAADAPLRLRSIHEYPKYQAEFPMSPVTSAHAGTYRCYGSLNSDPYLLSHPSEPLELVVSGPSMGSSPPPTGPISTPAG
PEDQPLTPTGSDPQSGLGRHLGVVIGILVAVVLLLLLLLLLFLILRHRRQGKHWTSSPAQLPTPRKKTSMLP
**SEQ ID NO. 393** (Human LILRB2 protein isoform 4; UniProt identifier Q8N423-4): 
MTGAYPKPTLSAQPSPVVTSGGRVTLQCESQVAFGGFILCKEGEDEHPQCLNSQPHARGSSRAIFSVGPVSPNRRWSHRCYGYDLN
SPYVWSSPSDLLELLVPGVSKKPSLSVQPGPVMAPGESLTLQCVSDVGYDRFVLYKEGERDLRQLPGRQPQAGLSQANFTLGPVSRS
YGGQYRCYGAHNLSSECSAPSDPLDILITGQIRGTPFISVQPGPTVASGENVTLLCQSWRQFHTFLLTKAGAADAPLRLRSIHEYPKY
QAEFPMSPVTSAHAGTYRCYGSLNSDPYLLSHPSEPLELVVSGPSMGSSPPPTGPISTPAGPEDQPLTPTGSDPQSGLGRHLGVVIGIL
VAVVLLLLLLLLLFLILRHRRQGKHWTSTQRKADFQHPAGAVGPEPTDRGLQWRSSPAADAQEENLYAAVKDTQPEDGVEMDTRAA
ASEAPQDVTYAQLHSLTLRRKATEPPPSQEREPPAEPSIYATLAIH
**SEQ ID NO. 394** Human Leukocyte immunoglobulin-like receptor subfamily A member 1; UniProt identifier: 075019: 
MTPIVTVLICLRLSLGPRTHVQAGTLPKPTLWAEPGSVITQGSPVTLWCQGILETQEYRLYREKKTAPWITRIPQEIVKKGQFPIPSIT
WEHTGRYRCFYGSHTAGWSEPSDPLELVVTGAYIKPTLSALPSPVVTSGGNVTLHCVSQVAFGSFILCKEGEDEHPQCLNSQPRTHG
WSRAIFSVGPVSPSRRWSYRCYAYDSNSPHVWSLPSDLLELLVLGVSKKPSLSVQPGPIVAPGESLTLQCVSDVSYDRFVLYKEGERD
FLQLPGPQPQAGLSQANFTLGPVSRSYGGQYRCSGAYNLSSEWSAPSDPLDILIAGQFRGRPFISVHPGPTVASGENVTLLCQSWGP
FHTFLLTKAGAADAPLRLRSIHEYPKYQAEFPMSPVTSAHSGTYRCYGSLSSNPYLLSHPSDSLELMVSGAAETLSPPQNKSDSKAGA
ANTLSPSQNKTASHPQDYTVENLIRMGIAGLVLVVLGILLFEAQHSQRSL
**SEQ ID NO. 395** Human Leukocyte immunoglobulin-like receptor subfamily A member 3; UniProt identifier Q8N6C8:

MTPILTVLICLGLSLDPRTHVQAGPLPKPTLWAEPGSVITQGSPVTLRCQGSLETQEYHLYREKKTALWITRIPQELVKKGQFPILSITW
EHAGRYCCIYGSHTAGLSESSDPLELVVTGAYSKPTLSALPSPVVTSGGNVTIQCDSQVAFDGFILCKEGEDEHPQCLNSHSHARGSS
RAIFSVGPVSPSRRWSYRCYGYDSRAPYVWSLPSDLLGLLVPGVSKKPSLSVQPGPVVAPGEKLTFQCGSDAGYDRFVLYKEWGRDF
LQRPGRQPQAGLSQANFTLGPVSRSYGGQYTCSGAYNLSSEWSAPSDPLDILITGQIRARPFLSVRPGPTVASGENVTLLCQSQGGM
HTFLLTKEGAADSPLRLKSKRQSHKYQAEFPMSPVTSAHAGTYRCYGSLSSNPYLLTHPSDPLELVVSGAAETLSPPQNKSDSKAGE.

## Claims

1. **An isolated antigen binding protein (ABP)** which specifically binds to the extra cellular domain (ECD) of leukocyte immunoglobulin-like receptor subfamily B1 (LILRB1) and LILRB2 protein and wherein the isolated ABP comprises at least one, preferably 3 or 6, complementarity determining regions (CDRs) and is capable of inhibiting the binding of LILRB1 and LILRB2 to a natural ligand thereof (a natural ligand of LILRB1 and/or LILRB2, such as HLA-G), wherein the ABP does not bind to, or binds with less affinity to (at least 2 times less), LILRA1 and LILRA3.

2. The isolated ABP of claim 1, wherein the ABP binds to the ECD of LILRB1 and LILRB2 with at least 5 times higher, or preferably 10 times higher binding affinity compared the binding of the ABP to an ECD of LILRA1 and/or LILRA3, preferably wherein the ABP binding affinity is determined by bio-layer interferometry (BLI).

3. The isolated ABP of claim 1 or 2, wherein the ABP binds to the ECD of LILRB1 and/or LILRB2 with a binding dissociation constant (KD) that is at least 2 times lower, preferably 3 times lower, more preferably 5 times lower, most preferably 10 times lower, compared to the binding KD of the ABP to an ECD of LILRA1 and/or LILRA3, wherein the KD is determined by bio-layer interferometry (BLI), preferably under the conditions set out in example 3.

4. The isolated ABP of claim 3, wherein the ABP binds to an ECD of LILRB1 and LILRB2 with a dissociation constant (KD) that is at least 2 times lower, preferably 3 times lower, more preferably 5 times lower, most preferably 10 times lower, compared to the KD of the ABP to an ECD of LILRA1 and LILRA3, wherein the KD is determined by bio-layer interferometry (BLI), preferably under the conditions set out in example 3.

5. The isolated ABP of any one of claims 1 to 4, wherein ABP comprises one, preferably two, an antibody heavy chain sequence and one, preferably two, antibody light chain sequence, and wherein the antibody heavy chain sequence and the antibody light chain sequence are derived from one or a combination of the antibody parental clones A-001 to A-003, preferably from A-001 and/or A-003.

6. The isolated ABP of claim 5, wherein the ABP comprises comprises one, preferably two, an antibody heavy chain variable sequence and one, preferably two, antibody light chain variable sequence, wherein the antibody heavy and light chain variable sequence each comprise a sequence that is at least 90%, preferably at least 95%, more preferably at least 96%, 97%, 98%, 99% identical to an antibody heavy or light chain variable sequence shown for any of the parental antibody sequences A-001 to A-003, preferably A-001 and/or A-003.

7. The isolated ABP of any one of claims 1 to 6, wherein the ABP competes for binding to an ECD of LILRB1 and/or LILRB2, or to ECD of the variant of LILRB1 and/or LILRB2, with an endogenous LILRB1 and/or LILRB2 ligand or receptor, preferably wherein said endogenous LILRB1 and/or LILRB2 ligand or receptor is a HLA-G protein (or a variant of HLA-G)

8. The isolated ABP of claim 7, wherein the ABP is capable of inhibiting the binding of HLA-G protein or a variant thereof to LILRB1 and/or LILRB2 protein or a variant thereof with an IC50 of less than 100nM, less than 50nM, or preferably 20nM or less, such as 15nM or less, or less than 10nM, as determined by ELISA.

9. The isolated ABP of any one claims 1 to 8, comprising at least one complementarity determining region 3 (CDR3) having an amino acid sequence with at least 90% sequence identity to, or having no more than three or two, preferably no more than one amino acid substitution(s), deletion(s) or insertion(s) compared to, a sequence selected from SEQ ID Nos. 3, 7, 11, 15, 19, 23, 27, 31, 35, 39, 43, 47, 51, 55, 59, 63, 67, 71, 75, 79, 83, 87, 91, 95, 99, 103, 107, 111, 115, 119, 123, 127, 131, 135, 139, 143, 147, 151, 155, 159, 163, 167, 171, 175, 179, 183, 187, 191, 195, 199, 203, 207, 211, 215, 219, 223, 227, 231, 235, 239, 243, 247, 251, 255, 259, 263, 267, 271, 275, 279, 283, 287, 291, 295, 299, 303, 307, 311, 315, 319, 323, 327, 331, 335, 339, 343, 347, 351, 355, 359, 363, 367, 371, 375, 379, and 383.

10. The isolated ABP of any one of claims 1 to 9, wherein the ABP is an antibody, or an antigen binding fragment thereof, composed of at least one, preferably two, antibody heavy chain sequences, and at least one, preferably two, antibody light chain sequences, wherein at least one, preferably both, of the antibody heavy chain sequences and at least one, preferably both, of the antibody light chain sequences comprise CDR1 to CDR3 sequences in a combination selected from any of the following combinations of heavy and/or light chain CDRs, CDRs-A-001 to CDRs-A-048:
| **ABP Combination (ID)** | **Heavy Chain CDR1 to CDR3 (SEQ ID NO)** | | | **Light Chain CDR1 to CDR3 (SEQ ID NO)** | | |
|---|---|---|---|---|---|---|
| CDRs-A-001 | 1 | 2 | 3 | 5 | 6 | 7 |
| CDRs-A-002 | 9 | 10 | 11 | 13 | 14 | 15 |
| CDRs-A-003 | 17 | 18 | 19 | 21 | 22 | 23 |
| CDRs-A-004 | 25 | 26 | 27 | 29 | 30 | 31 |
| CDRs-A-005 | 33 | 34 | 35 | 37 | 38 | 39 |
| CDRs-A-006 | 41 | 42 | 43 | 45 | 46 | 47 |
| CDRs-A-007 | 49 | 50 | 51 | 53 | 54 | 55 |
| CDRs-A-008 | 57 | 58 | 59 | 61 | 62 | 63 |
| CDRs-A-009 | 65 | 66 | 67 | 69 | 70 | 71 |
| CDRs-A-010 | 73 | 74 | 75 | 77 | 78 | 79 |
| CDRs-A-011 | 81 | 82 | 83 | 85 | 86 | 87 |
| CDRs-A-012 | 89 | 90 | 91 | 93 | 94 | 95 |
| CDRs-A-013 | 97 | 98 | 99 | 101 | 102 | 103 |
| CDRs-A-014 | 105 | 106 | 107 | 109 | 110 | 111 |
| CDRs-A-015 | 113 | 114 | 115 | 117 | 118 | 119 |
| CDRs-A-016 | 121 | 122 | 123 | 125 | 126 | 127 |
| CDRs-A-017 | 129 | 130 | 131 | 133 | 134 | 135 |
| CDRs-A-018 | 137 | 138 | 139 | 141 | 142 | 143 |
| CDRs-A-019 | 145 | 146 | 147 | 149 | 150 | 151 |
| CDRs-A-020 | 153 | 154 | 155 | 157 | 158 | 159 |
| CDRs-A-021 | 161 | 162 | 163 | 165 | 166 | 167 |
| CDRs-A-022 | 169 | 170 | 171 | 173 | 174 | 175 |
| CDRs-A-023 | 177 | 178 | 179 | 181 | 182 | 183 |
| CDRs-A-024 | 185 | 186 | 187 | 189 | 190 | 191 |
| CDRs-A-025 | 193 | 194 | 195 | 197 | 198 | 199 |
| CDRs-A-026 | 201 | 202 | 203 | 205 | 206 | 207 |
| CDRs-A-027 | 209 | 210 | 211 | 213 | 214 | 215 |
| CDRs-A-028 | 217 | 218 | 219 | 221 | 222 | 223 |
| CDRs-A-029 | 225 | 226 | 227 | 229 | 230 | 231 |
| CDRs-A-030 | 233 | 234 | 235 | 237 | 238 | 239 |
| CDRs-A-031 | 241 | 242 | 243 | 245 | 246 | 247 |
| CDRs-A-032 | 249 | 250 | 251 | 253 | 254 | 255 |
| CDRs-A-033 | 257 | 258 | 259 | 261 | 262 | 263 |
| CDRs-A-034 | 265 | 266 | 267 | 269 | 270 | 271 |
| CDRs-A-035 | 273 | 274 | 275 | 277 | 278 | 279 |
| CDRs-A-036 | 281 | 282 | 283 | 285 | 286 | 287 |
| CDRs-A-037 | 289 | 290 | 291 | 293 | 294 | 295 |
| CDRs-A-038 | 297 | 298 | 299 | 301 | 302 | 303 |
| CDRs-A-039 | 305 | 306 | 307 | 309 | 310 | 311 |
| CDRs-A-040 | 313 | 314 | 315 | 317 | 318 | 319 |
| CDRs-A-041 | 321 | 322 | 323 | 325 | 326 | 327 |
| CDRs-A-042 | 329 | 330 | 331 | 333 | 334 | 335 |
| CDRs-A-043 | 337 | 338 | 339 | 341 | 342 | 343 |
| CDRs-A-044 | 345 | 346 | 347 | 349 | 350 | 351 |
| CDRs-A-045 | 353 | 354 | 355 | 357 | 358 | 359 |
| CDRs-A-046 | 361 | 362 | 363 | 365 | 366 | 367 |
| CDRs-A-047 | 369 | 370 | 371 | 373 | 374 | 375 |
| CDRs-A-048 | 377 | 378 | 379 | 381 | 382 | 383 |
in each case independently, optionally with no more than three or two, preferably no more than one, amino acid substitution(s), insertion(s) or deletion(s) compared to these sequences.

11. **An isolated ABP** which competes with an ABP as recited in any one of claims 1 to 10 for binding to the ECD of the LILRB1 and/or LILRB2 protein and is able to inhibit the binding of the LILRB1 and/or LILRB2 protein or the variant thereof to a natural ligand of LILRB1 and/or LILRB2, preferably wherein the isolated ABP.

12. The isolated ABP of any one of claims 1 to 11 that (i) inhibits a binding of a natural ligand of LILRB1 and/or LILRB2 to LILRB1 and/or LILRB2, wherein such ligand is preferably HLA-G, and/or (ii) reduces development of immune-suppressive phenotypes, for example in a tumour microenvironment.

13. The isolated ABP of any one of claims 1 to 12 that is an antibody or an antigen binding fragment thereof, wherein the antibody is a monoclonal antibody, or wherein the antigen binding fragment is a fragment of a monoclonal antibody.

14. **An isolated nucleic acid** encoding for an ABP, or for an antigen binding fragment or a monomer of an ABP, wherein the ABP is one recited in any one of claims 1 to 13.

15. **A recombinant host cell** comprising a nucleic acid recited in claim 14.

16. **A pharmaceutical composition** comprising: (i) an ABP recited in any one of claims 1 to 13, or (ii) a nucleic acid recited in claim 14, or (iii) a recombinant host cell of claim 15, and a pharmaceutically acceptable carrier, stabiliser and/or excipient.

17. **A product for use in medicine,** wherein the product is selected from the list consisting of: (i) an isolated ABP recited in any one of claims 1 to 13, and (ii) an isolated nucleic acid recited in claim 14, (iii) a recombinant host cell of claim 15, and (iv) a pharmaceutical composition of claim 16.

18. The product for use of claim **Fehler! Verweisquelle konnte nicht gefunden werden.,** wherein the use in medicine is a treatment of a proliferative disorder, and wherein cells involved in the proliferative disorder express a natural ligand of LILRB1 and/or LILRB2, and wherein such cells induce an immune-suppressive phenotype in immune cells, such as macrophages, preferably of the subject.

19. The product for use of claim 17 or 18, wherein the product is for use in enhancing an immune response in a mammalian subject to be treated, preferably for use in promoting immune-activation of a humoral or cell-mediated immune response in the subject, such as promoting pro-inflammatory polarization of macrophages towards an M1 phenotype, for example for treating a proliferative disease, such as a cancer disease, of for treating an infectious disease.

20. The product for use of any one of claims 17 to 19, wherein the product has any one or any combination of the following characteristics:
a. specific binding to human LILRB1 and/or (preferably and) LILRB2 (e.g. comprising the amino acid sequence of SEQ ID NO: 353 (LILRB1) and SEQ ID NO: 358 (LILRB2)), e.g., with a KD 50 nM or less, or of 20nM or less; more preferably of 10nM or less or 5nM or less
b. lack of specific binding to a LILRA protein, such as preferably LILRA1 and/or LILRA3;
c. stimulates T cell activation, e.g., in a mixed lymphocyte reaction (MLR) assay, as measured by increased T cell proliferation or IFN-gamma secretion, e.g.;
d. stimulates differentiation or activation of monocytes into macrophages, e.g., stimulates differentiation of monocytes into pro-inflammatory macrophages, e.g., as shown in an assay described in the Examples;
e. inhibits binding of LILRB1 and/or (preferably and) LILRB2 to HLA-A and HLA-B, preferably to HLA-G;
f. has a binding profile as shown in **Table 2;**
g. promotes pro-inflammatory polarization of macrophages towards M1 macrophages; and
h. does not induce (or trigger) basophil activation.
